# EUROPEAN PATENT APPLICATION

(11) **EP 4 296 359 A1**
(43) Date of publication of application: **27.12.2023**
(21) Application number: 22742556.8
(22) Date of filing: 18.01.2022
(51) Int. Cl.: C12N 15/31, A61K 38/55, A61P 1/00, A61P 31/00, C12N 1/20, C12N 15/63

(54) **COMPOSITION FOR DECOMPOSITION OF TRYPSIN OR TMPRSS2**

(30) Priority: 19.01.2021 US 202163138798 P; 04.08.2021 US 202163229077 P
(71) Applicant: RIKEN, Wako-shi, Saitama 351-0198 (JP); Keio University, Tokyo, 108-8345 (JP)
(72) Inventor: LI Youxian, Wako-shi, Saitama 351-0198 (JP); WATANABE Eiichiro, Wako-shi, Saitama 351-0198 (JP); KAWASHIMA Yusuke, Wako-shi, Saitama 351-0198 (JP); WANG Zhujun, Wako-shi, Saitama 351-0198 (JP); OHARA Osamu, Wako-shi, Saitama 351-0198 (JP); HONDA Kenya, Tokyo 160-8582 (JP); ATARASHI Koji, Tokyo 160-8582 (JP)
(74) Representative: V.O.
(86) International application number: PCT/JP2022/001494
(87) International publication number: WO 2022/158434

(57) **Abstract**

A composition for decomposition of trypsin or TMPRSS2 containing, as an active ingredient: bacteria that have 00502 protein, or a protein having 30% or higher sequence identity with an amino acid sequence of the 00502 protein and having a trypsin-binding ability; or bacteria that have 00509 protein, or a protein having 30% or higher sequence identity with an amino acid sequence of the 00509 protein and having a trypsin-binding ability.

## Description

### [Technical Field]

The present invention relates to a composition for decomposition of trypsin or TMPRSS2. More specifically, the present invention relates to a composition for decomposition of trypsin or TMPRSS2, a diagnostic drug for diseases caused by trypsin or TMPRSS2, and a quasi-drug for diseases caused by trypsin or TMPRSS2. Priority is claimed on United States Provisional Patent Application No. US63/138,798, filed January 19, 2021, and United States Provisional Patent Application No. US63/229,077 filed August 4, 2021, the contents of which are incorporated herein by reference.

### [Background Art]

The gastrointestinal tract is a unique organ that is constantly exposed to innumerable molecules derived from food and microorganisms. The gastrointestinal tract also encounters host-derived molecules such as digestive enzymes, in addition to external contents. In the upper gastrointestinal tract, digestive enzymes play an important role in breaking down large amounts of ingested nutrients from food into smaller components and absorption into the body. On the other hand, the large intestine mainly absorbs water, and no digestive enzymes are required, but rather this disturbance of the activity is thought to be associated with the change in the composition of the microbiota, the disruption of the mucosal barrier, and the generation of inflammation.

The intestinal tissue has various regulatory mechanisms and protective mechanisms, such as the production of mucin and an enzyme-inactivating molecule, in order to maintain homeostasis and a barrier function. In addition, it is known that the intestinal microbiota greatly contribute to the maintenance of a stable environment by reducing or changing substances in the lumen (refer to Non Patent Document 1, for example).

### [Citation List]

### [Non Patent Document]

### [Non Patent Document 1]

J. L. Round and S. K. Mazmanian, The gut microbiota shapes intestinal immune responses during health and disease, Nature reviews immunology, 9, 313-323, 2009.

### [Summary of Invention]

### [Technical Problem]

However, the details of the regulation of intraluminal proteins by microorganisms have not been fully elucidated. In particular, little has been elucidated about the properties of microorganisms relating to the regulation of digestive enzymes remaining in the large intestine. An object of the present invention is to provide a technique for regulating the activity of proteases remaining in the large intestine by elucidating the functions of microorganisms relating to the regulation of the proteases.

### [Solution to Problem]

The present invention includes the following aspects.
[1] A composition for decomposition of trypsin or TMPRSS2 containing, as an active ingredient: bacteria that have 00502 protein, or a protein having 30% or higher sequence identity with an amino acid sequence of the 00502 protein and having a trypsin-binding ability; or bacteria that have 00509 protein, or a protein having 30% or higher sequence identity with an amino acid sequence of the 00509 protein and having a trypsin-binding ability.
[2] A composition for decomposition of trypsin or TMPRSS2 containing, as an active ingredient: bacteria that have 00502 protein, or a protein having 30% or higher sequence identity with an amino acid sequence of the 00502 protein and having a trypsin-binding ability; and bacteria that have 00509 protein, or a protein having 30% or higher sequence identity with an amino acid sequence of the 00509 protein and having a trypsin-binding ability.
[3] A composition for decomposition of trypsin or TMPRSS2 containing, as an active ingredient: bacteria that have a gene consisting of a base sequence set forth in SEQ ID NO: 1, or a gene having 30% or higher sequence identity with the base sequence set forth in SEQ ID NO: 1 and encoding a protein having a trypsin-binding ability; or bacteria that have a gene consisting of a base sequence set forth in SEQ ID NO: 2, or a gene having 30% or higher sequence identity with the base sequence set forth in SEQ ID NO: 2 and encoding a protein having a trypsin-binding ability.
[4] A composition for decomposition of trypsin or TMPRSS2 containing, as an active ingredient: bacteria that have a gene consisting of a base sequence set forth in SEQ ID NO: 1, or a gene having 30% or higher sequence identity with the base sequence set forth in SEQ ID NO: 1 and encoding a protein having a trypsin-binding ability; and bacteria that have a gene consisting of a base sequence set forth in SEQ ID NO: 2, or a gene having 30% or higher sequence identity with the base sequence set forth in SEQ ID NO: 2 and encoding a protein having a trypsin-binding ability.
[5] The composition for decomposition of trypsin or TMPRSS2 according to any one of [1] to [4], in which the bacteria have a type IX secretion system (T9SS).
[6] The composition for decomposition of trypsin or TMPRSS2 according to [5], in which the T9SS contains PorV protein, PorU protein, PorN protein, PorM protein, PorL protein, PorK protein, or PorP protein.
[7] The composition for decomposition of trypsin or TMPRSS2 according to any one of [1] to [6], in which the bacteria are bacteria belonging to the genus *Paraprevotella,* the genus *Prevotella,* the genus *Prevotellamasilia,* or the genus *Bacteroidetes.*
[8] The composition for decomposition of trypsin or TMPRSS2 according to [7], in which the bacteria belonging to the genus *Paraprevotella* are *Paraprevotella clara,* and the bacteria belonging to the genus *Prevotella* are at least one type of bacteria selected from the group consisting of *Prevotella rara, Prevotella rodentium,* and *Prevotella muris.*
[9] The composition for decomposition of trypsin or TMPRSS2 according to any one of [1] to [8], in which the bacteria are bacteria that have a 16S rRNA gene consisting of a base sequence set forth in SEQ ID NO: 3 or a base sequence set forth in SEQ ID NO: 4, or bacteria that have a 16S rRNA gene consisting of a base sequence having 97% or higher sequence identity with the base sequence set forth in SEQ ID NO: 3 or the base sequence set forth in SEQ ID NO: 4.
[10] The composition for decomposition of trypsin or TMPRSS2 according to any one of [1] to [6], in which the bacteria are at least one type of bacteria selected from the group consisting of *Paraprevotella* sp. MSP 0303, *Paraprevotella* sp. MSP 0335, *Prevotellamassilia timonensis, Bacteroidetes* sp. MSP 0288, *Bacteroidetes* sp. MSP 0410, *Bacteroidetes* sp. MSP 0435, and *Porphyromonas gingivalis.*
[11] The composition for decomposition of trypsin or TMPRSS2 according to any one of [1] to [10], in which the bacteria are live bacteria.
[12] The composition for decomposition of trypsin or TMPRSS2 according to any one of [1] to [10], in which the bacteria are dead bacteria.
[13] A composition for decomposition of trypsin or TMPRSS2 containing, as an active ingredient: 00502 protein, or a protein having 30% or higher sequence identity with an amino acid sequence of the 00502 protein and having a trypsin-binding ability; or 00509 protein, or a protein having 30% or higher sequence identity with an amino acid sequence of the 00509 protein and having a trypsin-binding ability.
[14] A composition for decomposition of trypsin or TMPRSS2 containing, as an active ingredient: 00502 protein, or a protein having 30% or higher sequence identity with an amino acid sequence of the 00502 protein and having a trypsin-binding ability; and 00509 protein, or a protein having 30% or higher sequence identity with an amino acid sequence of the 00509 protein and having a trypsin-binding ability.
[15] The composition for decomposition of trypsin or TMPRSS2 according to any one of [1] to [14], in which the composition for decomposition of trypsin or TMPRSS2 is for a treatment of a disease caused by trypsin or TMPRSS2.
[16] The composition for decomposition of trypsin or TMPRSS2 according to any one of [1] to [15], in which the disease caused by trypsin or TMPRSS2 is an inflammatory bowel disease (ulcerative colitis, Crohn's disease), irritable bowel syndrome, an infectious disease, acute pancreatitis, or chronic pancreatitis.
[17] The composition for decomposition of trypsin or TMPRSS2 according to [16], in which the infectious disease is a virus infectious disease or a bacterial infectious disease.
[18] The composition for decomposition of trypsin or TMPRSS2 according to [16] or [17], in which the inflammatory bowel disease, the irritable bowel syndrome, or the infectious disease is a disease associated with TMPRSS2 or IgA.
[19] A diagnostic drug for diseases caused by trypsin or TMPRSS2, the diagnostic drug containing: a specific binding substance that detects 00502 protein, or a protein having 30% or higher sequence identity with an amino acid sequence of the 00502 protein and having a trypsin-binding ability, or that detects 00509 protein, or a protein having 30% or higher sequence identity with an amino acid sequence of the 00509 protein and having a trypsin-binding ability.
[20] A diagnostic drug for diseases caused by trypsin or TMPRSS2, the diagnostic drug containing: a primer set or a probe that detects a gene consisting of a base sequence set forth in SEQ ID NO: 1, or a gene having 30% or higher sequence identity with the base sequence set forth in SEQ ID NO: 1 and encoding a protein having a trypsin-binding ability, or that detects a gene consisting of a base sequence set forth in SEQ ID NO: 2, or a gene having 30% or higher sequence identity with the base sequence set forth in SEQ ID NO: 2 and encoding a protein having a trypsin-binding ability.
[21] A quasi-drug for diseases caused by trypsin or TMPRSS2, the quasi-drug containing, as an active ingredient: bacteria that have 00502 protein, or a protein having 30% or higher sequence identity with an amino acid sequence of the 00502 protein and having a trypsin-binding ability, or that have 00509 protein, or a protein having 30% or higher sequence identity with an amino acid sequence of the 00509 protein and having a trypsin-binding ability.
[22] A quasi-drug for diseases caused by trypsin or TMPRSS2, the quasi-drug containing, as an active ingredient: 00502 protein, or a protein having 30% or higher sequence identity with an amino acid sequence of the 00502 protein and having a trypsin-binding ability; or 00509 protein, or a protein having 30% or higher sequence identity with an amino acid sequence of the 00509 protein and having a trypsin-binding ability.

### [Advantageous Effects of Invention]

According to the present invention, a technique for regulating the activity of proteases can be provided.

### [Brief Description of Drawings]

FIG. 1 is a diagram showing the results of proteomics analysis in Experimental Example 1.
FIG. 2 is a graph showing the results of a trypsin activity test on the faeces of specific-pathogen-free (SPF) mice and germ-free (GF) mice in Experimental Example 1.
FIG. 3 is a photograph showing the results of detecting an anionic trypsin protease (PRSS2) by western blot analysis of the faeces of the SPF mice and the GF mice in Experimental Example 1.
FIG. 4 is a fluorescence micrograph showing the results of detecting mucus (UEA1) and the anionic trypsin protease (PRSS2) by immunostaining of large intestine sections of the SPF mice and the GF mice in Experimental Example 1.
FIG. 5 is a graph showing the results of measuring the trypsin activity in different sites of the small intestine and the large intestine of the GF mice and the SPF mice in Experimental Example 1.
FIG. 6 is a graph showing the results of measuring the faecal trypsin activity in the GF mice to which faecal samples collected from healthy donors were administered in Experimental Example 2.
FIG. 7 is a graph showing the results of measuring the faecal trypsin activity of mice in each group in Experimental Example 2.
FIG. 8 is a photograph showing the western blot results of measuring the decomposition of trypsin in Experimental Example 2.
FIG. 9 is a photograph showing the western blot results of measuring the decomposition of human trypsin in Experimental Example 2.
FIG. 10 is a photograph showing the western blot results of measuring the trypsin-decomposing ability of various bacteria in Experimental Example 2.
FIG. 11 is a photograph showing the results of analyzing the decomposition of recombinant mouse PRSS2 (rmPRSS2) by western blot after pretreating rmPRSS2 with a protease inhibitor and then incubating with *P. clara* (1C4) in Experimental Example 3.
FIG. 12 is a photograph showing the results of observing the binding of rmPRSS2 to the surface of bacteria with a confocal microscope in Experimental Example 3.
FIG. 13 is a photograph showing the results of analyzing the decomposition of rmPRSS2 by western blot after incubating *P. clara* (1C4), which had been pretreated with tunicamycin, with rmPRSS2 in Experimental Example 3.
FIG. 14 is a photograph showing the results of observing the binding of rmPRSS2 to the surface of bacteria with a confocal microscope after incubating each of *P. clara* (1C4) subjected to a tunicamycin treatment or *P. clara* (1C4) not subjected to a tunicamycin treatment with rmPRSS2 in Experimental Example 3.
FIG. 15 is a schematic diagram showing the alignment of the genetic organization of the type IX secretion system (T9SS) in the genome of *P. clara* (JCM 14859), *P. xylaniphila* (JCM 14860), and *P. gingivalis* (ATCC 33277) in Experimental Example 3.
FIG. 16 is a schematic diagram showing homologous recombination that deletes the expression of PorU in Experimental Example 3.
FIG. 17 is a photograph showing the results of analyzing the decomposition of rmPRSS2 by western blot after incubating a mutant *P. clara* (JCM 14859) with rmPRSS2 in Experimental Example 3.
FIG. 18 is a photograph showing the results of analyzing, by western blot, the decomposition of rmPRSS2 mediated by a wild-type *P. clara* (JCM 14859) or the series of mutant *P. clara* (JCM 14859) in Experimental Example 3.
FIG. 19 is a photograph showing the results of observing the binding of rmPRSS2 to the surface of bacteria with a confocal microscope after incubating each of *P. clara* (Δ00502) and *P. clara* (Δ00509) with rmPRSS2 in Experimental Example 3.
FIG. 20 is a photograph showing the results of analyzing the decomposition of rmPRSS2 by western blot after incubating *P. clara* (Δ00502) and *P. clara* (Δ00509) with rmPRSS2 in Experimental Example 3.
FIG. 21 is a schematic diagram showing the genome sequence of *Paraprevotella* strains analyzed in Experimental Example 3.
FIG. 22 is a photograph showing the results of analyzing the decomposition of rmPRSS2 by western blot after incubating *P. clara* mutant strains, from which each of 00502 to 00509 genes had been deleted, with rmPRSS2 in Experimental Example 3.
FIG. 23 is a graph showing the results of measuring the protease activity of recombinant 00502 protein or 00509 protein by cleavage of F1TC-labeled casein in Experimental Example 4.
FIG. 24 is a photograph showing the results of analyzing the decomposition of rmPRSS2 by western blot after incubating free-form recombinant 00502 protein and 00509 protein with rmPRSS2 or incubating recombinant 00502 protein and 00509 protein, which were bound to microbeads, with rmPRSS2 in Experimental Example 4.
FIG. 25 is a photograph showing the results of observing the binding state of rmPRSS2 and protein-binding beads with a confocal microscope after incubating the recombinant 00502 protein, the recombinant 00509 protein, and bovine serum albumin (BSA), which were bound to microbeads, together with rmPRSS2 in Experimental Example 4.
FIG. 26 is a photograph showing the results of analyzing the decomposition of trypsin by western blot after incubating the cecal contents of the GF mice with a culture medium control (-) or recombinant 00502 protein bound to microbeads in Experimental Example 4.
FIG. 27 is a model of trypsin decomposition mediated by bacteria of the genus *Paraprevotella,* proposed by the inventors of the present invention.
FIG. 28 is a graph showing the results of quantitatively determining the DNA of a *P. clara* strain (wild-type (WT), Δ00502, or Δ00509) in the faeces of the GF mice inoculated with the *P. clara* strain together with 2-mix in Experimental Example 5.
FIG. 29 is a graph showing the results of measuring the faecal trypsin activity of the GF mice inoculated with the *P. clara* strain (wild-type (WT), Δ00502, or Δ00509) together with the 2-mix in Experimental Example 5.
FIG. 30 is a graph showing the results of measuring the faecal trypsin activity of the GF mice inoculated with a *P. clara* strain (wild-type (WT), Δ00502) together with 34-mix in Experimental Example 5.
FIG. 31 is a photograph showing the results of analyzing, by western blot, each protein in the faeces of the GF mice inoculated with a *P. clara* strain (wild-type (WT), Δ00502, or Δ00509) together with 2-mix in Experimental Example 5.
FIG. 32 is a photograph showing the results of analyzing, by western blot, each protein in the faeces of the GF mice, the faeces of the GF mice inoculated with a wild-type *P. clara* strain together with 2-mix, a specimen obtained by mixing the above-mentioned two types of the faeces, and a specimen obtained by adding a trypsin inhibitor (TCLK) to the specimen in which the above-mentioned two types of the faeces were mixed in Experimental Example 5.
FIG. 33 is a graph showing changes in body weight of the mice in each group after infection with *C*. *rodentium* in Experimental Example 6.
FIG. 34 (in the upper part of FIG. 34) is an image of the large intestine of each of the mice 7 days after infection with C. *rodentium* in Experimental Example 6. FIG. 34 (in the lower part of FIG. 34) is a photomicrograph showing a representative image of hematoxylin and eosin staining of cecal tissue in Experimental Example 6.
FIG. 35 is a graph showing the histological score of the cecal tissue based on hematoxylin and eosin staining in Experimental Example 6.
FIG. 36 is a photograph showing the results of analyzing each protein in faeces by western blot in Experimental Example 6.
FIG. 37 is a photograph showing the results of evaluating the aggregation effect of faecal IgA by ex vivo incubation of live *C*. *rodentium* and a faecal fluid in Experimental Example 6.
FIG. 38 is a schematic diagram showing an experimental schedule of Experimental Example 7.
FIG. 39 is a graph showing changes in body weight of the mice after infection with *C*. *rodentium* in Experimental Example 7.
FIG. 40 is a graph showing the measurement values of the CFU of *C*. *rodentium* in a cecal patch in Experimental Example 7.
FIG. 41 is a graph showing the measurement values of the CFU of *C*. *rodentium* in luminal contents in Experimental Example 7.
FIG. 42 is a photograph showing the results of analyzing each protein in cecal luminal contents by western blot in Experimental Example 7.
FIG. 43 is a schematic diagram showing an outline of a mouse hepatitis virus (MHV) infection experiment in Experimental Example 8.
FIG. 44 is a graph showing the survival curves of the mice after MHV infection in Experimental Example 8.
FIG. 45 is a graph showing the results of measuring viral titers of MHV in liver, brain, and faeces in Experimental Example 8.
FIG. 46 is a representative image showing the results of hematoxylin and eosin staining of liver tissue of the mice in each group in Experimental Example 8.
FIG. 47 is a diagram showing the homologues of genes (00502 gene and 00509 gene) associated with trypsin decomposition and the species encoding the same, which were searched with a calculator in Experimental Example 9.
FIG. 48 is a diagram showing the structure of loci of genes associated with trypsin decomposition in each bacterial species, and the sequence identity (%) with the genes of *P. clara* strains in Experimental Example 9.
FIG. 49 is a graph showing the results of measuring the trypsin activity in the faeces of patients in a non-lED control group (healthy subjects) from a Japanese cohort, an ulcerative colitis (UC) group, and a Crohn's disease (CD) group in Experimental Example 9.
FIG. 50 is a graph showing the *Paraprevotella* carriage rate in a population of patients diagnosed with ulcerative colitis (UC) and Crohn's disease (CD) from a PRISM cohort and an HMP2 cohort in Experimental Example 9.
FIG. 51 is a photograph showing the results of measuring the decomposition of trypsin by a *Prevotella rodentium* strain and a *Prevotella muris* strain in Experimental Example 9.
FIG. 52 is a photograph showing the results of measuring the trypsin-decomposing activity by a *Prevotella rara* (MSP 0081) strain in Experimental Example 9.
FIG. 53 is a schematic diagram showing an example of a fusion protein of 00502 protein or its homologue and/or 00509 protein or its homologue, and an antibody constant region.

### [Description of Embodiments]

### [Composition for decomposition of trypsin or TMPRSS2]

In one embodiment, the present invention provides a composition for decomposition of trypsin or TMPRSS2 containing, as an active ingredient: bacteria that have 00502 protein, or a protein having 30% or higher sequence identity with an amino acid sequence of the 00502 protein and having a trypsin-binding ability; or bacteria that have 00509 protein, or a protein having 30% or higher sequence identity with an amino acid sequence of the 00509 protein and having a trypsin-binding ability.

As will be described later in Examples, the inventors of the present invention clarified that bacteria having 00502 protein or bacteria having 00509 protein adsorb trypsin or TMPRSS2 on the bacterial cell surface to decompose trypsin or TMPRSS2 by autolysis of trypsin or TMPRSS2.

Accordingly, the composition containing bacteria having 00502 protein or bacteria having 00509 protein as an active ingredient can be used for the usage of the decomposition of trypsin or TMPRSS2. Accordingly, it can be said that the composition of the present embodiment is a decomposition agent for trypsin or TMPRSS2.

The decomposition of trypsin or TMPRSS2 can be used for industrial applications or, can be used for pharmaceutical applications as will be described later.

The accession number of UniProtKB of the 00502 protein of a *Paraprevotella clara* (YIT 11840) strain (catalog number "JCM: 14859") is G5SNC9, which will be described later. In addition, the 00502 protein of the *Paraprevotella clara* (YIT 11840) strain is encoded by an HMPREF9441_00858 gene (UniProtKB). The NCBI accession number for the HMPREF9441_00858 gene is NZ_JH376591 REGION: complement (87340..91131). The amino acid sequence of the 00502 protein of the *Paraprevotella clara* (YIT 11840) strain is set forth in SEQ ID NO: 5, and the base sequence of the cDNA of the HMPREF9441_00858 gene is set forth in SEQ ID NO: 1.

In addition, the amino acid sequence of the 00502 protein of a *Paraprevotella clara* (1C4) strain is set forth in SEQ ID NO: 6, and the base sequence of the cDNA of the gene encoding the 00502 protein of the *Paraprevotella clara* (1C4) strain is set forth in SEQ ID NO: 7.

In addition, the amino acid sequence of the 00502 protein of a *Paraprevotella xylaniphila* (82A6) strain is set forth in SEQ ID NO: 8, and the base sequence of the cDNA of the gene encoding the 00502 protein of the *Paraprevotella xylaniphila* (82A6) strain is set forth in SEQ ID NO: 9.

In addition, the amino acid sequence of the 00502 protein of a *Paraprevotella xylaniphila* (YIT 11841) strain (catalog number "JCM: 14860") is set forth in SEQ ID NO: 10, and the base sequence of the cDNA of the gene encoding the 00502 protein of the *Paraprevotella xylaniphila* (YIT 11841) strain is set forth in SEQ ID NO: 11.

In addition, the amino acid sequence of the 00502 protein of a *Prevotella rara* (109) strain (catalog number "DSM: 105141") is set forth in SEQ ID NO: 12, and the base sequence of the cDNA of the gene encoding the 00502 protein of the *Prevotella rara* (109) strain is set forth in SEQ ID NO: 13.

In addition, the amino acid sequence of the 00502 protein of a *Prevotella rodentium* (PJ1A) strain (catalog number "DSM: 105243") is set forth in SEQ ID NO: 14, and the base sequence of the cDNA of the gene encoding the 00502 protein of the *Prevotella rodentium* (PJ1A) strain is set forth in SEQ ID NO: 15.

In addition, the amino acid sequence of the 00502 protein of a *Prevotella muris* (PMUR) strain (catalog number "DSM: 103722") is set forth in SEQ ID NO: 16, and the base sequence of the cDNA of the gene encoding the 00502 protein of the *Prevotella muris* (PMUR) strain is set forth in SEQ ID NO: 17.

The accession number of UniProtKB of the 00509 protein of a *Paraprevotella clara* (YIT 11840) strain (catalog number "JCM: 14859") is GSSNC1, which will be described later. In addition, the 00509 protein of the *Paraprevotella clara* (YIT 11840) strain is encoded by an HMPREF9441_00850 gene (UniProtKB). The NCBI accession number for the HMPREF9441_00850 gene is NZ_JH376591 REGION: 73848..76931. The amino acid sequence of the 00509 protein of the *Paraprevotella clara* (Y1T 11840) strain is set forth in SEQ ID NO: 18, and the base sequence of the cDNA of the HMPREF9441_00850 gene is set forth in SEQ ID NO: 2.

In addition, the amino acid sequence of the 00509 protein of a *Paraprevotella clara* (1C4) strain is set forth in SEQ ID NO: 19, and the base sequence of the cDNA of the gene encoding the 00509 protein of the *Paraprevotella clara* (1C4) strain is set forth in SEQ ID NO: 20.

In addition, the amino acid sequence of the 00509 protein of a *Paraprevotella xylaniphila* (82A6) strain is set forth in SEQ ID NO: 21, and the base sequence of the cDNA of the gene encoding the 00509 protein of the *Paraprevotella xylaniphila* (82A6) strain is set forth in SEQ ID NO: 22.

In addition, the amino acid sequence of the 00509 protein of a *Paraprevotella xylaniphila* (YIT 11841) strain (catalog number "JCM: 14860") is set forth in SEQ ID NO: 23, and the base sequence of the cDNA of the gene encoding the 00509 protein of the *Paraprevotella xylaniphila* (YIT 11841) strain is set forth in SEQ ID NO: 24.

In addition, the amino acid sequence of the 00509 protein of the *Prevotella rara* (109) strain (catalog number "DSM: 105141") is set forth in SEQ ID NO: 25, and the base sequence of the cDNA of the gene encoding the 00509 protein of the *Prevotella rara* (109) strain is set forth in SEQ ID NO: 26.

In addition, the amino acid sequence of the 00509 protein of the *Prevotella rodentium* (PJ1A) strain (catalog number "DSM: 105243") is set forth in SEQ ID NO: 27, and the base sequence of the cDNA of the gene encoding the 00509 protein of the *Prevotella rodentium* (PJ1A) strain is set forth in SEQ ID NO: 28.

As will be described later in the Examples, the composition for decomposition of trypsin or TMPRSS2 according to the present embodiment may contain, as an active ingredient, bacteria having the homologue of 00502 protein or bacteria having the homologue of 00509 protein.

Examples of proteins as the homologue of 00502 protein include proteins having 30% or higher, 40% or higher, 50% or higher, 60% or higher, 70% or higher, 80% or higher, 90% or higher, or 95% or higher sequence identity with the amino acid sequence set forth in SEQ ID NO: 5, and having a trypsin-binding ability. It is preferable that the bacteria having the homologue of 00502 protein have a trypsin-binding ability or TMPRSS2-binding ability and further have an activity of decomposing trypsin or TMPRSS2.

Examples of the homologue of 00509 protein include proteins having 30% or higher, 40% or higher, 50% or higher, 60% or higher, 70% or higher, 80% or higher, 90% or higher, or 95% or higher sequence identity with the amino acid sequence set forth in SEQ ID NO: 18, and having a trypsin-binding ability. It is preferable that the bacteria having the homologue of 00509 protein have a trypsin-binding ability or TMPRSS2-binding ability and further have an activity of decomposing trypsin or TMPRSS2.

In the composition for decomposition of trypsin or TMPRSS2 according to the present embodiment, the phrase "containing as an active ingredient" means that bacteria having 00502 protein or the homologue of the 00502 protein or bacteria having 00509 protein or the homologue of the 00509 protein are contained in an amount sufficient to decompose trypsin or TMPRSS2. Alternatively, the phrase means that bacteria having 00502 protein or the homologue of the 00502 protein or bacteria having 00509 protein or the homologue of the 00509 protein are contained as a major active ingredient.

In the composition for decomposition of trypsin or TMPRSS2 according to the present embodiment, the bacteria having 00502 protein or the homologue of the 00502 protein mean bacteria expressing the 00502 protein or the homologue of the 00502 protein.

The bacteria expressing 00502 protein may be bacteria having the HMPREF9441_00858 gene (SEQ ID NO: 1) encoding the 00502 protein. In addition, the bacteria having the homologue of the 00502 protein may be bacteria having a gene having 30% or higher, 40% or higher, 50% or higher, 60% or higher, 70% or higher, 80% or higher, 90% or higher, or 95% or higher sequence identity with the base sequence set forth in SEQ ID NO: 1 and encoding the cDNA encoding a protein having a trypsin-binding ability. The 00502 protein may be derived from a gene endogenous to bacteria or may be derived from an exogenous gene.

Similarly, the bacteria having 00509 protein or the homologue of the 00509 protein mean bacteria expressing the 00509 protein or the homologue of the 00509 protein.

The bacteria expressing 00509 protein may be bacteria having the HMPREF9441_00850 gene (SEQ ID NO: 2) encoding the 00509 protein. In addition, the bacteria having the homologue of the 00509 protein may be bacteria having a gene having 30% or higher, 40% or higher, 50% or higher, 60% or higher, 70% or higher, 80% or higher, 90% or higher, or 95% or higher sequence identity with the base sequence set forth in SEQ ID NO: 2 and encoding the cDNA encoding a protein having a trypsin-binding ability. The 00509 protein may be derived from a gene endogenous to bacteria or may be derived from an exogenous gene.

As long as the composition for decomposition of trypsin or TMPRSS2 according to the present embodiment has the activity of decomposing trypsin or TMPRSS2, the composition may contain only bacteria having 00502 protein or the homologue of the 00502 protein, may contain only bacteria having 00509 protein or the homologue of the 00509 protein, or may contain both the bacteria having 00502 protein or the homologue of the 00502 protein and the bacteria having 00509 protein or the homologue of the 00509 protein. Alternatively, the composition for decomposition of trypsin or TMPRSS2 according to the present embodiment may contain bacteria having 00502 protein or the homologue of the 00502 protein and having 00509 protein or the homologue of the 00509 protein. Such bacteria can be produced by genetic modification or the like.

In the composition for decomposition of trypsin or TMPRSS2 according to the present embodiment, it is preferable that bacteria having 00502 protein or the homologue of the 00502 protein or bacteria having 00509 protein or the homologue of the 00509 protein have a type IX secretion system (T9SS).

Furthermore, the T9SS preferably contains PorV protein, PorU protein, PorN protein, PorM protein, PorL protein, PorK protein, or PorP protein.

As will be described later in the Examples, the inventors of the present invention clarified that 00502 protein or the homologue of the 00502 protein, or 00509 protein or the homologue of the 00509 protein is transported by T9SS to the bacterial surface across the outer membrane, thereby binding to the bacterial surface.

For example, in a *Paraprevotella clara* strain to be described later, the NCBI accession number for PorV protein is WP_008622445.1, the NCBI accession number for PorU protein is WP_008622443.1, the NCBI accession number for PorN protein is WP_008623210.1, the NCBI accession number for PorM protein is WP_008623211.1, the NCBI accession number for PorL protein is WP_008623213.1, the NCBI accession number for PorK protein is WP_008623215.1, and the NCBI accession number for PorP protein is WP_008623217.1. In addition, in bacteria other than the *Paraprevotella clara* strain, the homologues of these proteins in the bacteria constitute T9SS.

In the composition for decomposition of trypsin or TMPRSS2 according to the present embodiment, bacteria having 00502 protein or the homologue of the 00502 protein or having 00509 protein or the homologue of the 00509 protein may be bacteria belonging to the genus *Paraprevotella,* the genus *Prevotella,* the genus *Prevotellamasilia,* or the genus *Bacteroidetes.*

As will be described later in the Examples, the inventors of the present invention clarified that bacteria belonging to the genus *Paraprevotella,* the genus *Prevotella,* the genus *Prevotellamasilia,* or the genus *Bacteroidetes* decompose trypsin or TMPRSS2. Examples of bacteria belonging to the genus *Paraprevotella* include *Paraprevotella clara, Paraprevotella xylaniphila, Paraprevotella* sp. MSP 0303, and *Paraprevotella* sp. MSP 0335. Examples of bacteria belonging to the genus *Prevotella* include *Prevotella rara, Prevotella rodentium,* and *Prevotella muris.*

The bacteria belonging to the genus *Paraprevotella* are bacteria which have a 16S rRNA gene consisting of the base sequence set forth in SEQ ID NO: 3 or the base sequence set forth in SEQ ID NO: 4, or have a 16S rRNA gene consisting of a base sequence having 97% or higher sequence identity with the base sequence set forth in SEQ ID NO: 3 or the base sequence set forth in SEQ ID NO: 4. In addition, when the sequence identity of the 16S rRNA gene is 97% or higher, the bacteria are determined to belong to the same species.

The base sequence set forth in SEQ ID NO: 3 is the base sequence of the 16S rRNA gene of a *Paraprevotella clara* (YIT 11840) strain (catalog number "JCM: 14859"), which will be described later. In addition, the base sequence set forth in SEQ ID NO: 29 is the base sequence of the 16S rRNA gene of a *Paraprevotella clara* (1C4) strain. In addition, the base sequence set forth in SEQ ID NO: 4 is the base sequence of the 16S rRNA gene of a *Paraprevotella xylaniphila* (82A6) strain, which will be described later. In addition, the base sequence set forth in SEQ ID NO: 30 is the base sequence of the 16S rRNA gene of a *Paraprevotella xylaniphila* (YIT 11841) strain (catalog number "JCM: 14860").

In the composition for decomposition of trypsin or TMPRSS2 according to the present embodiment, bacteria having 00502 protein or the homologue of the 00502 protein or having 00509 protein or the homologue of the 00509 protein may be at least one type of bacteria selected from the group consisting of *Paraprevotella* sp. MSP 0303, *Paraprevotella* sp. MSP 0335, *Prevotellamassilia timonensis, Bacteroidetes* sp. MSP 0288, *Bacteroidetes* sp. MSP 0410, *Bacteroidetes* sp. MSP 0435, and *Porphyromonas gingivalis.*

Examples of *Prevotella rara* include a *Prevotella rara* (MSP 0081) strain and a *Prevotella rara* (109) strain. Examples of *Prevotella rodentium* include a *Prevotella rodentium* (PJ1A) strain (catalog number "DSM: 105243"). Examples of *Prevotella muris* include *Prevotella muris* (PMUR) strain (catalog number "DSM: 103722"). Examples of *Prevotellamassilia timonensis* include a *Prevotellamassilia timonensis* (MSP 0224) strain. Examples of *Porphyromonas gingivalis* include a *Porphyromonas gingivalis* (ATCC 33277) strain.

As will be described below in the Examples, the inventors of the present invention clarified that these bacteria decompose trypsin or TMPRSS2.

The base sequence set forth in SEQ ID NO: 31 is the base sequence of the 16S rRNA gene of the *Prevotella rara* (109) strain (catalog number "DSM: 105141"). In addition, the base sequence set forth in SEQ ID NO: 32 is the base sequence of the 16S rRNA gene of the *Prevotella rodentium* (PJ1A) strain (catalog number "DSM: 105243"). In addition, the base sequence set forth in SEQ ID NO: 33 is the base sequence of the 16S rRNA gene of the *Prevotella muris* (PMUR) strain (catalog number "DSM: 103722").

In the composition for decomposition of trypsin or TMPRSS2 according to the present embodiment, the above-mentioned bacteria may be live bacteria or dead bacteria as long as they have the activity of decomposing trypsin or TMPRSS2.

For example, the composition for decomposition of trypsin or TMPRSS2 according to the present embodiment may contain the above-mentioned bacteria in a powder form, a freeze-dried form, or the like, which are embedded in an aqueous form such as a solution or a suspension or a semi-solid form. In one aspect, the composition or the bacteria are freeze-dried. In one aspect, a subset of the bacteria in the composition is freeze-dried. A method of freeze-drying the composition containing the bacteria is well known in the technical field. For example, United States Patent No. 3261761, United States Patent No. 4205132, and PCT International Publication No. WO2012/098358 can be referred to. These documents are incorporated in the present specification by reference.

The bacteria may be freeze-dried as a combination or may be separately freeze-dried and combined. The bacteria may be combined with a pharmaceutically acceptable carrier before being combined with the other bacteria. A plurality of the freeze-dried bacteria may be combined in a freeze-dried form. A mixture of the bacteria combined once may be subsequently combined with a pharmaceutically acceptable carrier. In one aspect, the bacteria are a solid substance that has been freeze-dried. In one aspect, the composition is a solid substance that has been freeze-dried.

In one embodiment, the present invention provides a composition for decomposition of trypsin or TMPRSS2 containing, as an active ingredient: 00502 protein, or a protein having 30% or higher sequence identity with an amino acid sequence of the 00502 protein and having a trypsin-binding ability; or 00509 protein, or a protein having 30% or higher sequence identity with an amino acid sequence of the 00509 protein and having a trypsin-binding ability.

As will be described below in the Examples, the inventors of the present invention clarified that the 00502 protein or the 00509 protein decomposes trypsin or TMPRSS2. It is preferable that the 00502 protein or the 00509 protein be immobilized on a solid phase surface.

The solid phase is not particularly limited, and examples thereof include particles made of resin, glass, metal, or the like; surfaces of containers such as plates and tubes; and films made of resin. The particles may be magnetic particles.

The solid phase may be a pharmaceutically acceptable solid phase. Examples of the pharmaceutically acceptable solid phase include liposomes; high-molecular-weight nanoparticles such as protein nanoparticles and lipid nanoparticles; iron nanoparticles; nanoemulsions such as lipid microspheres; micelles; vaccine adjuvants; and nanocrystals. The pharmaceutically acceptable solid phase is preferably one approved by the Pharmaceuticals and Medical Devices Agency (PMDA, an independent administrative institution), the United States Food and Drug Administration (FDA), and the European Medicines Agency (EMA).

A method of immobilizing the 00502 protein or the 00509 protein on a solid phase is not particularly limited, and examples thereof include bonding with a chemical linker, bonding with avidin-biotin, and physical adsorption.

As will be described later in the Examples, the composition for decomposition of trypsin or TMPRSS2 according to the present embodiment may contain, as an active ingredient, the homologue of the 00502 protein or the homologue of the 00509 protein.

Examples of the homologue of the 00502 protein are the same as those described above and include proteins having 30% or higher, 40% or higher, 50% or higher, 60% or higher, 70% or higher, 80% or higher, 90% or higher, or 95% or higher sequence identity with the amino acid sequence set forth in SEQ ID NO: 5, and having a trypsin-binding ability. It is preferable that the homologue of the 00502 protein have a trypsin-binding ability or TMPRSS2-binding ability.

Examples of the homologue of the 00509 protein are the same as those described above and include proteins having 30% or higher, 40% or higher, 50% or higher, 60% or higher, 70% or higher, 80% or higher, 90% or higher, or 95% or higher sequence identity with the amino acid sequence set forth in SEQ ID NO: 18, and having a trypsin-binding ability. It is preferable that the homologue of the 00509 protein have a trypsin-binding ability or TMPRSS2-binding ability.

In the composition for decomposition of trypsin or TMPRSS2 according to the present embodiment, the phrase "containing as an active ingredient" means that the 00502 protein or the homologue of the 00502 protein or the 00509 protein or the homologue of the 00509 protein is contained in an amount sufficient to decompose trypsin or TMPRSS2. Alternatively, the phrase means that the 00502 protein or the homologue of the 00502 protein or the 00509 protein or the homologue of the 00509 protein is contained as a major active ingredient.

The 00502 protein or the homologue of the 00502 protein, or the 00509 protein or the homologue of the 00509 protein may be added with a peptide tag for protein purification, for protein detection, or for binding to a solid phase. The peptide tag is not particularly limited, and examples thereof include a histidine tag, a FLAG tag, and a MYC tag.

As long as the composition for decomposition of trypsin or TMPRSS2 according to the present embodiment has the activity of decomposing trypsin or TMPRSS2, the composition may contain only the 00502 protein or the homologue of the 00502 protein, may contain only the 00509 protein or the homologue of the 00509 protein, or may contain both the 00502 protein or the homologue of the 00502 protein and the 00509 protein or the homologue of the 00509 protein.

When the composition for decomposition of trypsin or TMPRSS2 according to the present embodiment contains both the 00502 protein or its homologue and the 00509 protein or its homologue, the 00502 protein or its homologue and the 00509 protein or its homologue may be bound.

The 00502 protein or its homologue and the 00509 protein or its homologue may linearly bound or may be cyclically bound. In addition, the 00502 protein or its homologue and the 00509 protein or its homologue may be directly bound or may be bound by a linker. The linker is not particularly limited, and examples thereof include a peptide consisting of an amino acid sequence in which GGGGS (SEQ ID NO: 34) is repeated 1 to 4 times.

The 00502 protein or its homologue and/or the 00509 protein or its homologue may be in the form of a fusion protein with an antibody constant region. The antibody constant region may be a constant region derived from a human antibody or may be a constant region derived from a human IgG-type antibody.

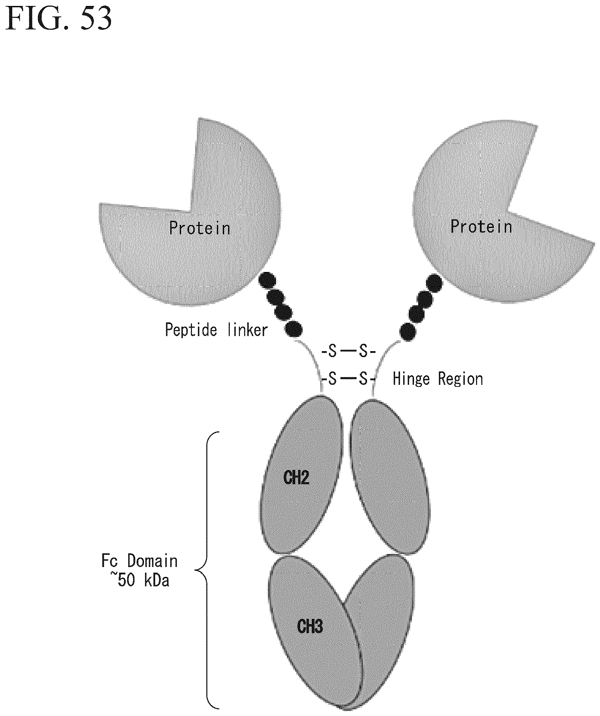

FIG. 53 is a schematic diagram showing an example of the fusion protein of the 00502 protein or its homologue and/or the 00509 protein or its homologue, and the antibody constant region. In FIG. 53, "Protein" indicates the 00502 protein or its homologue or the 00509 protein or its homologue, "CH2" indicates an antibody constant region CH2 domain, and "CH3" indicates an antibody constant region CH3 domain.

As shown in FIG. 53, the 00502 protein or its homologue and/or the 00509 protein or its homologue may be bound to the antibody constant region by a linker. The linker is not particularly limited, and examples thereof include a peptide consisting of an amino acid sequence in which GGGGS (SEQ ID NO: 34) is repeated 1 to 4 times.

### [Pharmaceutical composition for treatment of diseases caused by trypsin or TMPRSS2]

In one embodiment, the above-mentioned composition for decomposition of trypsin or TMPRSS2 may be used for a treatment of diseases caused by trypsin or TMPRSS2. In other words, in one embodiment, the present invention provides a pharmaceutical composition for a treatment of diseases caused by trypsin or TMPRSS2. The pharmaceutical composition of the present embodiment contains, as an active ingredient, bacteria having 00502 protein or the homologue of the 00502 protein, bacteria having 00509 protein or the homologue of the 00509 protein, the 00502 protein or the homologue of the 00502 protein, or the 00509 protein or the homologue of the 00509 protein.

In the pharmaceutical composition of the present embodiment, the 00502 protein, the homologue of the 00502 protein, the 00509 protein, the homologue of the 00509 protein, and the bacteria having these are the same as those described above.

In the pharmaceutical composition of the present embodiment, examples of the diseases caused by trypsin or TMPRSS2 include an inflammatory bowel disease (ulcerative colitis, Crohn's disease), irritable bowel syndrome, acute pancreatitis, and chronic pancreatitis.

Alternatively, in the pharmaceutical composition of the present embodiment, examples of the diseases caused by trypsin or TMPRSS2 include infectious diseases. Examples of the infectious diseases include a virus infectious disease and a bacterial infectious disease. In addition, examples of the inflammatory bowel disease, the irritable bowel syndrome, or the infectious diseases mentioned above include diseases associated with TMPRSS2 or IgA. Examples of infectious diseases in which infection is associated with TMPRSS2 include coronavirus infectious diseases. Examples of infectious diseases associated with IgA include *Salmonella* infectious disease.

The pharmaceutical composition of the present embodiment may be formulated together with a pharmaceutically acceptable carrier. As the pharmaceutically acceptable carrier, one that is used for the formulation of a common pharmaceutical composition can be used without particular limitation. More specific examples thereof include binding agents such as gelatin, corn starch, gum tragacanth, and gum arabic; excipients such as starch and crystalline cellulose; and swelling agents such as alginic acid.

The pharmaceutical composition of the present embodiment may further contain additives. Examples of the additives include lubricants such as calcium stearate and magnesium stearate; sweetening agents such as sucrose, lactose, saccharin, and maltitol; flavoring agents such as peppermint and *Gaultheria adenothrix* oil; stabilizers such as benzyl alcohol and phenol; and buffering agents such as a phosphoric acid salt and sodium acetate.

The pharmaceutical composition of the present embodiment can be formulated by appropriately combining the above-mentioned carriers and additives, and admixing them in a unit dosage form that is required for commonly accepted pharmaceutical practice.

A method of administering the pharmaceutical composition of the present embodiment is not particularly limited and may be appropriately determined depending on the symptom, the body weight, the age, the gender, and the like of patients. Examples thereof include tablets, powders, capsules, liquid medications, enemas, and suppositories. Tablets, powders, capsules, and liquid medications are orally administered. Enemas and suppositories are intestinally administered. The pharmaceutical composition is preferably in the dosage form capable of delivering the active ingredient (bacteria having 00502 protein or the homologue of the 00502 protein, bacteria having 00509 protein or the homologue of the 00509 protein, the 00502 protein or the homologue of the 00502 protein, or the 00509 protein or the homologue of the 00509 protein) to the intestine.

The dose of the pharmaceutical composition varies depending on the symptom, the body weight, the age, the gender, and the like of patients and cannot be unconditionally determined, but when the active ingredient is live bacteria, it is considered that the active ingredient is administered one time to several times at a dose of 0.1 to 100 mg/kg body weight per dosage unit form, for example. In addition, when the active ingredient is dead bacteria or protein, it is considered that the active ingredient is administered one time to several times per day at a dose of 0.1 to 100 mg/kg body weight per dosage unit form, for example.

The pharmaceutical composition of the present embodiment may be a quasi-drug. The quasi-drug is a product with a specifically recognized efficacy or effect and has a mild action on the human body. Examples of the quasi-drug of the present embodiment include, but are not limited to, forms such as drink agents, stomach medicines, and intestinal drugs.

### [Diagnostic drug for diseases caused by trypsin or TMPRSS2]

In one embodiment, the present invention provides a diagnostic drug for diseases caused by trypsin or TMPRSS2, the diagnostic drug containing: a specific binding substance that detects 00502 protein, or a protein having 30% or higher sequence identity with an amino acid sequence of the 00502 protein and having a trypsin-binding ability, or that detects 00509 protein, or a protein having 30% or higher sequence identity with an amino acid sequence of the 00509 protein and having a trypsin-binding ability.

In other words, the diagnostic drug of the present embodiment contains a specific binding substance that detects the 00502 protein, the homologue of the 00502 protein, the 00509 protein, and the homologue of the 00509 protein at a protein level.

Examples of the specific binding substance include antibodies, antibody fragments, and aptamers. Examples of the antibody fragments include Fab, F(ab')₂, Fab', and single chain antibodies (scFv). An antibody may be a monoclonal antibody or a polyclonal antibody. A commercially available antibody may also be used.

In the diagnostic drug of the present embodiment, the 00502 protein, the homologue of the 00502 protein, the 00509 protein, the homologue of the 00509 protein, and the bacteria having these are the same as those described above. Furthermore, the same applies to the diseases caused by trypsin or TMPRSS2 as described above.

By using the diagnostic drug of the present embodiment, the presence or absence of the 00502 protein, the homologue of the 00502 protein, the 00509 protein, the homologue of the 00509 protein, or the bacteria having these can be detected from a biological specimen derived from a subject.

The detection principle by the diagnostic drug of the present embodiment is not particularly limited, and examples thereof include an enzyme-linked immunosorbent assay (ELISA) method, a lateral flow immunoassay, a western blot, and a flow cytometry (FACS).

Examples of the biological specimen derived from the subject include faeces specimens. When the presence of the 00502 protein, the homologue of the 00502 protein, the 00509 protein, the homologue of the 00509 protein, or the bacteria having these is detected from a biological specimen derived from a subject by using the diagnostic drug of the present embodiment, it can be determined that the subject has not developed the diseases caused by trypsin or TMPRSS2.

When the presence of the 00502 protein, the homologue of the 00502 protein, the 00509 protein, the homologue of the 00509 protein, or the bacteria having these is not detected from a biological specimen derived from a subject, it can be determined that there is a possibility that the subject has developed the diseases caused by trypsin or TMPRSS2. In this case, the symptom of the diseases caused by trypsin or TMPRSS2 can be treated or alleviated by administering the above-mentioned pharmaceutical composition or quasi-drug to the subject.

In one embodiment, the diagnostic drug of the present embodiment may detect the 00502 protein, the homologue of the 00502 protein, the 00509 protein, and the homologue of the 00509 protein at a gene level.

In other words, in one embodiment, the present invention provides a diagnostic drug for diseases caused by trypsin or TMPRSS2, the diagnostic drug containing: a primer set or a probe that detects a HMPREF9441_00858 gene encoding 00502 protein, or a gene having 30% or higher sequence identity with the base sequence of the HMPREF9441_00858 gene and encoding a protein having a trypsin-binding ability, or that detects a HMPREF9441_00850 gene encoding 00509 protein, or a gene having 30% or higher sequence identity with the base sequence of the HMPREF9441_00850 gene and encoding a protein having a trypsin-binding ability. In other words, the diagnostic drug of the present embodiment contains: a primer set or a probe that detects a gene consisting of a base sequence set forth in SEQ ID NO: 1, or a gene having 30% or higher sequence identity with the base sequence set forth in SEQ ID NO: 1 and encoding a protein having a trypsin-binding ability, or that detects a gene consisting of a base sequence set forth in SEQ ID NO: 2, or a gene having 30% or higher sequence identity with the base sequence set forth in SEQ ID NO: 2 and encoding a protein having a trypsin-binding ability.

The HMPREF9441_00858 gene and the HMPREF9441_00850 gene are the same as those described above.

The gene having 30% or higher sequence identity with the base sequence of the HMPREF9441_00858 gene and encoding a protein having a trypsin-binding ability is a gene encoding the homologue of the 00502 protein described above.

Furthermore, the gene having 30% or higher sequence identity with the base sequence of the HMPREF9441_00850 gene and encoding a protein having a trypsin-binding ability is a gene encoding the homologue of the 00509 protein described above.

By using the diagnostic drug of the present embodiment, the presence or absence of bacteria having the 00502 protein, bacteria having the homologue of the 00502 protein, bacteria having the 00509 protein, and bacteria having the homologue of the 00509 protein can be detected from a biological specimen derived from a subject.

The detection principle by the diagnostic drug of the present embodiment is not particularly limited, and examples thereof include PCR, RNA sequencing (RNA-Seq), metagenomics analysis, and DNA microarray analysis.

The biological specimen derived from the subject is the same as that described above, and examples thereof include faeces specimens. When the presence of bacteria having the 00502 protein, bacteria having the homologue of the 00502 protein, bacteria having the 00509 protein, and bacteria having the homologue of the 00509 protein is detected from a biological specimen derived from a subject by using the diagnostic drug of the present embodiment, it can be determined that the subject has not developed the diseases caused by trypsin or TMPRSS2.

When the presence of bacteria having the 00502 protein, bacteria having the homologue of the 00502 protein, bacteria having the 00509 protein, and bacteria having the homologue of the 00509 protein is not detected from a biological specimen derived from a subject, it can be determined that there is a possibility that the subject has developed the diseases caused by trypsin or TMPRSS2.

In this case, the symptom of the diseases caused by trypsin or TMPRSS2 can be treated or alleviated by administering the above-mentioned pharmaceutical composition or quasi-drug to the subject.

### [Other embodiments]

In one embodiment, the present invention provides a method for treating diseases caused by trypsin or TMPRSS2, the method including: a step of administering, to a patient in need of treatment, an effective amount of bacteria that have 00502 protein, or a protein having 30% or higher sequence identity with an amino acid sequence of the 00502 protein and having a trypsin-binding ability, or an effective amount of bacteria that have 00509 protein, or a protein having 30% or higher sequence identity with an amino acid sequence of the 00509 protein and having a trypsin-binding ability.

In one embodiment, the present invention provides a method for treating diseases caused by trypsin or TMPRSS2, the method including: a step of administering, to a patient in need of treatment, an effective amount of bacteria that have a gene consisting of a base sequence set forth in SEQ ID NO: 1, or a gene having 30% or higher sequence identity with the base sequence set forth in SEQ ID NO: 1 and encoding a protein having a trypsin-binding ability, or an effective amount of bacteria that have a gene consisting of a base sequence set forth in SEQ ID NO: 2, or a gene having 30% or higher sequence identity with the base sequence set forth in SEQ ID NO: 2 and encoding a protein having a trypsin-binding ability.

In one embodiment, the present invention provides a method for treating diseases caused by trypsin or TMPRSS2, the method including: a step of administering, to a patient in need of treatment, an effective amount of 00502 protein, or a protein having 30% or higher sequence identity with an amino acid sequence of the 00502 protein and having a trypsin-binding ability, or an effective amount of 00509 protein, or a protein having 30% or higher sequence identity with an amino acid sequence of the 00509 protein and having a trypsin-binding ability.

In one embodiment, the present invention provides a method for diagnosing and treating diseases caused by trypsin or TMPRSS2, the method including: a step of detecting the presence or absence of the 00502 protein, the homologue of the 00502 protein, the 00509 protein, and the homologue of the 00509 protein from a biological specimen derived from a subject and indicating that the subject has developed the disease caused by trypsin or TMPRSS2 when the presence of the protein or the homologue is not detected; and a step of administering, to the subject when the presence of the protein or the homologue is not detected, an effective amount of bacteria that have 00502 protein, or a protein having 30% or higher sequence identity with an amino acid sequence of the 00502 protein and having a trypsin-binding ability, an effective amount of bacteria that have 00509 protein, or a protein having 30% or higher sequence identity with an amino acid sequence of the 00509 protein and having a trypsin-binding ability, an effective amount of the 00502 protein, or a protein having 30% or higher sequence identity with an amino acid sequence of the 00502 protein and having a trypsin-binding ability, or an effective amount of the 00509 protein, or a protein having 30% or higher sequence identity with an amino acid sequence of the 00509 protein and having a trypsin-binding ability.

In one embodiment, the present invention provides a method for diagnosing and treating diseases caused by trypsin or TMPRSS2, the method including: a step of detecting the presence or absence of bacteria having 00502 protein, bacteria having the homologue of the 00502 protein, bacteria having a gene consisting of a base sequence set forth in SEQ ID NO: 1, bacteria having a gene having 30% or higher sequence identity with the base sequence set forth in SEQ ID NO: 1 and encoding a protein having a trypsin-binding ability, bacteria having 00509 protein, bacteria having the homologue of the 00509 protein, bacteria having a gene consisting of a base sequence set forth in SEQ ID NO: 2, or bacteria having a gene having 30% or higher sequence identity with the base sequence set forth in SEQ ID NO: 2 and encoding a protein having a trypsin-binding ability from a biological specimen derived from a subject, and indicating that the subject has developed the disease caused by trypsin or TMPRSS2 when the presence of the bacteria having the protein, the homologue or the gene is not detected; and a step of administering, to the subject when the presence of the protein, the homologue or the bacteria having the gene is not detected, an effective amount of bacteria that have the 00502 protein, or a protein having 30% or higher sequence identity with an amino acid sequence of the 00502 protein and having a trypsin-binding ability, an effective amount of bacteria having a gene consisting of the base sequence set forth in SEQ ID NO: 1, an effective amount of bacteria having a gene having 30% or higher sequence identity with the base sequence set forth in SEQ ID NO: 1 and encoding a protein having a trypsin-binding ability, an effective amount of bacteria that have the 00509 protein, or a protein having 30% or higher sequence identity with an amino acid sequence of the 00509 protein and having a trypsin-binding ability, an effective amount of bacteria having a gene consisting of the base sequence set forth in SEQ ID NO: 2, an effective amount of bacteria having a gene having 30% or higher sequence identity with the base sequence set forth in SEQ ID NO: 2 and encoding a protein having a trypsin-binding ability, an effective amount of the 00502 protein, or a protein having 30% or higher sequence identity with an amino acid sequence of the 00502 protein and having a trypsin-binding ability, or an effective amount of the 00509 protein, or a protein having 30% or higher sequence identity with an amino acid sequence of the 00509 protein and having a trypsin-binding ability.

In one embodiment, the present invention provides a composition for treating diseases caused by trypsin or TMPRSS2, the composition containing, as an active ingredient: bacteria having 00502 protein; bacteria having a protein having 30% or higher sequence identity with an amino acid sequence of the 00502 protein and having a trypsin-binding ability; bacteria having a gene consisting of a base sequence set forth in SEQ ID NO: 1; bacteria having a gene having 30% or higher sequence identity with the base sequence set forth in SEQ ID NO: 1 and encoding a protein having a trypsin-binding ability; bacteria having 00509 protein; bacteria having a protein having 30% or higher sequence identity with an amino acid sequence of the 00509 protein and having a trypsin-binding ability; bacteria having a gene consisting of a base sequence set forth in SEQ ID NO: 2; or bacteria having a gene having 30% or higher sequence identity with the base sequence set forth in SEQ ID NO: 2 and encoding a protein having a trypsin-binding ability.

In one embodiment, the present invention provides a pharmaceutical composition for treating diseases caused by trypsin or TMPRSS2, the pharmaceutical composition containing, as an active ingredient: 00502 protein; a protein having 30% or higher sequence identity with an amino acid sequence of the 00502 protein and having a trypsin-binding ability; 00509 protein; or a protein having 30% or higher sequence identity with an amino acid sequence of the 00509 protein and having a trypsin-binding ability.

In one embodiment, the present invention provides use of the following bacteria for producing a pharmaceutical composition for treating diseases caused by trypsin or TMPRSS2: bacteria having 00502 protein; bacteria having a protein having 30% or higher sequence identity with an amino acid sequence of the 00502 protein and having a trypsin-binding ability; bacteria having a gene consisting of a base sequence set forth in SEQ ID NO: 1; bacteria having a gene having 30% or higher sequence identity with the base sequence set forth in SEQ ID NO: 1 and encoding a protein having a trypsin-binding ability; bacteria having 00509 protein; bacteria having a protein having 30% or higher sequence identity with an amino acid sequence of the 00509 protein and having a trypsin-binding ability; bacteria having a gene consisting of a base sequence set forth in SEQ ID NO: 2; or bacteria having a gene having 30% or higher sequence identity with the base sequence set forth in SEQ ID NO: 2 and encoding a protein having a trypsin-binding ability.

In one embodiment, the present invention provides use of the following proteins for producing a pharmaceutical composition for treating diseases caused by trypsin or TMPRSS2: 00502 protein; a protein having 30% or higher sequence identity with an amino acid sequence of the 00502 protein and having a trypsin-binding ability; 00509 protein; or a protein having 30% or higher sequence identity with an amino acid sequence of the 00509 protein and having a trypsin-binding ability.

### [Examples]

Next, the present invention will be described in more detail with reference to examples, but the present invention is not limited to the following examples.

### [Material and method]

### (Mice)

C57BL/6 mice bred under a specific-pathogen-free (SPF) condition or a germ-free (GF) condition were purchased from Sankyo Laboratories Japan, Japan SLC, Inc., Charles River Laboratories Japan, Inc., or CLEA Japan, Inc. GF mice and gnotobiotic mice were bred and maintained in a gnotobiotic facility of the RIKEN Center for Integrative Medical Sciences. All animal experiments were approved by the Committee of Experimental Animals of the RIKEN Yokohama Branch.

### (Bacterial strain)

*Paraprevotella clara* (JCM 14859), *Paraprevotella xylaniphila* (JCM 14860), *Paraprevotella copra* (JCM 13464), *Paraprevotella denticola* (JCM 13449), *Paraprevotella stercorea* (JCM 13469), and *Paraprevotella oulorum* (JCM 14966) were obtained from the Japan Collection of Microorganisms (JCM). *Paraprevotella clara* (P237E3b) and (P322B5) were provided by Vedanta Biosciences, Inc. *Paraprevotella xylaniphila* (82A6) was isolated by the inventors of the present invention.

### (Proteome analysis of cecal contents)

The protein was extracted from cecal contents by pipetting and mixing with tris buffered saline with Tween-20 (TBST) containing a protease inhibitor. Subsequently, insoluble matter was removed by centrifugation at 15,000 × g for 20 minutes at 4°C. Thereafter, the supernatant was transferred to a new tube, 25% trichloroacetic acid (final concentration: 12.5% v/v) was added thereto, and incubation was performed at 4°C for 1 hour. Subsequently, the supernatant was removed by centrifugation at 15,000 × g for 15 minutes at 4°C. Thereafter, the precipitate was washed with acetone twice, and the lid was opened for drying. Subsequently, the dried specimen was redissolved in 0.5% sodium dodecanoate and 100 mM Tris-HCl (pH 8.5) using a water bath type sonicator (Bioruptor UCD-200, Sonic Bio Corp.). The protein concentration of the redissolved sample was measured by a bicinchoninic acid (BCA) assay, and the protein concentration was adjusted to 1 µg/µL. A pretreatment for shotgun proteome analysis was performed according to the description previously reported (Kawashima Y., et al., Optimization of Data-Independent Acquisition Mass Spectrometry for Deep and Highly Sensitive Proteomic Analysis, Int J Mol Sci., 20 (23), 5932, 2019.).

Peptides were directly injected into a 75 µm × 15 cm PicoFrit emitter (New Objective) filled in-house with 2.7 µm core-shell C18 particles (CAPCELL CORE MP 2.7 µm, 160 Å material, Osaka Soda Co., Ltd.), and separation was performed by applying a gradient for 180 minutes at a flow velocity of 300 nL/minute using Eksigent ekspert nanoLC 400 HPLC system (Sciex Corporation).

The peptides eluted from the column were subjected to shotgun MS and SWATH-MS analysis (SWATH: Sequential Window Acquisition of All Theoretical Mass spectra) using a TripleTOF 5600+ mass spectrometer (Sciex Corporation). In the experiment using the shotgun MS, an MS1 spectrum was acquired for 250 milliseconds at a range of 400 to 1000 m/z. The top 25 precursor ions exceeding 150 counts/second with a charge state of 2+ to 5+ were selected, and fragmentation was performed by a rolling collision energy to acquire an MS2 spectrum for 100 milliseconds at a range of 100 to 1500 m/z. The dynamic exclusion time was set to 24 seconds.

In the experiment using the SWATH-MS, a mass spectrometer was operated in a continuous data-independent acquisition mode, and a range was increased by 12 m/z with precursor isolation windows. Using an isolation width of 13 m/z (1 m/z for overlapping windows), a set of 50 windows covering a precursor mass range of 400 to 1000 m/z was constructed. A SWATH MS2 spectrum was performed for 60 milliseconds at a range of 100 to 1500 m/z for each MS2 experiment. In each MS2 experiment, the rolling collision energy was used for fragmentation of precursor ions.

All shotgun MS files were cross-checked against the mouse UniProt reference proteomes of mice (Uniprot id. UP000000589, reviewed, canonical) using ProteinPilot Software v. 4.5 and the Paragon Algorithm (Sciex Corporation) to perform protein identification.

The threshold value of the reliability of the protein was such that an unused score of ProteinPilot was 1.3, and at least one peptide had a reliability of 95%. In the present study, the global false discovery rates for both peptides and proteins were less than 1%. The identified proteins were quantitatively determined from SWATH-MS data using PeakView v. 2.2 (Sciex Corporation).

### (Proteome analysis of culture supernatant of P. clara)

25% trichloroacetic acid (final concentration: 12.5% v/v) was added to the culture supernatant of *P. clara,* and incubation was performed at 4°C for 1 hour. Subsequently, the supernatant was removed by centrifugation at 15,000 × g for 15 minutes at 4°C. Thereafter, the precipitate was washed with acetone twice, and the lid was opened for drying. Subsequently, the dried specimen was redissolved in 0.5% sodium dodecanoate and 100 mM Tris-HCl (pH 8.5) using a water bath type sonicator (Bioruptor UCD-200). The protein concentration of the redissolved sample was measured by a BCA assay, and the protein concentration was adjusted to 1 µg/µL. The pretreatment for the shotgun proteome analysis was performed as described above.

Peptides were directly injected at 50°C into a 75 µm × 20 cm PicoFrit emitter filled in-house with 2.7 µm core-shell C18 particles, and thereafter separation was performed by applying a gradient for 80 minutes at a flow velocity of 100 nL/minute using UltiMate 3000 RSLCnano LC system (Thermo Fisher Scientific K.K.). The peptides eluted from the column were analyzed by overlapping window DIA-MS using Q Exactive HF-X (Thermo Fisher Scientific K.K.). An MS1 spectrum was acquired in a range of 495 to 785 m/z with a resolution of 30,000. The target value of automatic gain control was set at 3 × e⁶, and a maximum injection time was set to 55.

An MS2 spectrum was acquired at a range of 200 m/z or more with a resolution of 30,000, the target value of automatic gain control was set at 3 × e⁶, a maximum injection time was set to "auto", and stepped normalized collision energies were set to 22%, 26%, and 30 %. An isolation width of the MS2 was set to 4 m/z, and an overlapping window pattern at 500 to 780 m/z was used. The window layout was optimized by using Skyline software.

The MS files were cross-checked against the spectral library of *P. clara* by using Scaffold DIA (Proteome Software). The spectral library was generated from a protein sequence database of *P. clara* (Uniprot id. UP000000589, reviewed, canonical) by using Prosit software.

The protein sequence database of *P. clara* was independently created by metagenomics analysis. The search parameters of the Scaffold DIA were as follows. Experimental data search enzyme: trypsin, maximum number of missed cleavage sites: 1, allowable range of precursor mass: 8 ppm, allowable range of fragment mass: 8 ppm, and static modification: carbamidomethylation of cysteine.

The threshold value for protein identification was set such that the false discovery rates of peptides and proteins were 1% or less. The quantitative determination of the peptides was calculated by the EncyclopeDIA algorithm in the Scaffold DIA. For each peptide, four highest quality fragment ions were selected and quantitatively determined. The quantitative values of the proteins were estimated from the sum of the quantitative values of the peptides.

### (Peptidome analysis)

An acetonitrile (ACN) containing 0.1% trifluoroacetic acid (TFA) was added to the cecal contents and dried with a centrifugal evaporator. Acetone was added to the dried sample, and a low-molecular-weight compound that was soluble in lipids was extracted with a water bath type sonicator. Thereafter, centrifugation was performed at 15,000 × g for 15 minutes at 4°C.

After removing the supernatant, 70% ACN-HCl was added to the precipitate, and the peptides were redissolved in a water bath type sonicator. Thereafter, centrifugation was performed at 15,000 × g for 15 minutes at 4°C. Subsequently, the supernatant was transferred to a new tube and dried with a centrifugal evaporator. Subsequently, the dried sample was redissolved in 100 mM Tris-HCl containing a protease inhibitor, and then treated with 10 mM dithiothreitol at 50°C for 30 minutes. Thereafter, alkylation was carried out with 30 mM iodoacetamide for 30 minutes at room temperature in a dark place. Thereafter, acidification was carried out with 0.5% trifluoroacetic acid (final concentration). The acidified sample was desalted with MonoSpin C18 (GL Sciences Inc.).

Peptides were directly injected at 50°C into a 75 µm × 25 cm PicoFrit emitter (New Objective) filled in-house with core-shell C18 particles (CAPCELL CORE MP 2.7 µm, 160 Å, Osaka Soda Co., Ltd.), and thereafter separation was performed by applying a gradient for 90 minutes at a flow velocity of 100 nL/minute using UltiMate 3000 RSLCnano LC system (Thermo Fisher Scientific K.K.).

The peptides eluted from the column were analyzed by DDA-MS using Q Exactive HF-X (Thermo Fisher Scientific K.K.). An MS1 spectrum was acquired at a range of 350 to 1500 m/z with a resolution of 120,000 to achieve 3 × 10⁶ as the target value of automatic gain control (AGC).

The strongest 30 ions of more than 4.4 × 10³ with a charge state of 2+ to 5+ were fragmented by collision-induced dissociation with a normalized collision energy of 26% in a data-dependent mode. Tandem mass spectra were acquired with an Orbitrap mass spectrometer having a mass resolution of 30,000 at 200 m/z. The AGC target was set to 1 × 10⁵.

The MS files were searched with the mouse UniProt reference proteome (Uniprot id. UP000000589, reviewed, canonical) PEAKS Studio.

The search conditions were as follows: mass allowable range of precursor: 8 ppm, mass allowable range of fragment ions: 0.01 Da, presence or absence of enzyme, carbamidomethylation of fixed modification, and oxidation (M) of variable modification. Identification was performed by filtering such that the false discovery rate of peptides was 1 % or less.

### (Western blot)

Mouse faecal samples were suspended and diluted 50-fold with phosphate buffered saline (PBS) to which a protease inhibitor cocktail (Roche) was added. Subsequently, the suspended sample was centrifuged at 15,000 × g for 10 minutes at 4°C, and the supernatant was subjected to western blot. Mouse pancreatic tissue was rapidly frozen in liquid nitrogen. Proteins were extracted by TRIzol Reagent (Thermo Fisher Scientific K.K.), and the final protein concentration was adjusted to 4 µg/µL. For SDS-PAGE and blotting, Novex (registered trademark) NuPAGE (registered trademark) SDS-PAGE Gel system (Thermo Fisher Scientific K.K.) and iBlot^{™} 2 Dry Blotting System (Thermo Fisher Scientific K.K.) were used according to the manufacturer's instructions.

In some of the experiments, SDS-PAGE and PVDF membrane transfer (0.2 µm Transfer Membranes Immobilon-PSQ (Merck Millipore)) were performed according to the manufacturer's instructions (XV PANTERA SYSTEM (DRC)).

iBind^{™} Western Systems (Thermo Fisher Scientific K.K.) was used for staining.

The antibodies used are as follows. A rabbit anti-mouse PRSS2 antibody (Cosmo Bio Co., Ltd.), a rabbit anti-mouse HSP90 antibody (#4877, Cell Signaling Technology, Inc.), a rabbit anti-human PRSS2 antibody (LS-B15726, LSBio), a rabbit anti-human PRSS1 antibody (LS-331381, LSBio), a rabbit anti-mouse TMPRSS2 antibody (LS-C373022, LSBio, against the sequence of the protease domain), a rabbit anti-6-His antibody (A190-214A, Bethyl Laboratories, Inc.), a goat anti-mouse IgA α chain antibody (HRP) (ab97235, Abcam plc.), a rat anti-mouse κ chain antibody (HRP) (ab99632, Abcam plc.), a rabbit anti-mouse CELA3b antibody (OACD03205, Aviva Systems Biology Corporation), an anti-rabbit IgG antibody (HRP) (#7074, Cell Signaling Technology, Inc.), and a rabbit anti-mouse Reg3 antibody (51153-R005, Sino Biological, Inc.).

For a chemiluminescence assay, Chemi-Lumi One (Nacalai Tesque, Inc.) was used. For imaging, Molecular Imager (registered trademark) ChemiDoc^{™} XRS+ System (Bio-Rad Laboratories, Inc.) or iBright^{™} FL1500 was used.

### (RT-qPCR)

RNA of mouse pancreas was extracted by TRIzol Reagent (Thermo Fisher Scientific K.K.). The extracted RNA was converted to cDNA using ReverTra Ace (registered trademark) qPCR RT Master Mix with gDNA Remover (TOYOBO CO., LTD.).

RT-qPCR analysis was performed using THUNDERBIRD SYBR qPCR Mix (TOYOBO CO., LTD.) and LightCycler 480 (Roche) by a ΔΔCt method. GAPDH was used as an endogenous control. The base sequences of the primers used are as follows.
GAPDH Forward primer: 5'-GTCGTGGAGTCTACTGGTGTCTTC-3' (SEQ ID NO: 35)
GAPDH Reverse primer: 5'-GTCATATTTCTCGTGGTTCACACC-3' (SEQ ID NO: 36)
PRSS2 Forward primer: 5'-TGTGACCCTCAATGCCAGAG-3' (SEQ ID NO: 37)
PRSS2 Reverse primer: 5'-AGCACTGGGGCATCAACAC-3' (SEQ ID NO: 38)

### (Immunofluorescence staining)

Mouse large intestine tissue (including faeces) was collected and fixed in a Carnoy's solution (60% methanol, 30% chloroform, 10% glacial acetic acid) overnight at 4°C. Tissue Processor (Leica Microsystems) was used for paraffin embedding. Subsequently, a paraffin block was cut into thin sections (5.0 µm) with a microtome. Thereafter, paraffin was removed to perform immunostaining.

The antibodies used in the immunofluorescence method are as follows. A rabbit anti-PRSS2 antibody (LSBio), an Alexa 488-labeled goat anti-rabbit IgG antibody (Thermo Fisher Scientific K.K.), 4'-6-diamidino-2-phenylindole (DAPI) (DOJINDO LABORATORIES), and rhodamine-labeled UEA 1 (ULEX Europaeus Agglutinin 1, Vector Laboratories, Inc.). For immunofluorescence imaging, Leica TCS SP5 co-focused with Leica AF600 was used.

### (Measurement of trypsin activity in mice and human faecal samples)

Mouse intestinal contents or a mice faecal sample was diluted 500-fold (w/v) with a 0.9% NaCl solution. Human faeces were diluted 200-fold (w/v) with a 0.9% NaCl solution. The diluted solution was vortexed in a mini shaker for 20 minutes at 2,000 rpm, homogenized by pipetting, and then centrifuged at 10,000 × g for 15 minutes at 4°C. Subsequently, the supernatant was recovered to measure the trypsin activity by a Trypsin Activity Assay Kit (Colorimetric) for 100 tests (ab102531) according to the manufacturer's protocol. The absorbance at 405 nm was measured in a kinetic mode by using a PerkinElmer 2030 Multilabel Reader.

### (Colonization of GF mice by human microbiota)

Human faecal samples were collected at RIKEN and Keio University according to a research protocol approved by the Research Ethics Review Committee. Informed consent was obtained from each subject. A human faecal sample (preserved in 20% (v/v) glycerol) was transferred to an anaerobic chamber, thawed, and then sieved with a 100 µm mesh, transferred to a GF isolator, and orally administered to GF mice by 200 µL/mouse.

For an antibiotic treatment, each solution of 0.5 g/L ampicillin (Nacalai Tesque, Inc.), 0.5 g/L metronidazole (Nacalai Tesque, Inc.), and 1.0 g/L tylosin (Sigma-Aldrich) was produced with autoclaved tap water. The antibiotic solution was given, for 12 days, to mice to which the faecal contents derived from a donor C were orally administered. The antibiotic solution was changed once a week.

### (Separation and identification of colony-forming species from mouse faecal contents)

Mouse intestinal contents were mixed with PBS containing glycerol (20%), put in an anaerobic chamber, and stocked at -80°C. The stock was mixed with an equal volume of TS broth (BD) in the anaerobic chamber and plated on different agar plates described below. EG, ES, M10, NBGT, VS, and TS (BD), BL (Eiken Chemical Co., Ltd.), BBE (KYOKUTO PHARMACEUTICAL INDUSTRIAL CO., LTD), Oxoid CM0619 (Thermo Fisher Scientific K.K.), CM0619-supplemented SR0107 (Thermo Fisher Scientific K.K.), CM0619-supplemented SR0108 (Thermo Fisher Scientific K.K.), mGAM (Nissui Pharmaceutical Co., Ltd.), and Schaedler (BD). After culturing for 2 days, colonies having a different external appearance were transferred to a new EG plate. Thereafter, the colonies were cultured overnight in an EGEF liquid medium, mixed with glycerol (final concentration: 20% ((v/v)), and stocked at -80°C.

The formulation of an Eggerth Gagnon (EG) agar medium was as follows. Protease Peptone No. 3 (10.0 g), yeast extract (5.0 g), Na₂HPO₄ (4.0 g), glucose (1.5 g), soluble starch (0.5 g), L-cysteine HCl (0.5g), L-cysteine (0.2g), Tween 80 (0.5 g), agar (4.8 g), a horse meat extraction liquid (500 mL), and water up to 1000 mL + defibrinated horse blood (50 mL). In the EGEF medium, agar was removed, and defibrinated horse blood (50 mL) was replaced with a Fildes solution (40 mL).

Subsequently, the DNA genome of bacteria was extracted from the isolated strain by the same protocol as for DNA isolation from faeces. 16S rRNA was amplified by PCR using a KOD Plus Neo kit (TOYOBO CO., LTD.) according to the manufacturer's protocol. The Sanger sequence was outsourced to Eurofins Scientific. The base sequence was cross-checked against the NCBI database. The primer sequences for the Sanger sequence was as follows.
F27 primer: 5'-AGRGTTTGATYMTGGCTCAG-3' (SEQ ID NO: 39)
R1492 primer: 5'-TACGGYTACCTTGTTACGACTT-3' (SEQ ID NO: 40)

### (Sequence of 16S rRNA)

The frozen mouse faecal sample was thawed, and 100 µL of the suspension was mixed with 900 µL of a TE10 (10 mM Tris-HCl, 10 mM EDTA) buffer solution containing RNase A (final concentration: 100 µg/mL, Thermo Fisher Scientific K.K.) and lysozyme (final: 3.0 mg/mL, Sigma-Aldrich). The suspension was incubated at 37°C for 1 hour by gentle stirring. Purified Achromopeptidase (FUJIFILM Wako Pure Chemical Corporation) was added such that a final concentration was 2,000 units/mL, and incubation was further performed at 37°C for 30 minutes. Subsequently, sodium dodecyl sulfate (final: 1%) and Proteinase K (final: 1 mg/mL, Nacalai Tesque Inc.) were added to the suspension, and incubation was performed at 55°C for 1 hour.

Subsequently, high-molecular-weight DNA was extracted with phenol:chloroform: isoamyl alcohol (25:24:1), precipitated with isopropanol, washed thereafter with 70% ethanol, and resuspended in 100 µL of TE.

PCR was performed using Ex Taq (Takara Bio Inc.). The primer sequences for amplifying the V1-V2 region of the 16S rRNA gene were as follows.
27Fmod primer: 5'- 
AATGATACGGCGACCACCGAGATCTACACxxxxxxxxACACTCTTTCCCTACAC
GACGCTCTTCCGATCTAGRGTTTGATYMTGGCTCAG-3' (SEQ ID NO: 41,
 "xxxxxxxx" represents a Miseq (Illumina) index sequence)
)338R primer: 5'- 
CAAGCAGAAGACGGCATACGAGATxxxxxxxxGTGACTGGAGTTCAGACGTGTG
CTCTTCCGATCTTGCTGCCTCCCGTAGGAGT-3' (SEQ ID NO: 42, "xxxxxxxx"
 represents a Miseq (Illumina) index sequence)

PCR products were purified by Agencourt AMPure XP (Beckman Coulter, Inc.) according to the manufacturer's protocol. A 16S rRNA library was produced according to the manufacturer's protocol using a KAPA Library Quantification Kit (Kapa Biosystems, Inc.). The 16S rRNA was sequenced according to the standard protocol of MiSeq Reagent Kit ver3.

### (Gnotobiotic experiment)

The isolated strains except for *Phasolactobacterium faecium* (3G4) were cultured in an anaerobic chamber at a temperature of 37°C for 1 to 2 days. *Phasolactobacterium faecium* was cultured for 2 to 3 days in an Oxoid CM0619 agar plate to which 80 mM sodium succinate was added. The colonies were recovered and resuspended in EGEF. The bacterial density was adjusted based on an OD₆₀₀ value, and the mixture of the cultured strains was orally administered to GF mice (150 µL/mouse, total bacterial count: about 1 to 2 × 10⁸ CFU).

For quantitative determination of the DNA of *P. clara* in faeces, mouse faecal DNA was purified, and quantitative real-time PCR was performed to amplify sequences specific to the 16s rRNA gene of the *P. clara* strain with LightCycler 480 System (Roche) using THUNDERBIRD SYBR qPCR Mix (TOYOBO CO., LTD.). A standard curve was created from serial diluent solution of genomic DNA of the *P. clara* strain (JCM 14859). The base sequences of the primers used are as follows.
5'-CGTAGGAGTTTGGACCGTGT-3' (SEQ ID NO: 43)
5'-GCATGGGAGCGACAAATAAA-3' (SEQ ID NO: 44)

### (Citrobacter rodentium (C. rodentium) infection)

Any of 200 µL of 2-mix (*B. uniformis* and *P. merdae*) + *P. clara* (WT), 2-mix + *P. clara* (Δ00502), and 2-mix alone was orally inoculated to GF mice. After 14 days, the mice were infected with *C*. *rodentium* (150 µL/mouse) cultured overnight by oral administration, and euthanized on day 7.

For histological analysis, the cervical part of the mice was fixed with 4% paraformaldehyde and embedded in paraffin. The paraffin block was incised and stained with hematoxylin and eosin.

The degree of colitis was evaluated by a gastroenterology technician according to the following criteria: infiltration of inflammatory cells (score: 0 to 4), mucosal thickening (score: 0 to 4), depletion of goblet cells (score: 0 to 4), crypt abscess (score: 0 to 4), and disruption of tissue structure (score: 0 to 4). The final histological score was defined as the sum of the scores for these parameters.

For a colony-forming unit (CFU) assay, cecal patches or cecal luminal contents were collected, and after homogenization with PBS, serially diluted homogenates were plated on LB agar plates. CFU was counted after overnight culture at 37°C under aerobic conditions.

For the ex vivo evaluation of *C*. *rodentium*-specific IgA, the cecal contents were resuspended in a 5x (w/v) LB medium and centrifuged. The supernatant was filtered through a sterilization filter unit with a PVDF film having a pore size of 0.22 µm, and then mixed with an equal amount of a *C*. *rodentium* culture solution cultured overnight in vitro. This mixture was gently shaken at room temperature and incubated for 1 hour to examine the aggregation effect with a confocal microscope (Leica TCS SP8). Alternatively, after the incubation, the mixture was centrifuged and washed once with PBS. Thereafter, the bacterial cell pellet was dissolved in a 1 % SDS solution (diluted with a 50 mM Tris-HCl buffer solution to which 5 mM EDTA was added). The lysate was stained by western blot with a goat anti-mouse IgA α chain antibody (HRP) (ab97235) to evaluate the relative amount of *C*. *rodentium*-binding (*C*. *rodentium-*specific) IgA in the faecal contents.

For a vaccine administration experiment, 200 µL of 2-mix (*B. uniformis* and *P*. *merdae*) + *P. clara* (WT), or 2-mix + *P. clara* (Δ00502) was preliminarily administered to GF mice. After 4 days, *C*. *rodentium* (10¹⁰ cells/mouse) inactivated with peracetic acid was orally administered to the mice once a week for 3 weeks. After 3 weeks of immunization, the mice were infected with *C*. *rodentium* (150 µL/mouse) cultured overnight by oral administration, and euthanized on day 14.

The *C*. *rodentium* inactivated by peracetic acid was produced as follows. *C*. *rodentium* cultured overnight was recovered by centrifugation (16,000 × g, 10 minutes) and resuspended in sterilized PBS at a density of 10¹⁰ cells per 1 mL. Peracetic acid (240990, Sigma-Aldrich) was added such that a final concentration was 0.4%, and incubation was performed at room temperature for 1 hour. After washing 3 times with sterilized PBS, the final pellet was resuspended in PBS at a final concentration of 10¹¹ particles/mL and preserved at 4°C. Before using a vaccine, 200 mL of an LB medium was inoculated with 100 µL of an inactivated vaccine and cultured at 37°C overnight to confirm complete inactivation.

### (Mouse hepatitis virus (MHV) infection)

MHV-2 was provided by Makoto UJIKE (Nippon Veterinary and Life Science University). Five-week-old GF C57BL/6N male mice were obtained from CLEA Japan, Inc. or Sankyo Labo Service Corporation, and were separately bred in stainless steel isolators. 200 µL of 2-mix (*B. uniformis* and *P. merdae*) + *P. clara* (WT) or 2-mix *+ P. clara* (Δ00502) was orally administered to the GF mice. Two weeks after inoculation, the mice were orally infected with 4.5 × 10⁶ PFU of MHV-2, and the survival rate was observed every day for 10 days.

The liver and brain were collected on 4 or 5 days after infection to measure virus titers, and homogenization was performed with DNA/RNA Shield (Zymo Research Corporation). Viral RNA was extracted using a Quick-RNA Viral Kit (Zymo Research Corporation) according to the manufacturer's instructions, and cDNA was synthesized using ReverTra Ace (TOYOBO CO., LTD.) and a random primer (TOYOBO CO., LTD.).

Quantitative real-time PCR was performed with LightCycler 480 System (Roche) using THUNDERBIRD SYBR qPCR Mix (TOYOBO CO., LTD.) to amplify an orf1a gene. The relative amount of MHV was obtained from a standard curve created from a serial diluent solution of MHV genomic cDNA. The base sequences of the primers used are as follows.
5'-AAGAGTGATTGGCGTCCGTAC-3' (SEQ ID NO: 45)
5'-ATGGACACGTCACTGGCAGAG-3' (SEQ ID NO: 46)

### (Trypsin decomposition in vitro)

The bacteria cultured overnight were incubated with recombinant mouse trypsin (final concentration: 1 µg/mL) for 1 hour or with human trypsin (final concentration: 20 µg/mL) for 4 hours. Recombinant trypsin isoforms used were as follows: recombinant mouse PRSS2 (50383-M08H, Sino Biological, Inc.), recombinant human PRSS1 (LS-G135640), recombinant human PRSS2 (LS-G20167), and recombinant human PRSS3 (His-tag) (NBP2-52220).

In some experiments, recombinant mouse PRSS2 was first treated with one of the following trypsin inhibitors for 30 minutes before incubation with a *P. clara* culture solution. AEBSF (Sigma-Aldrich, final concentration: 2 mM), Leupeptin (Sigma-Aldrich, final concentration: 100 µM), and TLCK (Abeam plc., final concentration: 100 µM).

In some experiments, *P. clara* was cultured overnight in the presence of Tunicamycin (Sigma-Aldrich, final concentration: 10 µg/mL), 2-Fluoro-L-fucose (Cayman Chemical Company, final concentration: 250 µM), or the corresponding DMSO control, before incubation with recombinant mouse PRSS2.

In the experiment of evaluating the effect of Ca²⁺, *P. clara* was cultured in a low Ca²⁺ mGAM medium supplemented with or without 1 mM of Ca²⁺, before incubation with recombinant mouse PRSS2. In the experiment in which the supernatant of *P. clara* was used, *P. clara* cultured overnight was filtered through a sterilization filter unit formed using a PVDF film having a pore size of 0.22 µm.

### (Confocal microscope)

Recombinant mouse PRSS2 was labeled with Alexa Fluor^{™} 488 using an Alexa Fluor^{™} 488 Antibody Labeling Kit (A20181, Thermo Fisher Scientific K.K.), and was pretreated with an AEBSF inhibitor (150 µg/mL rmPRSS2 with 20 mM AEBSF).

Mouse PRSS2 labeled with Alexa Fluor^{™} 488 was incubated in an anaerobic chamber for 20 minutes with bacteria cultured at a final concentration of 5 µg/mL. The mixture was centrifuged, washed once with PBS, and then resuspended in PBS. A Leica TCS SP8 confocal microscope was used for capturing a confocal image.

### (Disuccinimidyl sulfoxide (DSSO) crosslinking)

DSSO (A33545) was purchased from Thermo Fisher Scientific K.K. A *P. clara* (1C4) strain cultured overnight was incubated with AEBSF-treated mouse recombinant PRSS2 for 20 minutes, washed once with PBS, and then resuspended in 10 mM of DSSO. After incubation at room temperature for 10 minutes, a tris-hydrochloride buffer solution (final concentration: 20 mM) was added to stop the reaction.

After washing with PBS, the pellet was dissolved in a 1% SDS solution (diluted with a 50 mM Tris-HCl buffer solution to which 5 mM EDTA was added). Only the *P. clara* (1C4) strain (without incubation with PRSS2) was treated in the same manner to be served as a negative control. The supernatant was stained with a rabbit anti-6-His antibody (A190-214A, Bethyl Laboratories, Inc.) and an anti-rabbit IgG antibody (HRP) (#7074, Cell Signaling Technology, Inc.), and was analyzed by western blot.

### (Whole-cell lysate, supernatant, protein staining of sugar chain-containing protein)

The *P. clara* (1C4) strain was cultured overnight in the presence of Tunicamycin (Sigma-Aldrich, final: 10 µg/mL), 2-Fluro-L-fucose (Cayman Chemical Company, final: 250 µM), or the corresponding DMSO control. Thereafter, the cultured bacteria were pelletized, washed once with PBS, and dissolved in a 1% SDS solution (obtained by dilution with a 50 mM Tris-HCl buffer solution to which 5 mM EDTA was added). SDS-PAGE was performed using Novex (registered trademark) NuPAGE (registered trademark) SDS-PAGE Gel system (Thermo Fisher Scientific K.K.). Proteins including the sugar chain were stained using a Pro-Q^{™} Emerald 300 Glycoprotein Gel and Blot Stain Kit (Thermo Fisher Scientific K.K.) according to the manufacturer's protocol. The proteins of a whole-cell lysate were stained with a Colloidal Blue Staining Kit (Thermo Fisher Scientific K.K.). The protein in the supernatant was concentrated with an Amicon Ultra Centrifugal Filter (10kD NMWL) and then stained with a Colloidal Blue Staining Kit (Thermo Fisher Scientific K.K.).

### (Production of mutant)

Deletion mutants (Δ03049 to 03053, Δ000502, and Δ000509) of the *P. clara* (JCM 14859) strain were produced as follows. First, a sequence of about 1 kb including a coding region was amplified by PCR and incorporated into a suicide vector (pLGB30) using a HiFi DNA Assembly (NEB) according to the manufacturer's protocol. Subsequently, 1 µL of each reaction liquid was transformed into electrocompetent *Escherichia coli* S17-1 λpir.

Subsequently, the transformant was bound to the *P. clara* (JCM 14859) strain as follows. A donor strain was cultured in an LB medium and a recipient strain was cultured in an EGEF medium such that an OD₆₀₀ was 0.5, and mixing was performed at a ratio of 1:1. This mixture was added dropwise to an EGEF agar plate and was aerobically cultured at 37°C for 16 hours. Subsequently, the exconjugants were selected on the EGEF agar medium plate containing tetracycline (10 µg/mL). The exconjugants were partially sensitive to the expression of ss-bfel toxin induced by rhamnose, and the growth was inhibited in the presence of 10 mM of rhamnose (overnight OD₆₀₀ of up to 0.3). Thereafter, to select the disappearance from the plasmid genome by second crossover, the exconjugants were cultured in EGEF broth, to which 10 mM of rhamnose was added, for at least three generations until the exconjugants lost to the restored cells (OD₆₀₀ reached up to 1.0 overnight). Thereafter, bacterial culture was performed, and a single colony was collected to confirm normal deletion by PCR.

A homologous sequence of about 0.5 to 1 kb of the coding region was incorporated into the suicide vector (pLGB30) to be transformed into an electrocompetent *Escherichia coli* S17-1 strain, and an insertion mutant was produced by the same procedure.

The transformant was conjugated with the *P. clara* (JCM 14859) strain by the same protocol. Exconjugants were selected on an EGEF agar plate containing tetracycline (10 µg/mL), confirmed by PCR, and then maintained with EGEF broth to which tetracycline (10 µg/mL) was added. The base sequences of all of the primers used to create the mutants are shown in Tables 1 to 5 below.

**[Table 1]**

| Primer for insertion mutant Primer name | Base sequence (5'-3') | SEQ ID NO. |
|---|---|---|
| PorU-cloning-Fw | GTGGATCCCCCGGGCTGCAGCATCCCTCCTCCATCTGT | 47 |
| PorU-cloning-Rv | CTGGAAGATAGCCAATTAGTTTGGGACGTAGGGGCAATC | 48 |
| PorU-mutant validation-Fw | TCCCGACCGACTGATCCTTTTTAACCC | 49 |
| PorU-mutant validation-Rv | CTGTGCCCAGTAATTTTGTG | 50 |
| 00029-cloning-Fw | GTGGATCCCCCGGGCTGCACCCCTTGTCCATGTTAGACG | 51 |
| 00029-cloning-Rv | CTGGAAGATAGGCAATTAGTGAAGTGACAGGGGATGCTA | 52 |
| 00029-mutant validation-Fw | AAACAAATAAATGAAGAAAATTTTATCTTTGCT | 53 |
| 00029-mutant validation-Rv | CTGGAAGATAGGCAATTAGTGAAGTGACAGGGGATGCTA | 54 |
| 00890-cloning-Fw | GTGGATCCCCCGGGCTGCAACACTGATGTCGTTGCCTTT | 55 |
| 00890-cloning-Rv | CTGGAAGATAGGCAATTAGGCAACCTTACCACAGGGAAA | 56 |
| 00890-mutant validation-Fw | AAACAAATAAATGAAGAAAATTTTATCTTTGCT | 57 |
| 00890-mutant validation-Rv | CTGGAAGATAGGCAATTAGGCAACCTTACCACAGGGAAA | 58 |
| 00822-cloning-Fw | GTGGATCCCCCGGGCTGCACAAGTAGAGTTCCGGTTCGG | 59 |
| 00822-cloning-Rv | CTGGAAGATAGGCAATTAGAAATCACGACCCTCGATGTG | 60 |
| 00822-mutant validation-Fw | AAACAAATAAATGAAGAAAATTTTATCTTTGCT | 61 |
| 00822-mutant validation-Rv | CTGGAAGATAGGCAATTAGAAATCACGACCCTCGATGTG | 62 |
| 00342-cloning-Fw | GTGGATCCCCCGGGCTGCAAGTTCGTTCTTCTCGATGGC | 63 |
| 00342-cloning-Rv | CTGGAAGATAGGCAATTAGCAACCGCGGGTATAACAAGA | 64 |
| 00342-mutant validation-Fw | AAACAAATAAATGAAGAAAATTTTATCTTTGCT | 65 |
| 00342-mutant validation-Rv | CTGGAAGATAGGCAATTAGCAACCGCGGGTATAACAAGA | 66 |
| 03191-cloning-Fw | GTGGATCCCCCGGGCTGCATGGGGCGTTTGTAAAAGTCA | 67 |
| 03191-cloning-Rv | CTGGAAGATAGGCAATTAGAAACCGCTGACGGTCTATTC | 68 |
| 03191-mutant validation-Fw | AAACAAATAAATGAAGAAAATTTTATCTTTGCT | 69 |
| 03191-mutant validation-Rv | CTGGAAGATAGGCAATTAGAAACCGCTGACGGTCTATTC | 70 |
| 00502-cloning-Fw | GTGGATCCCCCGGGCTGCATCCAGTCGTCAGACTTTCCT | 71 |
| 00502-cloning-Rv | CTGGAAGATAGGCAATTAGGAAGGGACTAAAATCGCCGT | 72 |
| 00502-mutant validation-Fw | AAACAAATAAATGAAGAAAATTTTATCTTTGCT | 73 |
| 00502-mutant validation-Rv | CTGGAAGATAGGCAATTAGGAAGGGACTAAAATCGCCGT | 74 |
| 02199-cloning-Fw | GTGGATCCCCCGGGCTGCAGGGTCGCTTACATTTTGCAC | 75 |
| 02199-cloning-Rv | CTGGAAGATAGGCAATTAGTCGGTGATTATGCTTTCGCA | 76 |
| 02199-mutant validation-Fw | AAACAAATAAATGAAGAAAATTTTATCTTTGCT | 77 |
| 02199-mutant validation-Rv | CTGGAAGATAGGCAATTAGTCGGTGATTATGCTTTCGCA | 78 |
| 00472-cloning-Fw | GTGGATCCCCCGGGCTGCACTTGCTTCAAATCACGCTGG | 79 |
| 00472-cloning-Rv | CTGGAAGATAGGCAATTAGGTTGCGTAATGTGAACGGTG | 80 |
| 00472-mutant validation-Fw | AAACAAATAAATGAAGAAAATTTTATCTTTGCT | 81 |
| 00472-mutant validation-Rv | CTGGAAGATAGGCAATTAGGTTGCGTAATGTGAACGGTG | 82 |
| 01041-cloning-Fw | GTGGATCCCCCGGGCTGCATGTCGAAAGGCATCATCTGG | 83 |
| 01041-cloning-Rv | CTGGAAGATAGGCAATTAGACCATTACGGCCATTACCAC | 84 |
| 01041-mutant validation-Fw | AAACAAATAAATGAAGAAAATTTTATCTTTGCT | 85 |
| 01041-mutant validation-Rv | CTGGAAGATAGGCAATTAGACCATTACGGCCATTACCAC | 86 |

**[Table 2]**

| Primer for insertion mutant Primer name | Base sequence (5'-3') | SEQ ID NO. |
|---|---|---|
| 00823-cloning-Fw | GTGGATCCCCCGGGCTGCAGTGCCAGTTTCAACCAATCG | 87 |
| 00823-cloning-Rv | CTGGAAGATAGGCAATTAGCTTGTACCCCAGCAGATGTC | 88 |
| 00823-mutant validation-Fw | AAACAAATAAATGAAGAAAATTTTATCTTTGCT | 89 |
| 00823-mutant validation-Rv | CTGGAAGATAGGCAATTAGGTAAGCTGGCGCTTGAAATC | 90 |
| 00729-cloning-Fw | GTGGATCCCCCGGGCTGCATTGTTTTGGTTGTAACTGCC | 91 |
| 00729-cloning-Rv | CTGGAAGATAGGCAATTAGGTCAATACGAACTGCAACTG | 92 |
| 00729-mutant validation-Fw | AAACAAATAAATGAAGAAAATTTTATCTTTGCT | 93 |
| 00729-mutant validation-Rv | CTGGAAGATAGGCAATTAGGTCAATACGAACTGCAACTG | 94 |
| 00935-cloning-Fw | GTGGATCCCCCGGGCTGCAGACCAATTAAGCACGTCAAG | 95 |
| 00935-cloning-Rv | CTGGAAGATAGGCAATTAGTGGTACAATTCGCTCAAAGA | 96 |
| 00935-mutant validation-Fw | AAACAAATAAATGAAGAAAATTTTATCTTTGCT | 97 |
| 00935-mutant validation-Rv | CTGGAAGATAGGCAATTAGTGGTACAATTCGCTCAAAGA | 98 |
| 01686-cloning-Fw | GTGGATCCCCCGGGCTGCATACTCCCGTGTTTTTAGTGG | 99 |
| 01686-cloning-Rv | CTGGAAGATAGGCAATTAGGGATGCCAGTTACCTGATAG | 100 |
| 01686-mutant validation-Fw | AAACAAATAAATGAAGAAAATTTTATCTTTGCT | 101 |
| 01686-mutant validation-Rv | CTGGAAGATAGGCAATTAGGGATGCCAGTTACCTGATAG | 102 |
| 03166-cloning-Fw | GTGGATCCCCCGGGCTGCATCAAACCACACACAGAGAAA | 103 |
| 03166-cloning-Rv | CTGGAAGATAGGCAATTAGGAATTCGGCCAAACCTTATG | 104 |
| 03166-mutant validation-Fw | AAACAAATAAATGAAGAAAATTTTATCTTTGCT | 105 |
| 03166-mutant validation-Rv | CTGGAAGATAGGCAATTAGGAATTCGGCCAAACCTTATG | 106 |
| 00509-cloning-Fw | GTGGATCCCCCGGGCTGCAAGACAATCGAGGCTATCATAC | 107 |
| 00509-cloning-Rv | CTGGAAGATAGGCAATTAGATAGCGTGAATGGAAAACCT | 108 |
| 00509-mutant validation-Fw | AAACAAATAAATGAAGAAAATTTTATCTTTGCT | 109 |
| 00509-mutant validation-Rv | GTGGAAGATAGGGAATTAGATAGCGTGAATGGAAAACCT | 110 |
| 00002-cloning-Fw | GTGGATCCCCCGGGCTGCAATCTATCTCAACCTCCTGCT | 111 |
| 00002-cloning-Rv | CTGGAAGATAGGCAATTAGGAAGGAATCAGCGTAGATGT | 112 |
| 00002-mutant validation-Fw | AAACAAATAAATGAAGAAAATTTTATCTTTGCT | 113 |
| 00002-mutant validation-Rv | CTGGAAGATAGGCAATTAGGAAGGAATCAGCGTAGATGT | 114 |
| 00104-cloning-Fw | GTGGATCCCCCGGGCTGCATGTGCCGATAACCTGAATGG | 115 |
| 00104-cloning-Rv | CTGGAAGATAGGCAATTAGCAGAGAAAAGGGCTGGTGTT | 116 |
| 00104-mutant validation-Fw | AAACAAATAAATGAAGAAAATTTTATCTTTGCT | 117 |
| 00104-mutant validation-Rv | CTGGAAGATAGGCAATTAGCAGAGAAAAGGGCTGGTGTT | 118 |
| WecA-cloning-Fw | TCCCGACCGAGGTCTTTGTCCCCTTCTCCG | 119 |
| WecA-cloning-Rv | TCTTACACCGGCGGGTTGCTTTTTATGCCT | 120 |
| WecA-mutant validalion-Pw | GCCTGATGCATGGAAAAACC | 121 |
| WccA-mutaut validation-Rv | CTGGAAGATAGGCAATTAG | 122 |
| 00503-cloning-Fw | GTGGATCCCCCGGGCTGCACGGATAACGGAATTACCCAT | 123 |
| 00503-cloning-Rv | CTGGAAGATAGGCAATTAGTCTGACAAAGGTTGGTTGAA | 124 |
| 00503-mutant validation-Fw | AAACAAATAAATGAAGAAAATTTTATCTTTGCT | 125 |
| 00503-mutant validation-Rv | CTGGAAGATAGGCAATAGTCTGACAAAGGTTGGTTGAA | 126 |

**[Table 3]**

| Primer for insertion mutant Primer name | Base sequence (5'-3') | SEQ ID NO. |
|---|---|---|
| 00504-cloning-Fw | CTCCATCCCCCCGGCTCCATCTATAACCCCCAGAAGGAA | 127 |
| 00504-cloning-Rv | CTGGAAGATAGGCATTAGAACAATACCGTCAACCTCTC | 128 |
| 00504-mutant validation-Fw | AAACAAATAAATGAAGAAAATTTTATCTTTGCT | 129 |
| 00504-mutant validation-Rv | CTGGAAGATAGGCAATTAGAACAATACCGTCAACCTCTC | 130 |
| 00505-cloning-Fw | CTCCATCCCCCCGGCTCCATTTATTACCCCTCCTATCCC | 131 |
| 00505-cloning-Rv | CTGGAAGATAGGCAATTAGACTCCAACAGCATCTTCAAT | 132 |
| 00505-mutant validation-Fw | AAACAAATAAATGAAGAAAATTTTATCTTTGCT | 133 |
| 00505-mutant validation-Rv | CTGGAAGATAGGCAATTAGACTCCAACAGCATCTTCAAT | 134 |
| 00506-cloning-Fw | CTCCATCCCCCCGGCTCCAGTAGTCTATCCCTTTCTCCT | 135 |
| 00506-cloning-Rv | CTGGAAGATAGGCAATTAGATTCATTACTGGTCGGAACC | 136 |
| 00506-mutant validation-Fw | AAACAAATAAATGAAGAAAATTTTATCTTTGCT | 137 |
| 00506-mutant validation-Rv | CTGGAAGATAGGCAATTAGATTCATTACTGGTCGGAACC | 138 |
| 00507-cloning-Fw | GTGGATCCCCCGGGCTGCAGGGATACTGAATGTCTGTCC | 139 |
| 00507-cloning-Rv | CTGGAAGATAGGCAATTAGACAGTGTAATCATCAACGCT | 140 |
| 00507-mutant validation-Fw | AAACAAATAAATGAAGAAAATTTTATCTTTGCT | 141 |
| 00507-mutant validation-Rv | CTGGAAGATAGGCAATTAGACAGTGTAATCATCAACGCT | 142 |
| 00508-cloning-Fw | GTGGATCCCCCGGGCTGCAATTTTCAATCCAGTCCCTCC | 143 |
| 00508-cloning-Rv | CTGGAAGATACCCAATTACTACAGTTTCCCCATACCAAA | 144 |
| 00508-mutant validation-Fw | AAACAAATAAATGAAGAAAATTTTATCTTTGCT | 145 |
| 00508-mutant validation-Rv | CTGGAAGATACCCAATTACTACAGTTTCCCCATACCAAA | 146 |

**[Table 4]**

| Primer for knockout Primer name | Base sequence (5'-3') | SEQ ID NO. |
|---|---|---|
| 03048-03053 KO-upstream cloning-Fw | GTGGATCCCCCGGGCTGCATATGAACTACAAAGTCATCG | 147 |
| 03048-03053 KO-upstream cloning-Rv | TTCAAGAAGCACCACCGTTATGATATA | 148 |
| 03048-03053 KO-downstream cloning-Fw | TAACGGTGGTGCTTCTTGAAAATTAAG | 149 |
| 03048-03053 KO-downstream cloning-Rv | CTGGAAGATAGGCAATTAGGACGATTTCGTGGACA | 150 |
| 03048-03053 KO-validation-Fw1 | CTGGATCCCCCGGGCTGCATATGAACTACAAACTCATCG | 151 |
| 03048-03053 KO-validation-Rv2 | CTGGAAGATAGGCAATTAGGACGATTTCGTGGACA | 152 |
| 03048-03053 KO-validation-Fw3 | TCAACGCACCATTACTTTC | 153 |
| 03048-03053 KO-validation-Rv4 | ATGAAAAACAGTTTACTTGC | 154 |

**[Table 5]**

| Primer for knockout Primer name | Base sequence (5'-3') | SEQ ID NO. |
|---|---|---|
| 00502 KO-upstream cloning-Fw | GTGGATCCCCCGGGCTGCATACAATCCGTTGGAACTGTC | 155 |
| 00502 KO-upstream cloning-Rv | ATTAGTATTTCAGCATTCATTTGCTGGTGA | 156 |
| 00502 KO-downstream cloning-Fw | ATGAATGCTGAAATACTAATTTTAGATAGACTATAATTTG | 157 |
| 00502 KO-downstream cloning-Rv | CTGGAAGATAGGCAATTAGGAACTGGTCGTAAAAATCAC | 158 |
| 00502 KO-validation-Fwl | GTGGATCCCCCGGGCTGCAIACAATCCGTTGGAACTGTC | 159 |
| 00502 KO-validation-Rv2 | CTGGAAGATAGGCAATTAGGAACTGGTCGTAAAAATCAC | 160 |
| 00502 KO-validation-Fw3 | GTGGATCCCCCGGGCTGCATCCAGTCGTCAGACTTTCCT | 161 |
| 00502 KO-validation-Rv4 | CTGGAAGATAGGCAATTAGGAAGGGACTAAAATCGCCGT | 162 |
| 00509 KO-upstream cloning-Fw | GTGGATCCCCCGGGCTGCAATCCTTCCACTTTTCCTCTC | 163 |
| 00509 KO-upstream cloning-Rv | AATTGATCGTTCTAAAGCCGACGATTTAAG | 164 |
| 00509 KO-downstream cloning-Fw | CGGCTTTAGAACGATCAATTTTAAAATGAATATAT | 165 |
| 00509 KO-downstream cloning-Rv | CTGGAAGATAGGCAATTAGTTCAACGAAAACAGAACATC | 166 |
| 00509 KO-validation-Fw1 | GTGGATCCCCCGGGCTGCAATCCTTCCACTTTTCCTCTC | 167 |
| 00509 KO-validation-Rv2 | CTGGAAGTAGGCAATTAGTTCAACGAAAACAGAACATC | 168 |
| 00509 KO-validation-Fw3 | GTGGATCCCCCGGGCTGCAAGACAATCGAGGCATCATAC | 169 |
| 00509 KO-validation-Rv4 | CTGGAAGATAGGCAATTAGATAGCGTGAATGGAAAACCT | 170 |

### (Transmission electron microscope (TEM))

A wild-type (WT) strain or a Δ00502 *P. clara* (JCM 14589) strain was incubated for 20 minutes with mouse recombinant PRSS2 (50383-M08H, Sino Biological, Inc., final concentration: 5 µg/mL), washed with PBS, and then fixed at room temperature for 2 hours using a 4% paraformaldehyde-1% glutaraldehyde solution. After washing with 0.05 M PBS, the pellets were gradually dehydrated with ethanol (50%, 70%, 80%, 90%, 95%, 100%). The dehydrated pellets were infiltrated with an LRW resin (with 100% ethanol and LRW at 1:1 for 1 hour, thereafter with 100% ethanol and LRW at 1:2 overnight, and further with 100% LRW for 5 hours). After the infiltration, the sample was cured with a gelatin capsule (53°C, 24 hours). The polymerized LFW block was incised with a Leica Ultracut UCT to obtain an 80 nm section.

For immunogold staining, the section was first blocked with 0.05 M of PBS, to which 1% BSA was added, and then stained with a rabbit anti-6-His antibody (A190-214A, Bethyl Laboratories, Inc.) for 60 minutes. After washing with 0.05 M of PBS, the section was stained with a 12 nm colloidal gold-labeled goat anti-rabbit IgG antibody for 60 minutes. After washing again with 0.05 M of PBS, the section was fixed with 1% glutaraldehyde dissolved in 0.05 M of PBS, washed with water, and then stained with uranyl acetate for 5 minutes. All images were captured with a JEOL JEM-1400 transmission electron microscope.

### (Expression of recombinant protein and binding to magnetic microbeads)

In order to produce recombinant 00502 protein and recombinant 00509 protein, the coding regions (excluding the N-terminal sequence encoding a signal peptide) of the genes of both proteins were cloned into an expression vector pET-28b (+) (#69865, Novagen), and a C-terminal His-tag was introduced according to the supplier's protocol.

Subsequently, the expression vector was transformed into Rosetta-gami B(DE3) Competent Cells (#71136, Novagen). Subsequently, the transformant was grown exponentially, and 0.4 mM IPTG (16758, Sigma-Aldrich) was added to induce the expression of the protein. After culturing overnight at 25°C, the cells were lysed using B-PER^{™} Bacterial Protein Extraction Reagent (#78243, Thermo Fisher Scientific K.K.), and the recombinant 00502 protein and the recombinant 00509 protein were prepared using Pierce^{™} Ni-NTA Magnetic Agarose Beads (#78605, Thermo Fisher Scientific K.K.), and Pierce^{™} Polyacrylamide Spin Desalting Columns (#89849, Thermo Fisher Scientific K.K.).

Subsequently, the purified recombinant 00502 protein, the purified recombinant 00509 protein, or bovine serum albumin (#23209, Thermo Fisher Scientific K.K.) were bound to micromagnetic beads (Dynabeads^{™}) using Dynabeads^{™} Antibody Coupling kit (14311D, Thermo Fisher Scientific K.K.) according to the manufacturer's protocol. 15 µg of the protein was added per 1 mg of the beads.

Subsequently, 1 mg of the protein-bound Dynabeads^{™} was resuspended in 200 µL of an EGEF medium and mixed with recombinant mouse PRSS2 (final concentration: 3 µg/mL), Alexa Fluor^{™} 488-labeled mouse PRSS2 (final concentration: 5 µg/mL) pretreated with AEBSF, or 50 µL of GF cecal contents (50-fold diluted with PBS). The base sequences of all of the primers used to produce the recombinants are shown in Table 6 below.

**[Table 6]**

| Primer for recombinant Primer name | Base sequence (5'-3') | SEQ ID NO. |
|---|---|---|
| pET-28b-backbone (for r00502)-Fw | ATTCGTAAAACACCACCACCACCACCACTG | 171 |
| pET-28b-backbone (for r00502)-Rv | TCACGCCGTCCATGGTATATCTCCTTCTTA | 172 |
| r00502-cloning-Fw | ATATACCATGGACGGCGTGAGCCAACCTAC | 173 |
| r00502-cloning-Rv | GGTGGTGGTGTTTTACGAATACTTTCTTCA | 174 |
| pET-28b-backbone (for r00509)-Fw | GATGGTAAAACACCACCACCACCACCACTG | 175 |
| pET-28b-backbone (for r00509)-Rv | CTTCACCAACCATGGTATATCTCCTTCTTA | 176 |
| r00509-cloning-Fw | ATATACCATGGTTGGTGAAGATTTACATTT | 177 |
| r00509-cloning-Rv | GGTGGTGGTGTTTTACCATCATCTTCTTAC | 178 |

### (Protease activity assay)

The protease activity of the *P. clara* culture solution, the *P. clara* culture solution supernatant, the recombinant 00502 protein, and the recombinant 005009 protein were measured using Pierce^{™} Fluorescent Protease Assay Kit (#23266, Thermo Fisher Scientific K.K.) according to the manufacturer's protocol.

Using PerkinElmer 2030 Multilabel Reader equipped with an excitation filter and a fluorescence filter of fluorescein (485/538 nm), an increase in total fluorescence due to digestion of the FITC-casein substrate to smaller fluorescein-labeled fragments was detected. The protease activity was measured as a change in relative fluorescence units (RFUs).

### (Metagenomics analysis of published human faecal samples from cohorts)

The metagenomes obtained from human faecal samples of each cohort of PRISM, HMP2, FHS, 500FG, CVON, and Jie were assembled into a non-redundant gene catalog de novo and aggregated into metagenomic species using MSPminer to quantitatively determine relative abundances.

USEARCH ublast (at protein level) was adopted to search the gene catalog for the homologues of the genes of the *P. clara* strain and the genes of a *P. xylanphila* strain at a trypsin-associated locus including the gene encoding the 00502 protein and the gene encoding the 00509 protein, and for other six nearby genes. Hits with a minimum e-value of 0.1 were maintained. As a result, it was confirmed in the gene catalog that all eight genes of each type were present.

In order to identify additional putative homologues and species encoding this locus, first, the similarity between the homologues corresponding to the *P. clara* strain and the *P. xylanphila* strain was evaluated, and the minimum identity (Id) and the coverage (Cov) threshold value of the ublast hits corresponding to each gene at the locus were set as follows. 00502: Id = 25%, Cov = 90% 00503: Id = 70%, Cov = 90% 00504: Id = 60%, Cov = 90% 00505: Id = 60%, Cov = 90% 00506: Id = 50%, Cov = 90% 00507: Id = 25%, Cov = 90% 00508: Id = 45%, Cov = 80% 00509: Id = 20%, Cov = 30%

Subsequently, whether or not other metagenome species (MPS) encode the homologues of the 00502 to 00509 genes of the *P. clara* strain and the *P. xylanphila* strain was evaluated, and MSP 0355 having a homologue of 8 and MSP 0305 having a homologue and 7 were identified. MSP 0355 and MSP 0305 had previously been annotated only to *Bacteroidetes,* but at this time, these proteomes were compared to the Unified Human Gastrointestinal Genome (UHGG) collection using ublast. As a result, MSP 0355 was annotated as GUT_GENOME 140082 and MSP 0305 was annotated as GUT_GENOME 016875, and the majority of genes (> 90%) mapped to a single species representative of the UHGG with high reliability (median value of amino acid identity > 99% and e value < 1 × e⁻¹⁸⁴). In the UHGG, both were phylogenetically classified as a Paraprevotella species.

### (Statistics)

All statistical analyzes were performed using GraphPad Prism software (GraphPad Software, Inc.). One-way ANOVA which was used in combination with Tukey's test was used for the multiple comparison. For comparison between the two groups, a Mann-Whitney test (non-parametric) or a paired t test (parametric) with Welch's correction was used. In order to examine the correlation between two variables, Spearman's rank correlation was used. A Log-rank (Mantel-Cox) test was used for the survival analysis.

### [Experimental Example 1]

### (Regulation of trypsin in large intestine by microbiota)

In order to examine the effect of the intestinal microbiota on the protein distribution in the large intestine, the cecal contents of germ-free (GF) mice and specific-pathogen-free (SPF) mice were collected to perform proteomics analysis mass spectrometry. It was found that 324 types out of 713 types of host-derived proteins detected were larger in the SPF mice than in the GF mice (> 2-fold, p < 0.05). They also contained immune-related molecules such as α-defensin 21 (Defa21) and peptidoglycan recognition protein 1 (Pglyrpl). On the other hand, in the GF mice, 45 types of proteins were abundantly present (> 2-fold, p < 0.05) than in the SPF mice.

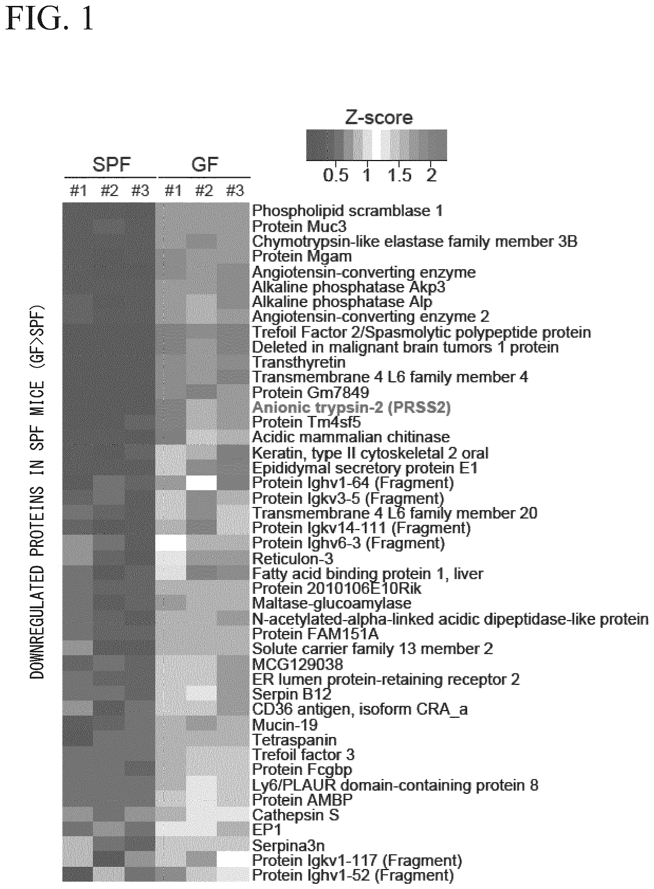

FIG. 1 is a diagram showing the results of proteomics analysis. FIG. 1 shows proteins of which the expression level was increased in the cecum of the GF mice than in the SPF mice. Among them, an anionic trypsin protease (encoded by the Prss2 gene) was focused on. The level of the anionic trypsin protease (PRSS2) was significantly increased in the cecal contents of the GF mice.

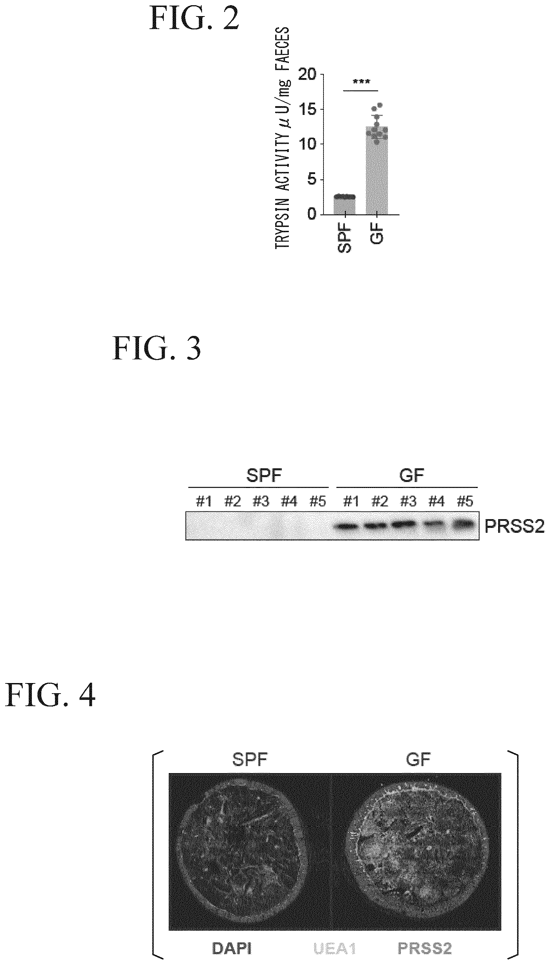

FIG. 2 is a graph showing the results of a trypsin activity test on the faeces of the SPF mice and the GF mice. FIG. 3 is a photograph showing the results of detecting the anionic trypsin protease (PRSS2) by western blot analysis of the faeces of the SPF mice and the GF mice. FIG. 4 is a fluorescence micrograph showing the results of detecting mucus (UEA1) and the anionic trypsin protease (PRSS2) by immunostaining of large intestine sections of the SPF mice and the GF mice. In addition, cell nuclei were stained with DAPI.

As a result, it was found that the results of the trypsin activity test on the faeces of the SPF mice and the GF mice, the western blot analysis of the faeces of the SPF mice and the GF mice, and the immunostaining of the large intestine sections were also the same as the results of the proteomics analysis, and found that, in the GF mice, the anionic trypsin protease was abundantly present in the cecal contents and the faeces as compared to the SPF mice. Hereinafter, the anionic trypsin protease may be simply referred to as trypsin.

Trypsin is produced in the pancreas as an inactive precursor (trypsinogen), and then secreted into the duodenum to be activated by enteropeptidase. Therefore, the expression of trypsinogen in the pancreas of the GF mice and the SPF mice was examined. As a result, it was found that GF and SPF had equivalent levels of proteins and mRNA of PRSS2 in the pancreas. From this result, a possibility that the difference in trypsin levels observed in the large intestine was due to the difference in production amounts in the pancreas was ruled out.

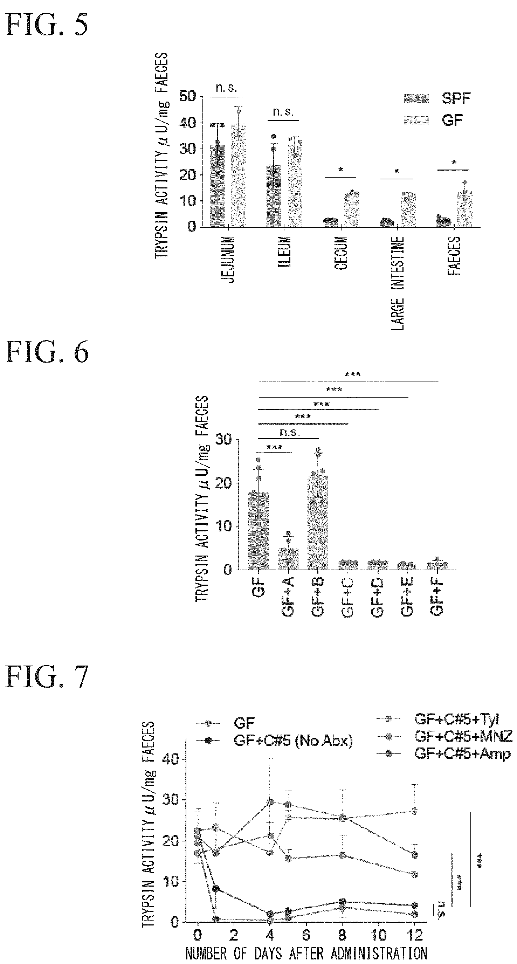

Subsequently, intraluminal trypsin activity was examined at different sites in the small intestine and the large intestine of the GF mice and the SPF mice. FIG. 5 is a graph showing the results of measuring the trypsin activity in the jejunum, the ileum, the cecum, the large intestine, and faeces. Furthermore, in FIG. 5, "n.s." indicates that there is no significant difference, and "*" indicates that there is a significant difference at p < 0.05.

As a result, it was found that the amounts of trypsin of the SPF mice and the GF mice were similar up to the distal tip of the small intestine. On the other hand, in the large intestine, the trypsin activity of the SPF mice was significantly decreased as compared to that of the GF mice, clarifying that microbiota play an important role in the regulation of trypsin in the large intestine.

### [Experimental Example 2]

### (Identification of Paraprevotella strain capable of accelerating trypsin decomposition)

It has been previously reported that the microbiota of the large intestine inactivate pancreatic proteases, but bacteria involved in this process have not been identified. Therefore, an attempt was made to isolate and identify bacteria that reduce trypsin from human microbiota.

First, faecal samples collected from 6 healthy Japanese donors (donors A to F) were administered to the GF mice. Subsequently, the trypsin activity in the faeces of the GF mice was measured. FIG. 6 is a graph showing the results of measuring the faecal trypsin activity in the GF mice to which the faecal samples collected from each of the healthy donors (A to F) were administered. Furthermore, in FIG. 6, "n.s." indicates that there is no significant difference, and "***" indicates that there is a significant difference at p < 0.001.

As a result, it was clarified that that the human faecal samples examined differed in their abilities to reduce faecal trypsin activity in the mice. Specifically, the faecal trypsin activity was not decreased in the mice to which the microbiota of the donor B were administered, whereas the faecal trypsin activity was significantly decreased in the mice to which the microbiota of the donors C, D, E, and F were administered.

Subsequently, the mice (mice C#5) to which the microbiota of the donor C was administered were selected, and the cecal contents thereof were collected. Furthermore, these cecal contents were orally administered to new GF mice (GF + C#5 mice). In order to narrow down the microbiota, the GF + C#5 mice were divided into 4 groups. Ampicillin (Amp), metronidazole (MNZ), tylosin (Tyl), or control (no antibiotic, No Abx) was administered to each of the groups via drinking, and the trypsin activity in the faeces of each of the group was measured over time.

FIG. 7 is a graph showing the results of measuring the faecal trypsin activity of the mice in each of the group. Furthermore, in FIG. 7, "n.s." indicates that there is no significant difference, and "***" indicates that there is a significant difference at p < 0.001. As a result, in the GF + C#5 mice which had not been treated with an antibiotic, the faecal trypsin activity was decreased within a few days. Notably, the faecal trypsin activity was more decreased in the Amp-treated group, whereas there was no decrease in the faecal trypsin activity in the MNZ-treated group or the Tyl-treated group. These results indicate that the microbiota of C#5 contains bacteria which decreases the trypsin activity and were concentrated in the Amp-treated group but reduced in the MNZ-treated or Tyl-treated group.

Follow-up study was performed on one (mouse C5-Amp#5) of the Amp-treated mice. The cecal contents were collected and cultured in vitro using various media under anaerobic conditions. Subsequently, 432 different colonies were isolated, and the base sequence of the 16S rRNA gene was analyzed. As a result, the cells were subdivided into 35 unique strains. These 35 strains widely covered the bacterial species colonizing the C5-Amp#5 mouse. When the mixture of the 35 strains of the isolated bacteria (35-mix) was administered to GF mice (GF + 35-mix), the faecal trypsin activity was significantly decreased. The level of this decrease was the same as the decrease in the mice colonized by the microbiota of the original donor C.

Subsequently, in order to narrow down the bacteria that serve as effectors, the 16S rRNA gene sequence of the faecal samples obtained in the above-mentioned antibiotic administration test was analyzed. A Spearman's rank correlation test was performed to evaluate the relativity between the relative abundance of each of the 35 strains and a decrease in the trypsin activity. As a result, 14 strains out of the 35 strains showed a negative correlation with the trypsin activity (ρ ≤ -0.3).

Subsequently, gnotobiotic mice were produced to compare the effects of the 14 bacteria and the remaining 21 bacteria. As a result, in GF + 14-mix mice, the same level of a potent decrease in the trypsin activity in the faeces was shown as in the GF + 35-mix mice, whereas GF + 21-mix mice did not decrease the activity. Subsequently, 9 strains significantly associated with a decrease in trypsin activity were further selected from this 14-mix (ρ ≤ -0.5, p < 0.05).

The colonization of the GF mice by 9-mix showed the same level of a potent decrease in the trypsin activity in the faeces as in the mice colonized by the 14-mix. Finally, the 9-mix was divided into 3-mix consisting of three Bacteroidales species, and 6-mix consisting of non-Bacteroidales species.

It was clarified that the 3-mix consisting of *Paraprevotella clara* (*P. clara*, strain ID: 1C4), *Bacteroides uniformis* (*B. uniformis*, strain ID: 3H3), and *Parabacteroides merdae* (*P. merdae*, strain ID: 1D4) was sufficient to decrease the faecal trypsin activity in vivo, and that the non-Bacteroidales mix with 6 species was completely ineffective in decreasing the faecal trypsin activity.

In order to specify the bacteria that decompose trypsin, each of the strains of the 9-mix was mixed with recombinant mouse trypsin (to which rmPRSS2 and C-terminal His-tag were added) and incubated to measure the decomposition of trypsin by western blot.

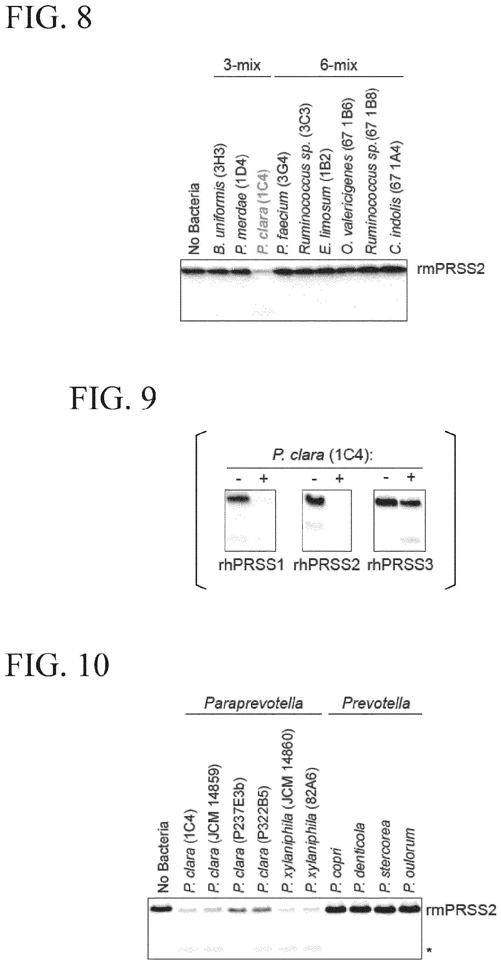

FIG. 8 is a photograph showing the western blot results. As a result, only *P. clara* (strain ID: 1C4) reduced trypsin. Consistent with this result, the mixture of *B. uniformis* 3H3 and *P. merdae* 1D4 (the mixture from which *P. clara* (1C4) was excluded from the 3-mix), or 34-mix did not show the ability to decrease the faecal trypsin activity in vivo.

Based on the above description, it was determined that, contrary to the initial hypothesis that microbiota communities are required for decreasing trypsin activity, only *P. clara* (1C4) is required for decreasing trypsin activity.

Subsequently, recombinant human trypsin isoforms PRSS1, PRSS2, and PRSS3 (rhPRSSl-3) were mixed with *P. clara* (1C4) and incubated to analyze the decomposition of human trypsin by western blot.

FIG. 9 is a photograph showing the western blot results. In FIG. 9, the symbol "-" indicates that *P. clara* was not added, and the symbol "+" indicates that *P. clara* was added. As a result, it was clarified that *P. clara* can promote the reduction of three known human trypsins (PRSS1 and PRSS2, or PRSS3 in a lower level) in addition to mice trypsin.

Subsequently, whether or not the effect of decreasing trypsin activity by *P. clara* was strain-specific was examined. Specifically, recombinant mouse PRSS2 (rmPRSS2) was incubated in vitro with the strain of the genus *Paraprevotella* and the strain of the genus *Prevotella* to analyze the decomposition of rmPRSS2 by western blot. *P. clara* strains (JCM 14859, P237E3b, P322B5) and *P. xylaniphila* strains (JCM 14860, 82A6) were used as the strain of the genus *Paraprevotella. Prevotella copri*, *Prevotella denticola*, *Prevotella stercorea*, and *Prevotella oulorum* were used as the strain of the genus *Prevotella.* FIG. 10 is a photograph showing the western blot results. In FIG. 10, the symbol "*" indicates the position of the cleaved fragment of PRSS2 detected by an anti-PRSS2 antibody.

*Paraprevotella* is a recently identified genus of *Prevotellaceae* and includes only two types of *P. clara* and *Paraprevotella xylaniphila.* Accordingly, the *P*. *xylaniphila* (JCM 14860) strain and the *P. xylaniphila* (82A6) strain which were isolated from healthy human faecal samples were examined.

As a result, it was clarified that three other *P. clara* strains (JCM 14859, P237E3b, P322B5) isolated from the faecal samples collected from healthy subjects including non-Japanese donors had the effect of reducing trypsin in common. In addition, it was clarified that both the *P. xylaniphila* (JCM 14860) strain and the *P. xylaniphila* (82A6) strain exhibit a potent trypsin-reducing ability similar to that of the *P. clara* strains.

On the other hand, all of the bacteria of the genus *Prevotella* (*Prevotella copri*, *Prevotella denticola*, *Prevotella stercorea*, and *Prevotella oulorum*), which have a close lineage with *Paraprevotella*, did not decrease the trypsin activity.

These results indicate that bacteria belonging to the genus *Paraprevotella* are a representative constituent element of human microbiota having a trypsin-decomposing ability.

### [Experimental Example 3]

### (Self-decomposition of trypsin by type IX secretion system-dependent polysaccharide-binding molecule)

The substrate specificity of *P. clara* was examined. Specifically, the cecal contents of the GF mice containing a large amount of trypsin together with various proteins were subjected to ex vivo incubation together with *P. clara* (1C4). Subsequently, the abundance of peptides derived from each protein was examined by peptidome analysis by LC-MS. As a result, among 7614 peptides derived from 276 proteins, trypsin was only one protein that exhibited a pattern of a clear increase in peptide concentration over time in the presence of *P. clara.*

These results indicate that *P. clara* has a narrow substrate specificity and high activity with respect to trypsin. In addition, when the reaction rate of trypsin decomposition by *P. clara* was examined, it was clarified that the reaction gradually occurs stoichiometrically.

In addition, the *P.* clara-mediated trypsin decomposition occurred only in the presence of a sufficient amount of divalent cations (for example, Ca²⁺). Accordingly, it was clarified that this decomposition is mediated by an enzyme (protease). However, this decomposition was not observed when trypsin was incubated with the culture supernatant of *P. clara.* Furthermore, when the live bacteria of *P. clara* or the filtered supernatant of *P. clara* was incubated together with a protease substrate (fluorescein-labeled casein), no proteolytic activity was detected.

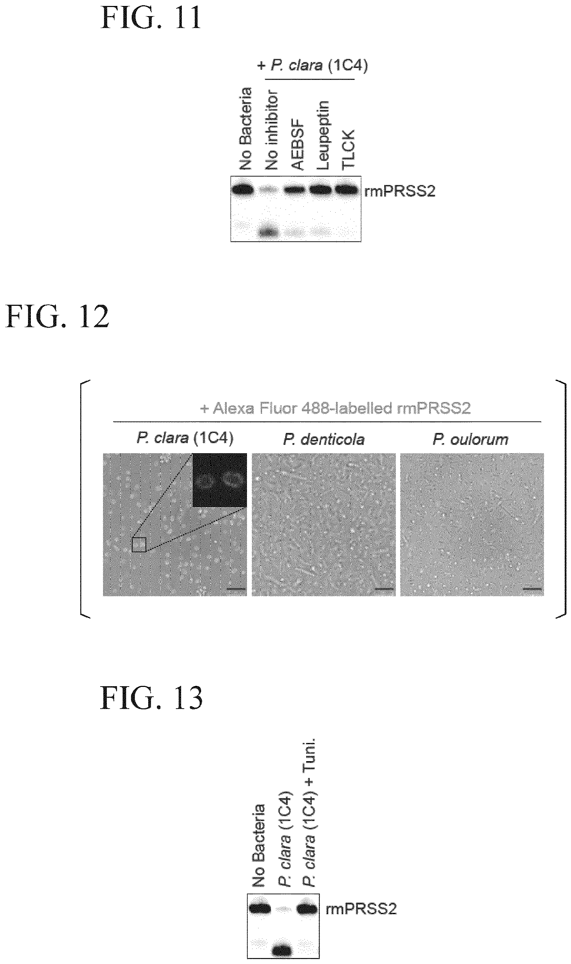

FIG. 11 is a photograph showing the results of analyzing the decomposition of recombinant mouse PRSS2 (rmPRSS2) by western blot after pretreating rmPRSS2 with a protease inhibitor and then incubating with *P. clara* (1C4). As a result, it was clarified that the trypsin-decomposing ability of *P. clara* was lost by pretreatment with AEBSF or leupeptin, which are a serine protease inhibitor, and further with TLCK, which is a specific trypsin inhibitor. This result indicates that the trypsin decomposition by *P*. *clara* is mediated by trypsin-dependent autolysis.

Subsequently, for the purpose of elucidating the mechanism by which *P. clara* promotes the autolysis of trypsin, trypsin was labeled with Alexa Fluor 488, and the interaction of trypsin and *P. clara* was visualized. FIG. 12 is a photograph showing the results of observing the binding of rmPRSS2 to the surface of bacteria with a confocal microscope after incubating Alexa Fluor 488-labeled rmPRSS2 with each of *P. clara* (1C4) and two *Prevotella* species, which are *P. denticola* and *P. oulorum.* In FIG. 12, the black square is an enlarged image of *P. clara* (1C4).

As a result, the fluorescent-labeled trypsin was observed to accumulate on the surface of *P. clara* within minutes. On the other hand, no accumulation of trypsin was observed in *Prevotella denticola* and *Prevotella oulorum.* Based on these results, it was thought that the trypsin decomposition occurred on the surface of *P. clara* via trypsin-binding surface molecules, suggesting that the trypsin-binding surface molecules promoted the accumulation and autolysis of trypsin.

Subsequently, in order to identify the trypsin-binding surface molecules of *P. clara,* a treatment was performed with disuccinimidyl sulfoxide (DSSO) as a chemical crosslinking agent that captures the complex of trypsin (His-tag rmPRSS2) and *P. clara-*derived molecules. Subsequently, the complex formed by crosslinking of rmPRSS2 and *P.* clara-derived molecules was analyzed by western blot.

As a result, by the DSSO treatment, a new band of a high-molecular-weight amount (up to 250 kDa) that was blotted with an anti-His-tag antibody appeared. This band indicates the presence of a high-molecular-weight complex containing trypsin.

Although mass spectrometry was not sensitive enough to detect crosslinked peptides derived from this complex, a band around 250 kDa that was smeared in size indicated that trypsin was interacting with heterogeneous molecules.

In *Bacteroidetes* including *Paraprevotella*, it is known that glycan complexes are modified on the cell surface. For this reason, it was thought that glycan-containing molecules present on the surface of *P. clara* are involved in the binding and decomposition of trypsin.

In order to examine this hypothesis, inhibitors targeting the sugar chain synthesis of *P. clara* were used. First, *P. clara* was treated with tunicamycin which is an inhibitor targeting a WecA-like transferase that mediates the first step in O-glycan formation of bacterial lipopolysaccharides (LPS).

FIG. 13 is a photograph showing the results of analyzing the decomposition of rmPRSS2 by western blot after incubating *P. clara* (1C4), which had been pretreated with tunicamycin (Tuni.) or a vehicle control, with rmPRSS2. As a result, it was clarified that no decomposition of trypsin was observed in *P. clara* treated with tunicamycin.

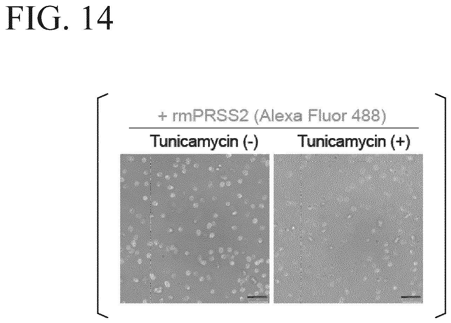

FIG. 14 is a photograph showing the results of observing the binding of rmPRSS2 to the surface of bacteria with a confocal microscope after incubating each of *P. clara* (1 C4) subjected to a tunicamycin treatment or *P. clara* (1 C4) not subjected to a tunicamycin treatment with Alexa Fluor 488-labeled rmPRSS2. In FIG. 14, "Tunicamycin (+%)" indicates the results of performing the tunicamycin treatment, and "Tunicamycin (-)" indicates the results of not performing the tunicamycin treatment. As a result, it was clarified that no accumulation of trypsin on the surface of *P. clara* was recognized in *P. clara* subjected to the tunicamycin treatment. Similarly, when *P. clara* was treated with 2-fluoro-L-fucose (2F-Fuc), which is a fucose transferase inhibitor that broadly inhibits the synthesis of fucose-containing sugar chains, both the binding of trypsin to the *P. clara* surface and the decomposition of trypsin were inhibited.

T9SS (type IX secretion system) is a bacterial mechanism that transports a protein having a conserved C-terminal domain to the surface across the outer membrane in cooperation with the Sec system. T9SS plays a role of removing the C-terminal domain by protease activity such as sortase and binding the protein transported to the outside of the cell to a surface polysaccharide.

The inventors of the present invention performed the following examination while presuming that the cell surface protein secreted by T9SS is responsible for the recruitment and decomposition of trypsin. First, a gene sequence presumed to be contained in T9SS was identified in the genomes of *P. clara* and *P. xylaniphila* species.

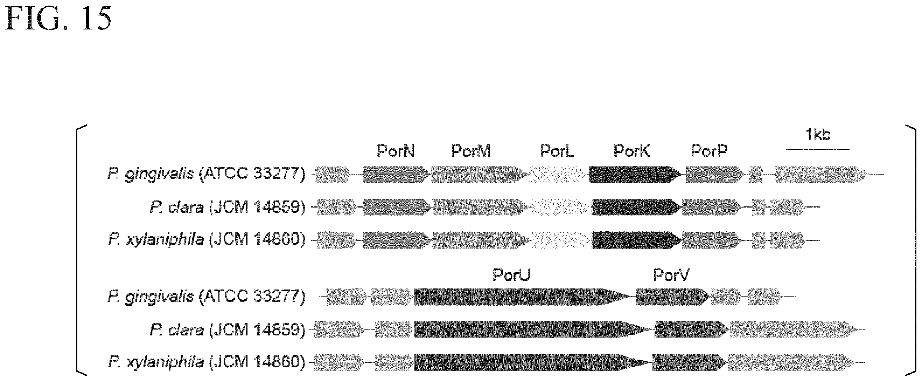

FIG. 15 is a schematic diagram showing the alignment of the genetic organization of T9SS in the genome of *P. clara* (JCM 14859), *P. xylaniphila* (JCM 14860), and *P. gingivalis* (ATCC 33277).

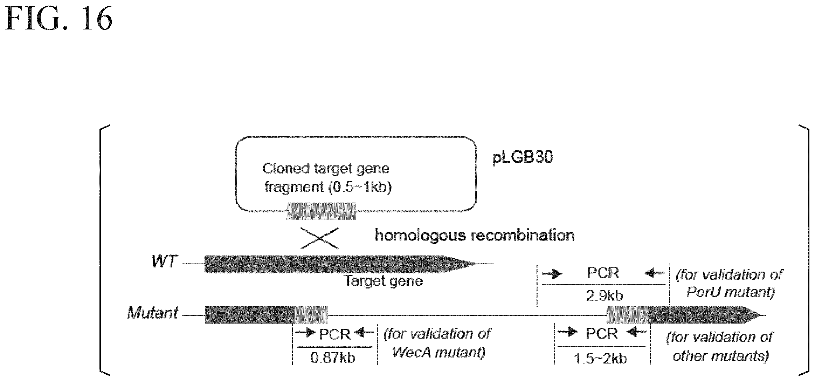

Subsequently, a mutant *P. clara* (JCM 14859) strain from which the expression of PorU, which is a basic constituent factor of T9SS, was deleted was produced by homologous recombination of the plasmid sequence. FIG. 16 is a schematic diagram showing homologous recombination that deletes the expression of PorU.

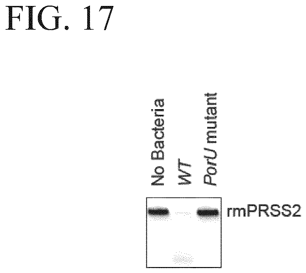

FIG. 17 is a photograph showing the results of analyzing the decomposition of rmPRSS2 by western blot after incubating the mutant *P. clara* (JCM 14859) with rmPRSS2. In FIG. 17, "PorU mutant" indicates the results of the mutant *P. clara* (JCM 14859), and "WT" indicates the results of a wild-type *P. clara* (JCM 14859). As a result, in the mutant *P. clara* (JCM 14859), trypsin decomposition was completely deleted, and it was clarified that a T9SS-dependent surface protein is involved in trypsin decomposition.

Subsequently, in order to identify a cell surface protein having an action effect on trypsin decomposition, a proteome analysis of the culture supernatant of *P. clara* was performed in the presence or absence of tunicamycin. As a result, 20 bacteria-derived proteins were found in the culture supernatant of *P. clara* subjected to the tunicamycin treatment.

Therefore, by introducing a plasmid sequence into each of the 20 target loci or by removing gene clusters (Δ03048 to 03053), the series of mutant *P. clara* strains in which the synthesis of tunicamycin-sensitive proteins was inhibited were produced.

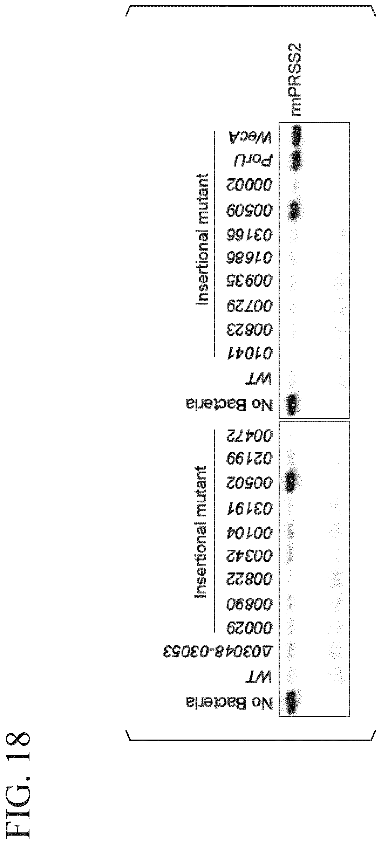

FIG. 18 is a photograph showing the results of analyzing, by western blot, the decomposition of rmPRSS2 mediated by a wild-type (WT) *P. clara* (JCM 14859) or the series of mutant *P. clara* (JCM 14859). In FIG. 18, "Δ03048-03053" indicates the results of the mutant *P. clara* from which the gene cluster was removed, and each of "00029", "00890", "00822", "00342", "00104", "03191", "00502", "02199", "00472", "01041", "00823", "00729", "00935", "01686", "03166", "00509", "00002", "PorU", and "WecA" indicates the results the mutant *P. clara* from which the described gene was deleted by plasmid insertion.

As a result, by disrupting the gene encoding the 00502 protein (UniProtKB ID: G5SNC9, Omp28-related extracellular membrane protein) of the 00509 protein (UniProtKB ID: G5SNC1, protein of unknown function), it was clarified that trypsin decomposition was lost in vitro as in the case of PorU or WecA (target factor of tunicamycin)-deficient mutant strains.

Subsequently, *P. clara* strains (Δ00502 and Δ00509) from which the 00502 protein of the 00509 protein was deleted were produced in place of the insertion mutant, and the same examination was performed.

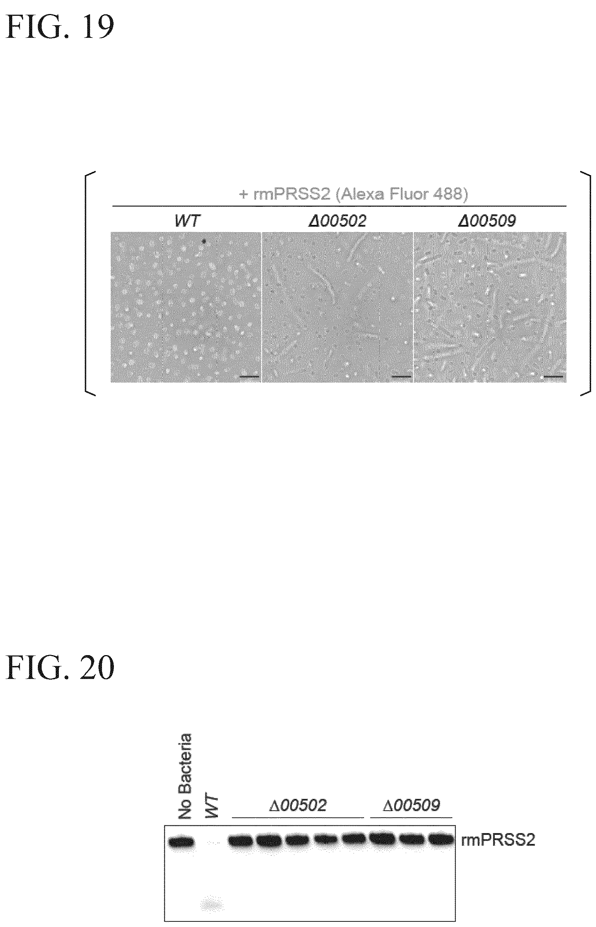

FIG. 19 is a photograph showing the results of observing the binding of rmPRSS2 to the surface of bacteria with a confocal microscope after incubating each of Δ00502 and Δ00509 with Alexa Fluor 488-labeled rmPRSS2. As a result, it was clarified that the recruitment of trypsin was deleted from both the Δ00502 strain and the Δ00509 strain.

FIG. 20 is a photograph showing the results of analyzing the decomposition of rmPRSS2 by western blot after incubating the Δ00502 strain and the Δ00509 strain with rmPRSS2. In FIG. 20, "WT" indicates the results of a wild-type *P. clara* (JCM 14859). As a result, it was clarified that trypsin decomposition was deleted from both the Δ00502 strain and the Δ00509 strain.

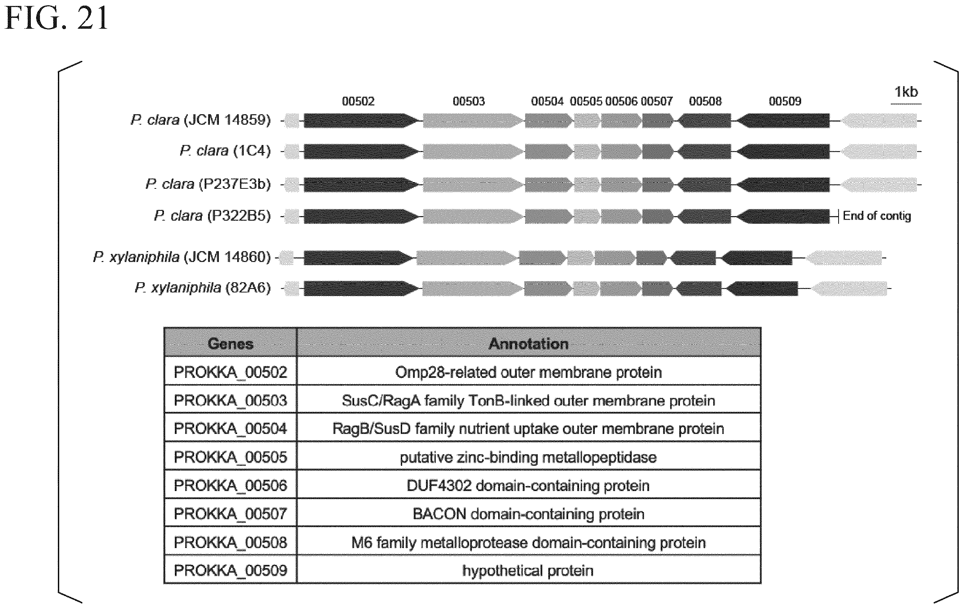

Subsequently, the genome sequence of *Paraprevotella* strains was analyzed. FIG. 21 is a schematic diagram showing the genome sequence of the *Paraprevotella* strains. As a result, it was clarified that all of the strains having a trypsin-decomposing ability had a 00502 gene and a 00509 gene. In contrast, none of the *Prevotella* species examined did not have homologues of a 00502 gene and a 00509 gene.

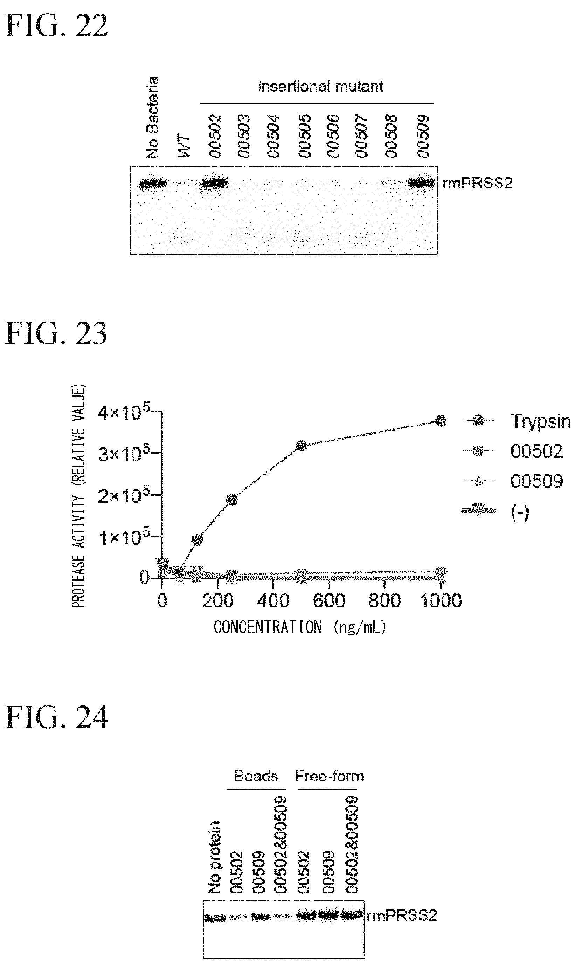

In addition, between the *Paraprevotella* strains, 00503 to 00508 genes were conserved at sites distant from the locus of the 00502 gene and the 00509 gene. FIG. 22 is a photograph showing the results of analyzing the decomposition of rmPRSS2 by western blot after incubating *P. clara* mutant strains, from which each of 00502 to 00509 genes had been deleted, with rmPRSS2. As a result, the *P. clara* mutant strains, from which the 00503 to 00508 genes had been deleted, had a trypsin-decomposing activity, and their involvement in the trypsin-decomposing process was ruled out.

### [Experimental Example 4]

### (Examination of 00502 protein and 00509 protein)

Recombinant 00502 protein and 00509 protein were produced. *Escherichia coli* into which an expression vector of 00502 protein or 00509 protein had been incorporated were treated with isopropyl-β-thiogalactopyranoside (IPTG) to induce the expression of the recombinant protein, and the expressed recombinant 00502 protein or 00509 protein was purified from a cell lysate with magnetic agarose beads.

FIG. 23 is a graph showing the results of measuring the protease activity of the recombinant 00502 protein or 00509 protein by cleavage of FITC-labeled casein. Trypsin (1 ng/µL) was used as a positive control. In FIG. 23, "(-)" indicates that no protein was added. The protease activity was expressed as a change in relative fluorescence units. As a result, it was clarified that the recombinant 00502 protein and 00509 protein did not have a protease activity.

FIG. 24 is a photograph showing the results of analyzing the decomposition of rmPRSS2 by western blot after incubating free-form recombinant 00502 protein and 00509 protein with rmPRSS2 of incubating recombinant 00502 protein and 00509 protein, which were bound to microbeads, with rmPRSS2.

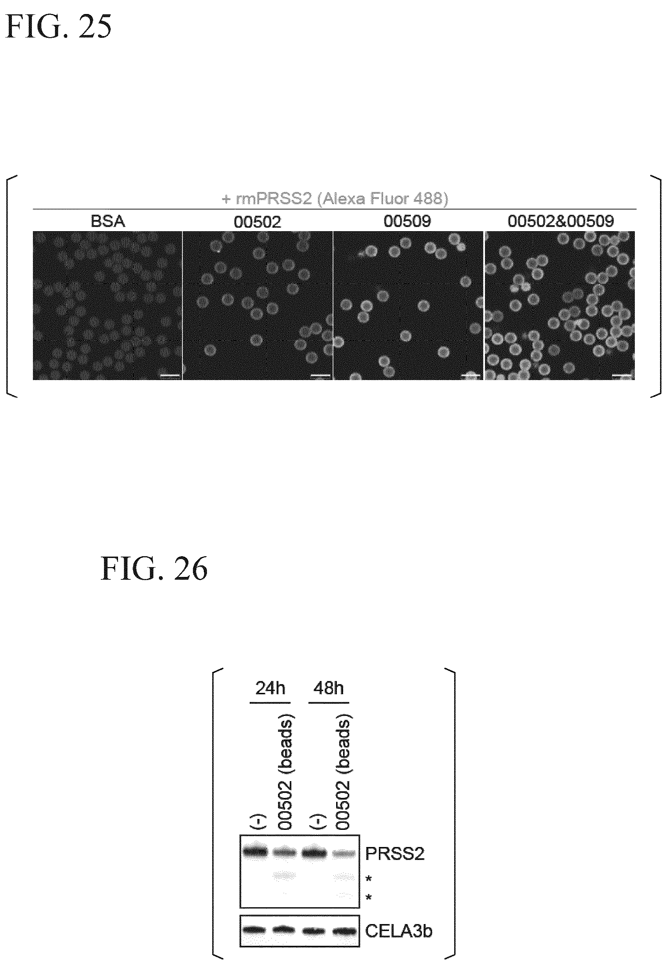

FIG. 25 is a photograph showing the results of observing the binding state of rmPRSS2 and protein-binding beads with a confocal microscope after incubating the recombinant 00502 protein, the recombinant 00509 protein, and bovine serum albumin (BSA), which were bound to microbeads, together with rmPRSS2. The scale bar is 5 µm. BSA was used as a negative control.

As a result, it was clarified that the free-form 00502 protein and 00509 protein did not exhibit a trypsin-decomposing activity. On the other hand, the recombinant 00502 protein bound to microbeads exhibited effective recruitment and decomposition of trypsin.

FIG. 26 is a photograph showing the results of analyzing the ex vivo decomposition of trypsin after 24 hours or 48 hours by western blot after incubating the cecal contents of the GF mice with a culture medium control (-) or the recombinant 00502 protein bound to microbeads. In FIG. 26, the symbol "*" indicates the position of the cleaved fragment of PRSS2 detected by an anti-PRSS2 antibody. As a result, predominant trypsin decomposition was detected by the recombinant 00502 protein bound to microbeads.

These results support the model of the inventors of the present invention in which the 00502 protein acts as a scaffold for trypsin binding and promotes the self-decomposition of trypsin. Furthermore, it was clarified that although trypsin was effectively bound to the recombinant 00509 protein bound to microbeads, trypsin decomposition did not occur.

From these results, it was suggested that the 00502 protein is a major constituent factor exerting the effect of the recruitment and autolysis of trypsin, whereas the 00509 protein has an action of assisting the recruitment of trypsin.

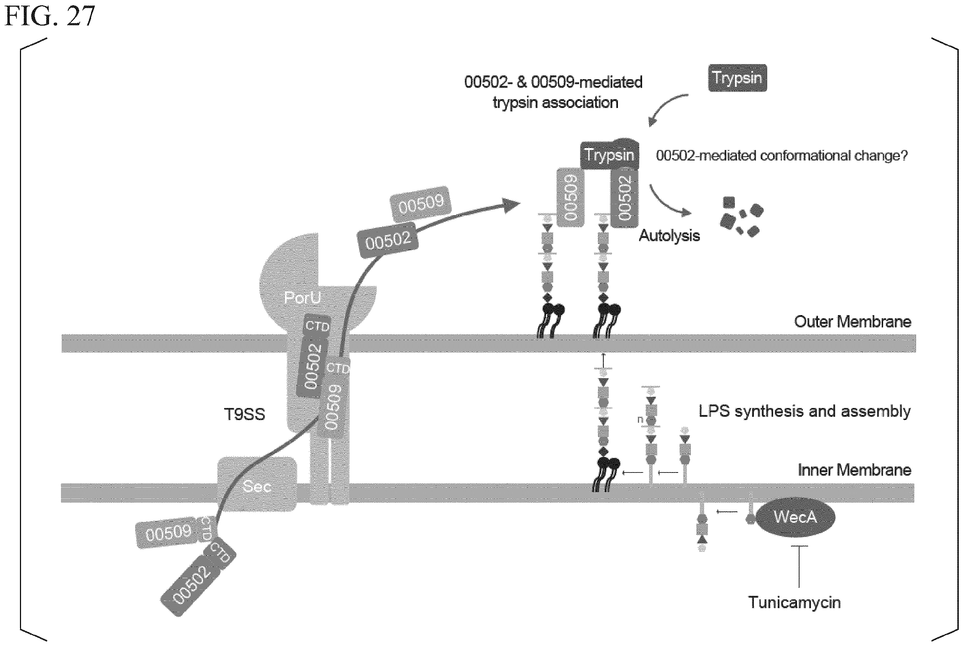

FIG. 27 shows a model of trypsin decomposition mediated by bacteria of the genus *Paraprevotella,* proposed by the inventors of the present invention. In FIG. 27, "Sec" indicates a SEC system in which proteins are excreted outside of the cytoplasmic membrane.

As shown in FIG. 27, the 00502 protein and the 00509 protein are transported across the extracellular membrane of *Paraprevotella* via the type IX secretion system (T9SS). PorU is a basic T9SS constituent factor and decomposes the C-terminal domain (CTD) of a T9SS-dependent protein to connect the LPS molecules of the bacteria of the genus *Paraprevotella* with their proteins. WecA mediates the first step in LPS O-glycan synthesis and results in the release of T9SS-dependent proteins by the disruption of the function of WecA due to the tunicamycin treatment, for example. The 00502 protein functions as a major effector constituent factor for the recruitment and autolysis of trypsin. On the other hand, the 00509 protein assists the recruitment of trypsin.

The exact mechanism by which the 00502 protein, rather than the 00509 protein, promotes the autolysis of trypsin is still unclear, but it is presumed that the binding of trypsin to the 00502 protein may change the structure of trypsin, thereby making the autolysis easy.

### [Experimental Example 5]

### (Colonization by P. clara strain contributes to maintenance of IgA)

In order to confirm the contribution of the 00502 and 00509 proteins in vivo to trypsin decomposition, GF mice which had been colonized by three *P. clara* (JCM 14859) strains (a wild-type (WT), Δ00502, or Δ00509) were analyzed.

Since the *P. clara* strains as a single strain did not colonize the mice, the mice were inoculated with the *P. clara* strain together with two strains not decomposing trypsin (2-mix: *Bacteroides uniformis* 3H3 and *Parabacteroides merdae* 1D4). By inoculation together with the 2-mix, all of the three *P. clara* strains effectively colonized the intestines of the mice.

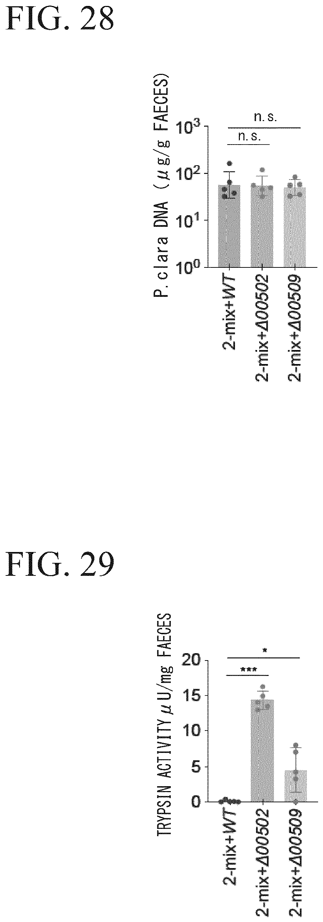

FIG. 28 is a graph showing the results of quantitatively determining the DNA of the *P. clara* strain (wild-type (WT), Δ00502, or Δ00509) in the faeces of the GF mice inoculated with the P. clara strains together with the 2-mix. In FIG. 28, "n.s." indicates that there is no significant difference. As a result, it was clarified that the deletion of the 00502 gene or the 00509 gene did not affect the abundance of the *P. clara* strains in vivo.

FIG. 29 is a graph showing the results of measuring the faecal trypsin activity of the GF mice inoculated with the *P. clara* strain (wild-type (WT), Δ00502, or Δ00509) together with the 2-mix. In FIG. 29, "*" indicates that there is a significant difference at p < 0.05, and "***" indicates that there is a significant difference at p < 0.001. As a result, consistent with the results in vitro, the mice colonized by the Δ00502 *P. clara* strain had a high trypsin activity in the faeces, whereas the mice colonized by the Δ00509 *P. clara* strain showed a partially decreased trypsin activity.

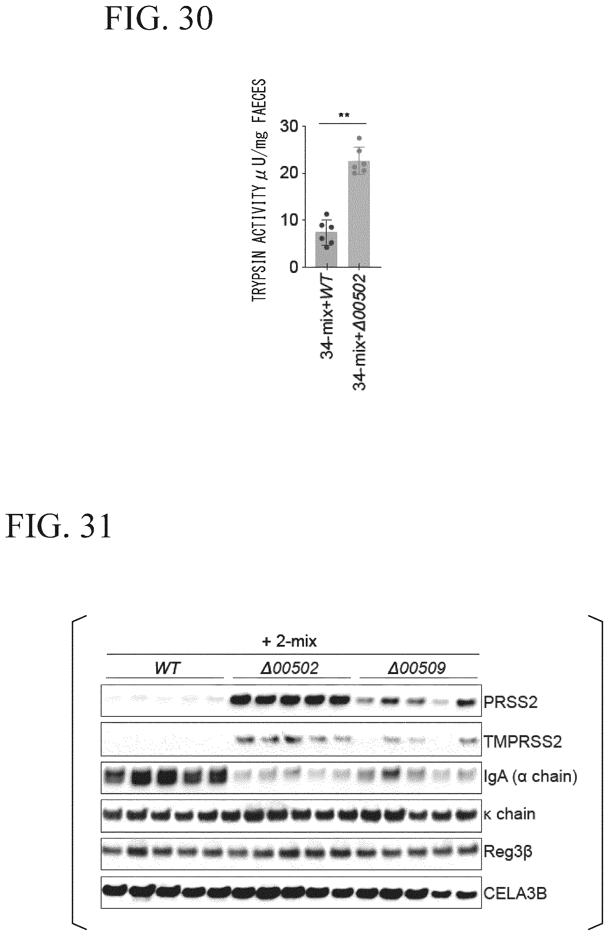

Subsequently, the importance of 00502 was examined under conditions in which more complex microbiota communities were present. FIG. 30 is a graph showing the results of measuring the faecal trypsin activity of the GF mice inoculated with the *P*. *clara* strain (wild-type (WT), Δ00502) together with the above-mentioned 34-mix (from which *P. clara* (1C4) was excluded from the 35-mix). In FIG. 30, "**" indicates that there is a significant difference at p < 0.01. As a result, it was clarified that the mice colonized by the Δ00502 *P. clara* had a higher faecal trypsin activity than in the mice colonized by the wild-type (WT) *P. clara.*

Based on the above results, it was confirmed that the essential role of the 00502 protein is to promote the trypsin decomposition in vivo.

Subsequently, the influence of the regulation of intestinal trypsin levels by the wild-type (WT) type *P. clara* and the mutant type *P. clara* on important intestinal defense factors such as IgA and antimicrobial peptides was examined.

FIG. 31 is a photograph showing the results of analyzing, by western blot, trypsin (PRSS2), a type II transmembrane serine protease (TMPRSS2), an IgA heavy chain, a κ light chain, Reg3β that is an antimicrobial peptide against gram-negative bacteria, and chymotrypsin like elastase 3B (CELA3B) which are in the faeces of the GF mice inoculated with the *P. clara* strain (wild-type (WT), Δ00502, or Δ00509) together with the 2-mix.

As a result, in the colonization of the mice by the wild-type *P. clara* strain, a larger amount of the IgA heavy chain (α chain) in the faeces was detected than in the colonization of the mice by the Δ00502 or Δ00509 *P. clara* strain. In contrast, because the κ light chain is resistant to trypsin, the κ light chain levels in the faeces were similar between the mice colonized by various types of the *P. clara* strains. In addition, Reg3β, which is an antimicrobial peptide against gram-negative bacteria, was resistant to trypsin decomposition as in the case of the κ light chain.

These results show that the colonization by the *P. clara* strains protects IgA from being decomposed by trypsin in vivo.

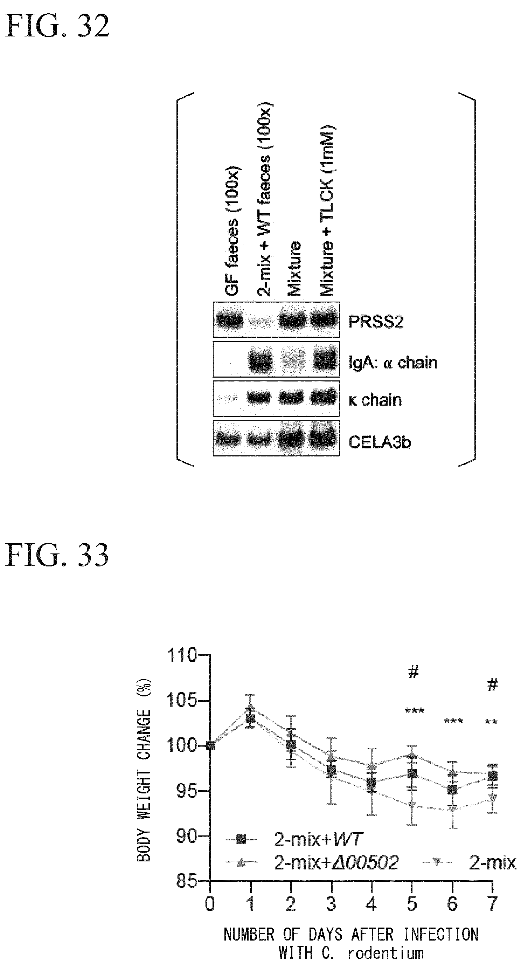

FIG. 32 is a photograph showing the results of analyzing trypsin (PRSS2), an IgA heavy chain, a κ light chain, and chymotrypsin like elastase 3B (CELA3B) by western blot after incubating, at 37°C for 48 hours, the faeces of the GF mice, the faeces of the GF mice inoculated with the wild-type *P. clara* strain together with the 2-mix, a specimen obtained by mixing the above-mentioned two types of the faeces, and a specimen obtained by adding a trypsin inhibitor (TCLK) to the specimen in which the above-mentioned two types of the faeces were mixed.

As a result, it was shown from the results of the first lane that in the faeces of the GF mice, trypsin was contained but the IgA heavy chain was decomposed. In addition, it was shown from the results of the second lane that in the faeces of the GF mice inoculated with the wild-type *P. clara* strain together with the 2-mix, trypsin was reduced but the IgA heavy chain remained. In addition, it was shown from the results of the third lane that the IgA heavy chain remaining in the faeces of the GF mice inoculated with the wild-type *P. clara* strain together with the 2-mix was decomposed by the trypsin remaining in the faeces of the GF mice. In addition, it was shown from the results of the fourth lane that the addition of the trypsin inhibitor inhibited the decomposition of the IgA heavy chain in the lane 3. In addition, it was shown from the results of the third and fourth lanes that the IgA heavy chain is more easily decomposed by trypsin than the κ light chain.

### [Experimental Example 6]

### (Examination of influence of colonization by P. clara strain on infection with enteric pathogenic bacteria)

Whether the *P. clara* strain-mediated trypsin decomposition could maintain an IgA amount even under conditions of infection with enteric pathogenic bacteria was examined.

GF mice were inoculated with the 2-mix or with the wild-type (WT) or Δ00502 *P. clara* strain in addition to the 2-mix, and after 14 days, the mice were infected with the *Citrobacter rodentium* (*C. rodentium*) which is a pathogen of mice mainly infecting the large intestine.

FIG. 33 is a graph showing changes in body weight of the mice in each group after infection with *C*. *rodentium.* In FIG. 33, "**" indicates that there is a significant difference at p < 0.01, and "***" indicates that there is a significant difference at p < 0.001 (2-mix + Δ00502 vs. 2-mix). In addition, "#" indicates that there is a significant difference at P < 0.05 (2-mix + WT vs. 2-mix).

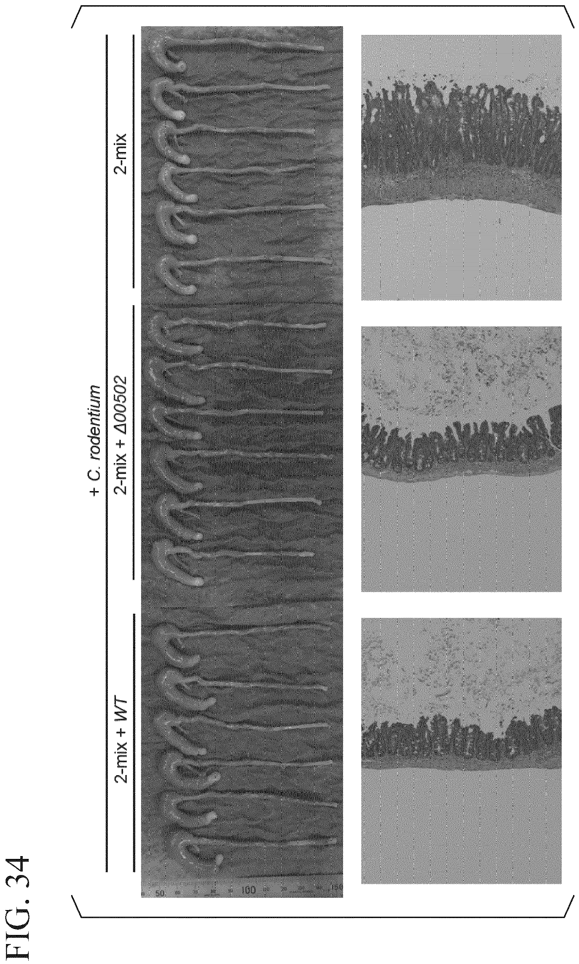

The upper part of FIG. 34 is an image of the large intestine of each of the mice 7 days after infection with *C*. *rodentium.* In the 2-mix group, the cecum was whitish and contracted. The lower part of FIG. 34 is a photomicrograph showing a representative image of hematoxylin and eosin staining of cecal tissue.

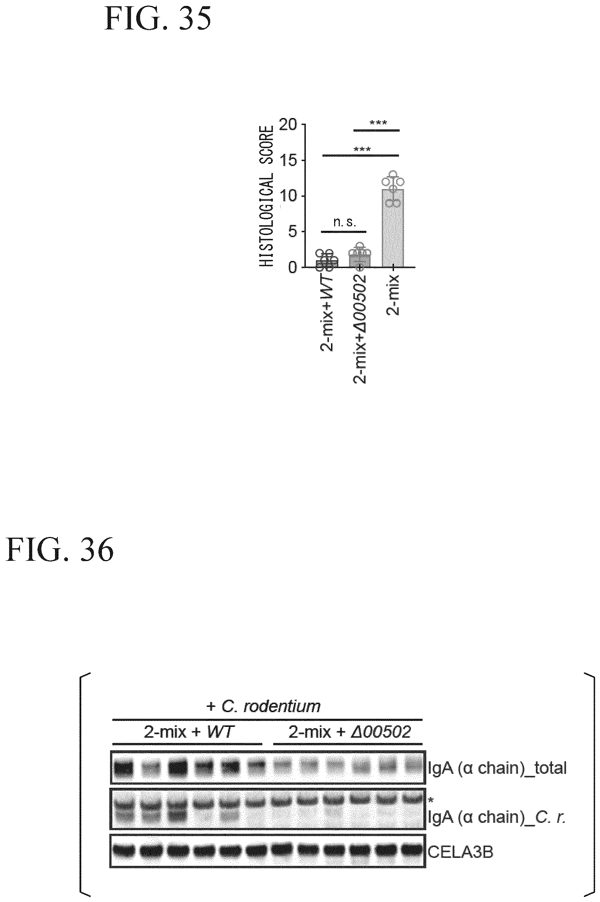

FIG. 35 is a graph showing the histological score of the cecal tissue based on hematoxylin and eosin staining. Furthermore, in FIG. 35, "n.s." indicates that there is no significant difference, and "***" indicates that there is a significant difference at p < 0.001.

As a result, in the 2-mix group, rapid body weight loss and severe cecal inflammation were shown after the infection with *C*. *rodentium.* On the other hand, in the mice colonized by *P. clara,* body weight loss was milder, and cecal inflammation was also milder. This was surprisingly the same in both the 2-mix + WT group and the 2-mix + Δ00502 group. These results suggest that the *P. clara* strains protect against the infection with *C*. *rodentium* by the mechanism independent of trypsin decomposition.

FIG. 36 is a photograph showing the results of analyzing the total IgA, C. *rodentium-specific* IgA, and chymotrypsin like elastase 3B (CELA3B) in faeces by western blot.

As a result, it was clarified that in the mice in the 2-mix + WT group, a high total IgA amount was maintained regardless of the degree of the cecal inflammation, and as compared to the 2-mix + Δ00502 group, fairly large amounts of the *C*. *rodentium-*specific IgA were present from as early as 7 days after the infection with *C*. *rodentium.*

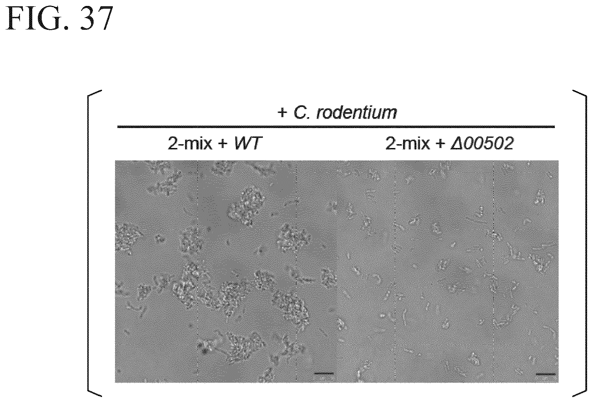

FIG. 37 is a photograph showing the results of evaluating the aggregation effect of faecal IgA by ex vivo incubation of live *C*. *rodentium* and a faecal fluid. As a result, when the faecal microbiota of the 2-mix + WT group and the live bacteria of *C*. *rodentium* were incubated, predominant bacterial aggregation was recognized. This result indicates the presence of the *C*. *rodentium*-specific IgA.

Based on the above description, it was shown that the *P. clara* strains protect pathogen-specific IgA, thereby enhancing an adaptive immune response against the pathogen, in addition to the protective effect by an unknown mechanism against the infection with *C*. *rodentium.*

### [Experimental Example 7]

### (P. clara strain-mediated trypsin decomposition enhances vaccine effect against infection with C. rodentium)

The *P. clara* strain-mediated trypsin decomposition and the consequent protection of pathogen-specific IgA may enhance the oral vaccine effect against intestinal pathogens, thereby further increasing resistance to re-exposure to the same pathogen.

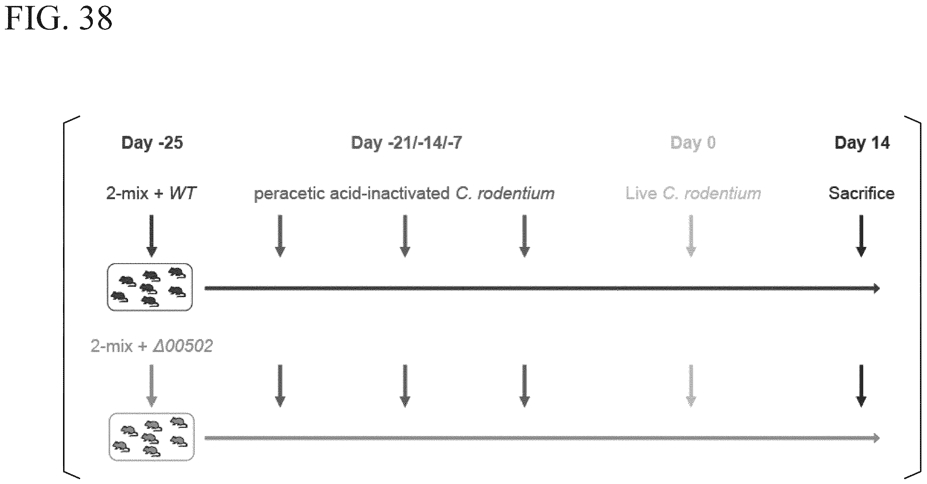

To confirm this hypothesis, the following examination was performed. FIG. 38 is a schematic diagram showing the schedule of the present experimental example. The GF mice colonized by the WT or Δ00502 *P. clara* strain in addition to the 2-mix were orally vaccinated once a week for 3 weeks with *C*. *rodentium* inactivated by peracetic acid. Thereafter, infection with the live *C*. *rodentium* was performed.

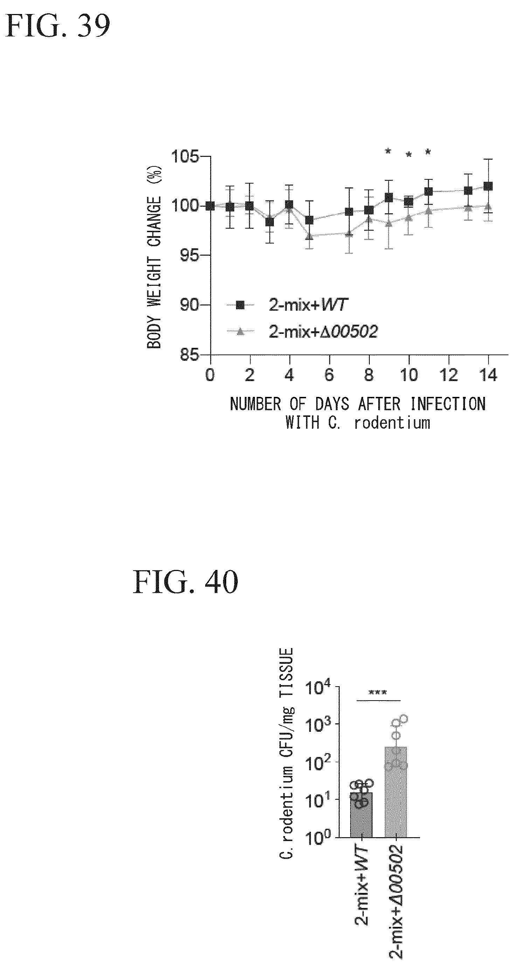

FIG. 39 is a graph showing changes in body weight of the mice after infection with *C*. *rodentium.* In FIG. 39, "*" indicates that there is a significant difference at p < 0.05. As a result, it was clarified that in the mice preliminarily inoculated with the wild-type *P. clara* strain, body weight loss was reduced.

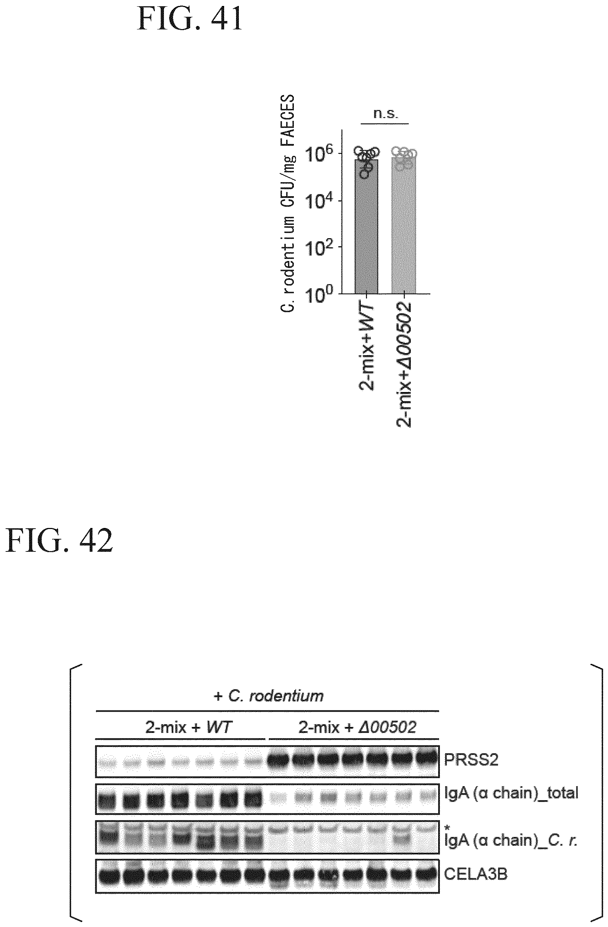

Subsequently, the cecal patch and the luminal contents were recovered from the mice 14 days after the infection with *C*. *rodentium,* and the CFU of *C*. *rodentium* was measured. FIG. 40 is a graph showing the measurement values of the CFU of C. *rodentium* in the cecal patch. FIG. 41 is a graph showing the measurement values of the CFU of *C*. *rodentium* in the luminal contents.

As a result, in both the 2-mix + WT group and the 2-mix + Δ00502 group, although the abundance of *C*. *rodentium* in the cecum was similar, the infiltration of *C*. *rodentium* into the cecal tissue was inhibited in the 2-mix + WT group.

FIG. 42 is a photograph showing the results of analyzing trypsin (PRSS2), total IgA, *C*. *rodentium-specific* IgA, and chymotrypsin like elastase 3B (CELA3B) in the cecal luminal contents by western blot.

As a result, in the cecum of the mice colonized by the wild-type (WT) *P. clara* strain, a significantly high total IgA amount and a significantly high amount of *C*. *rodentium-specific* IgA were detected. Based on this result, it was thought that the effect of the vaccine was great as the excellent protective effect against the invasion of *C*. *rodentium* to the 2-mix + WT group.

The above results support the view of the inventors of the present invention in which delivery of the *P. clara* strains makes it possible to more effectively correspond to intestinal pathogens that a host has previously encountered.

### [Experimental Example 8]

### (P. clara strain protects mice from coronavirus infection)

Trypsin-like proteases such as trypsin and a type II transmembrane serine protease (TMPRSS2) are known to be involved in the proteolytic activation of the spike protein of coronaviruses and in the fusion of virus and host cell membranes.

TMPRSS2, which is expressed as a transmembrane protein in lung and intestinal epithelial cells, is capable of releasing the protease domain by undergoing self-cleaving. Interestingly, the inventors of the present invention found that in Experimental Example 5 described above, the amount of TMPRSS2 in the faeces was reduced in the GF mice engrafted by the wild-type *P. clara* strain. This suggests that the *P. clara* strain has an effect similar to the release of the TMPRSS2 active form in vivo. This indicates that in the intestines, free-form TMPRSS2 may promote coronavirus infection together with trypsin.

A probability of the *P. clara* strain protecting against intestinal infection of coronavirus by decomposition of trypsin and TMPRSS2 was examined. Specifically, the influence of the colonization by the *P. clara* strains on the infection with mouse hepatitis virus (MHV) (mice tropic coronavirus) was examined.

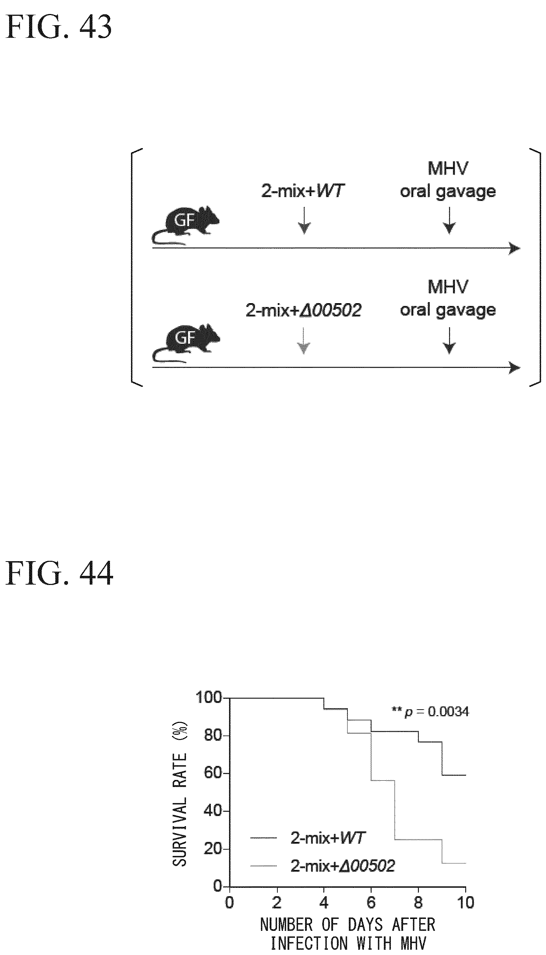

FIG. 43 is a schematic diagram showing an outline of a MHV infection experiment. GF mice was inoculated with the WT or Δ00502 *P. clara* (JCM 14859) strain in combination with the 2-mix, and after 14 days, oral infection with MHV was performed.

FIG. 44 is a graph showing the survival curves of the mice after MHV infection. As a result, it was clarified that the colonization by the wild-type *P. clara* strain resulted in prolonged survival of the mice after a fatal infection with MHV.

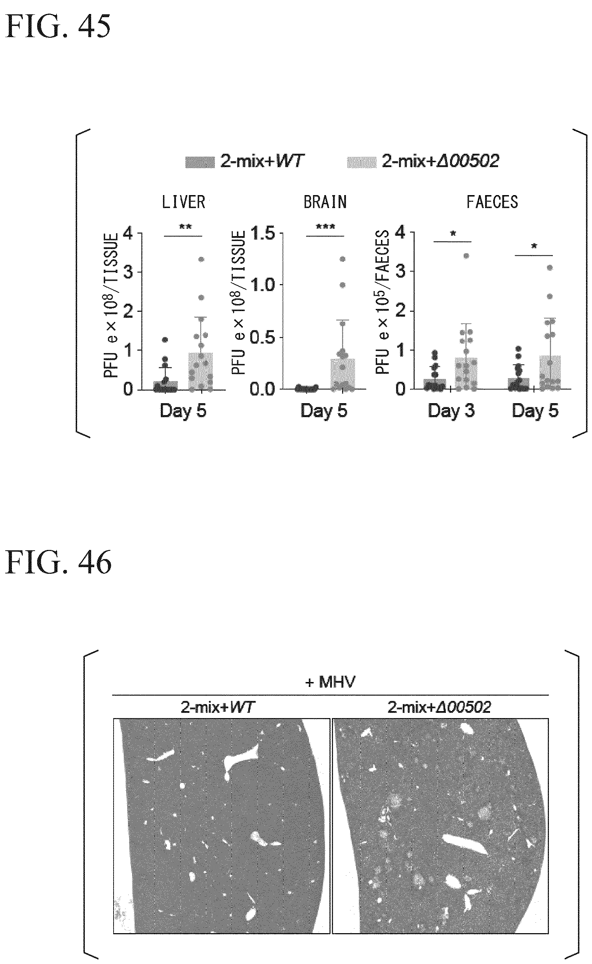

FIG. 45 is a graph showing the results of measuring viral titers of MHV in liver, brain, and faeces. In FIG. 45, "*" indicates that there is a significant difference at p < 0.05, "**" indicates that there is a significant difference at p < 0.01, and "***" indicates that there is a significant difference at p < 0.01.

As a result, it was clarified that the colonization by the wild-type *P. clara* strain significantly prevented the spread of the virus in the liver and the brain. In addition, the number of the virus particles excreted in the faeces was significantly smaller in the mice in the 2-mix + WT group than in the mice in the 2-mix + Δ00502 group.

FIG. 46 is a representative image showing the results of hematoxylin and eosin staining of liver tissue of the mice in each group. Histological analysis clarified that the mice colonized by the wild-type *P. clara* strain were protected from the necrotizing liver pathology caused by MHV.

The above result indicates that upon infection with coronavirus, the carrier of the *P. clara* strain brings a protective effect to the host.

### [Experimental Example 9]

### (Detection of genus Paraprevotella and related genes in human microbiota)

The abundance and the prevalence of the genus *Paraprevotella,* and trypsin-related 00502 genes and 00509 genes were analyzed by newly creating an intestinal bacterial gene catalog consisting of approximately 6 million non-redundant complete genes acquired from six geographically distinct cohorts.

As a result of performing a homology search at the USEARCH ublast (protein level) on the non-redundant intestinal bacterial gene catalog having 5,929,528 genes, the minimum e-value was aggregated to 0.1 hits.

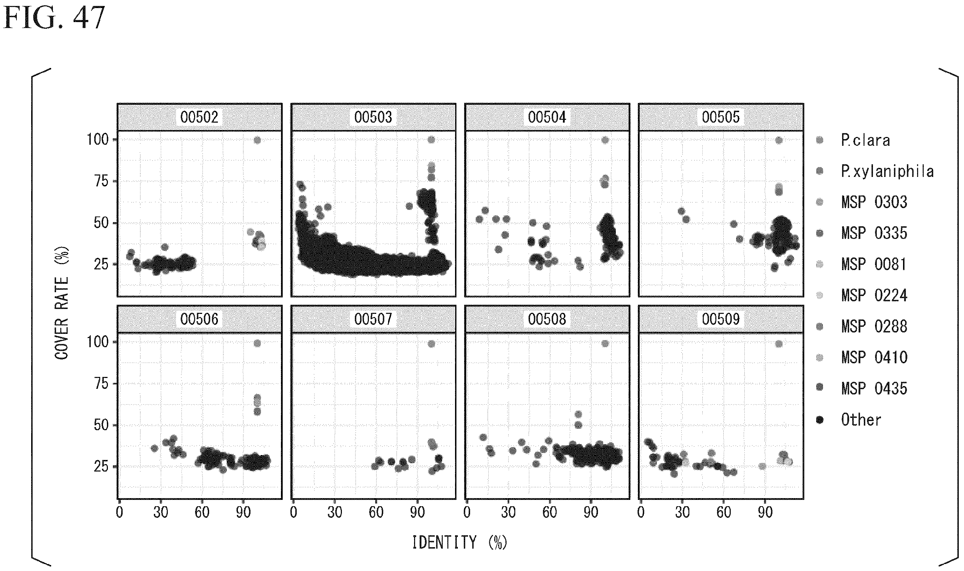

FIG. 47 is a diagram showing the homologues of genes (00502 gene and 00509 gene) associated with trypsin decomposition and the species encoding the same, which were searched with a calculator.

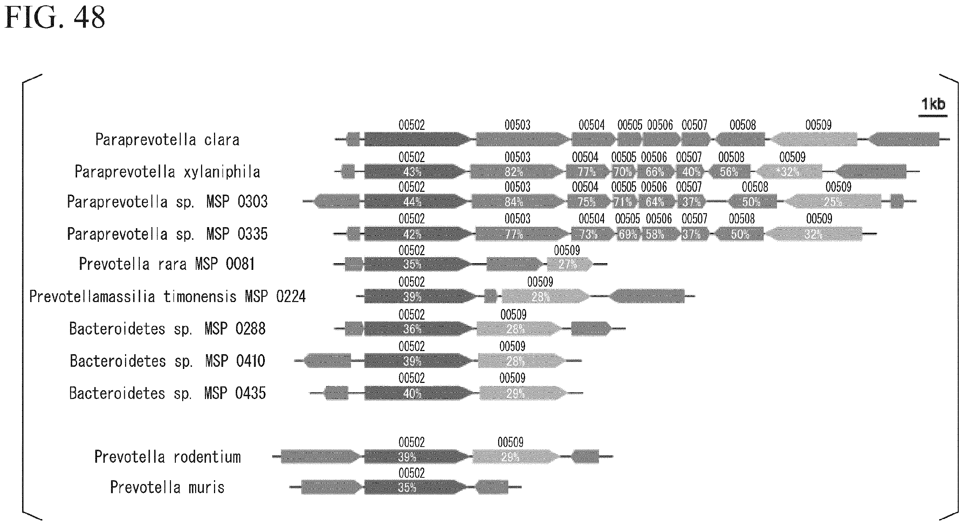

FIG. 48 is a diagram showing the structure of the loci of genes associated with trypsin decomposition in each bacterial species, and the sequence identity (%) with the genes of the *P. clara* strains.

As a result, first, whether or not the 00502 gene of the *P. clara* strain and the homologue of the 00502 gene of the *P. xylanphila* strain were the same as or almost the same as each other was confirmed. Similarly, whether or not the 00509 gene of the *P*. *clara* strain and the homologue of the 00509 gene of the *P. xylanphila* strain were the same as or almost the same as each other was confirmed. Furthermore, also regarding the 00503 to 00508 genes positioned between the 00502 gene and the 00509 gene, whether or not the gene of the *P. clara* strain and the homologue of the gene of the *P. xylanphila* strain were the same as or almost the same as each other was confirmed.

In addition, two metagenome species (MSP 0303 and MSP 0335) having the homologues of the 00502 to 00509 genes were identified, and it was confirmed that they may belong to the genus *Paraprevotella.*

Two metagenome species (MSP 0303 and MSP 0335) annotated to the genus *Paraprevotella* encoded all or almost all homologues of the 00502 to 00509 genes of the *P. clara* strain. The five MSPs (MSP 0081, MSP 0224, MSP 0288, MSP 0410, and MSP 0435) annotated with *Bacteroidetes* encoded the homologues of the 00502 gene and the 00509 gene, but lacked the homologues of the 00503 to 00508 genes.

In addition, it was thought that *Prevotella muris* did not have the homologue of the 00509 gene.

In the metagenomics analysis, the genus *Paraprevotella* showed a relative abundance of up to 3% on average, the abundance of which varies greatly from cohort-to-cohort (7% to 50% of samples). The *P. clara* strain is the most abundant species, followed by *P. xylaniphilla.*

These data suggest that *Paraprevotella* species make up a significant portion of the microbiota in humans, which may be associated with individual differences in susceptibility to intestinal pathogens for infection.

It is thought that human trypsin concentrations are regulated by a bacterial species that decomposes trypsin, such as *Paraprevotella,* as in the case of mice. It has been reported that the faecal trypsin concentration is increased in patients suffering from inflammatory bowel disease (IBD) such as ulcerative colitis (UC) and Crohn's disease (CD). Therefore, the trypsin activity in the faeces of patients in a non-IBD control group (healthy subjects) from a Japanese cohort, an ulcerative colitis (UC) group, and a Crohn's disease (CD) group was measured.

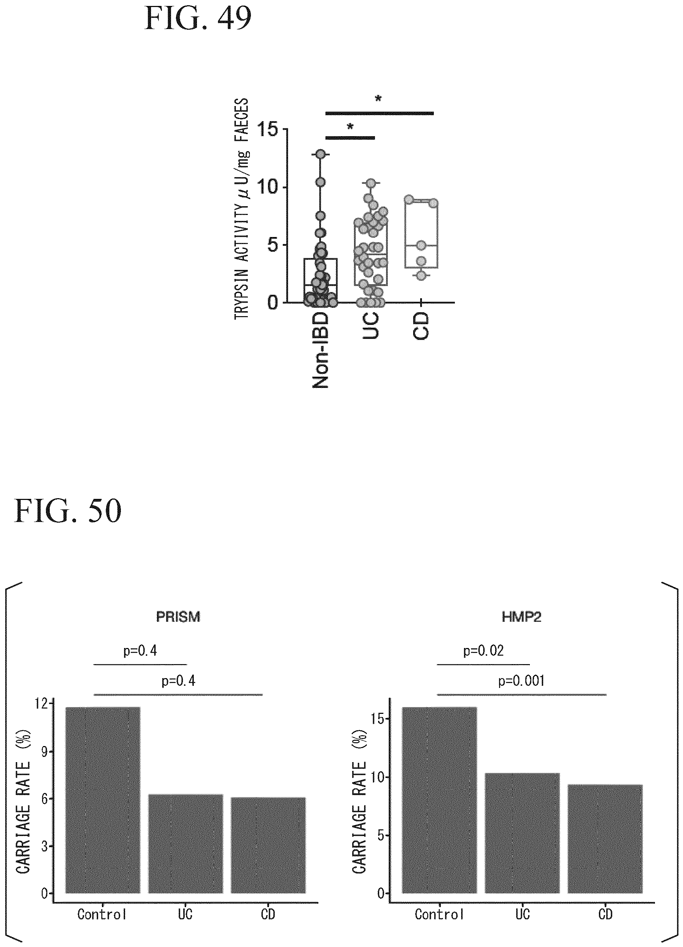

FIG. 49 is a graph showing the results of measuring the trypsin activity. In FIG. 49, "*" indicates that there is a significant difference at p < 0.05. As a result, it was clarified that the trypsin activity in the faeces was high in the UC patients and the CD patients as compared with the non-IBD control group.

Subsequently, the *Paraprevotella* carriage rate in the non-IBD control group of two IBD cohorts (PRISM and HMP2), the UC group, and the CD group was analyzed. FIG. 50 is a graph showing the *Paraprevotella* carriage rate in a population of patients diagnosed with ulcerative colitis (UC) and Crohn's disease (CD) in the PRISM cohort and the HMP2 cohort. As a result, both studies revealed that *Paraprevotella* was more abundantly present in the non-IBD specimens than in the UC or CD patients. In addition, this tendency was statistically significant in the HMP2 cohort which was larger.

These results suggest that the *Paraprevotella* carriage is associated with an intestinal health state.

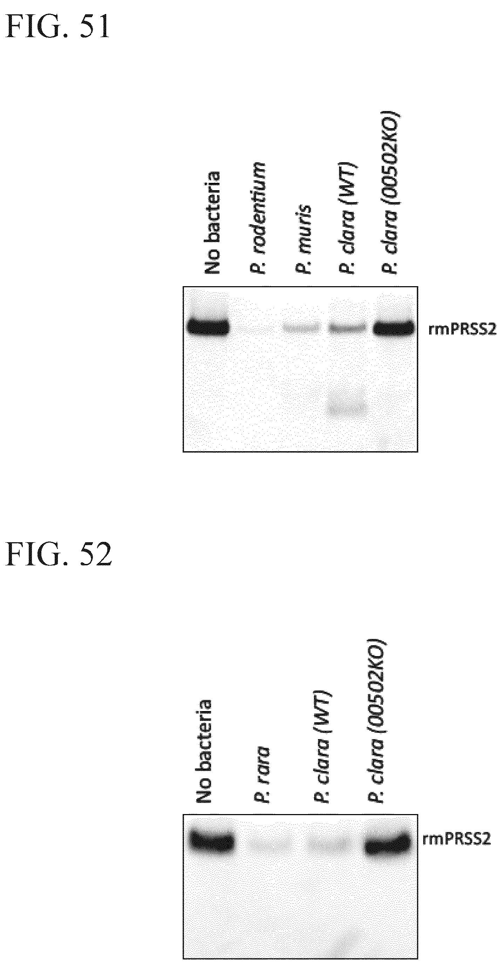

FIG. 51 is a photograph showing the results of measuring the decomposition of trypsin by western blot after mixing each strain of the *Prevotella rodentium* strain, the *Prevotella muris* strain, the wild-type *P. clara* strain as a positive control (*P. clara* (WT), and the *P. clara* strain in which the 00502 gene was knocked out as a negative control (*P*. *clara* (00502KO)) with recombinant mouse trypsin (rmPRSS2, C-terminal His-tag added), and incubating. As a result, it was clarified that the *Prevotella rodentium* strain and the *Prevotella muris* strain had a trypsin-decomposing activity.

FIG. 52 is a photograph showing the results of measuring the decomposition of trypsin by western blot after mixing each strain of the *Prevotella rara* (MSP 0081) strain, the wild-type *P. clara* strain as a positive control (*P*. *clara* (WT), and the *P*. *clara* strain in which the 00502 gene was knocked out as a negative control (*P*. *clara* (00502KO)) with recombinant mouse trypsin (rmPRSS2, C-terminal His-tag added), and incubating. As a result, it was clarified that the *Prevotella rara* (MSP 0081) strain had a trypsin-decomposing activity.

### [Industrial Applicability]

According to the present invention, a technique for regulating the activity of proteases can be provided.

## Claims

1. A composition for decomposition of trypsin or TMPRSS2 comprising, as an active ingredient:
bacteria that have 00502 protein, of a protein having 30% or higher sequence identity with an amino acid sequence of the 00502 protein and having a trypsin-binding ability; or
bacteria that have 00509 protein, of a protein having 30% or higher sequence identity with an amino acid sequence of the 00509 protein and having a trypsin-binding ability.

2. A composition for decomposition of trypsin or TMPRSS2 comprising, as an active ingredient:
bacteria that have 00502 protein, or a protein having 30% or higher sequence identity with an amino acid sequence of the 00502 protein and having a trypsin-binding ability; and
bacteria that have 00509 protein, or a protein having 30% or higher sequence identity with an amino acid sequence of the 00509 protein and having a trypsin-binding ability.

3. A composition for decomposition of trypsin or TMPRSS2 comprising, as an active ingredient:
bacteria that have a gene consisting of a base sequence set forth in SEQ ID NO: 1, or a gene having 30% of higher sequence identity with the base sequence set forth in SEQ ID NO: 1 and encoding a protein having a trypsin-binding ability; or
bacteria that have a gene consisting of a base sequence set forth in SEQ ID NO: 2, or a gene having 30% or higher sequence identity with the base sequence set forth in SEQ ID NO: 2 and encoding a protein having a trypsin-binding ability.

4. A composition for decomposition of trypsin or TMPRSS2 comprising, as an active ingredient:
bacteria that have a gene consisting of a base sequence set forth in SEQ ID NO: 1, or a gene having 30% or higher sequence identity with the base sequence set forth in SEQ ID NO: 1 and encoding a protein having a trypsin-binding ability; and
bacteria that have a gene consisting of a base sequence set forth in SEQ ID NO: 2, or a gene having 30% or higher sequence identity with the base sequence set forth in SEQ ID NO: 2 and encoding a protein having a trypsin-binding ability.

5. The composition for decomposition of trypsin or TMPRSS2 according to any one of Claims 1 to 4,
wherein the bacteria have a type IX secretion system (T9SS).

6. The composition for decomposition of trypsin or TMPRSS2 according to Claim 5,
wherein the T9SS contains PorV protein, PorU protein, PorN protein, PorM protein, PorL protein, PorK protein, or PorP protein.

7. The composition for decomposition of trypsin or TMPRSS2 according to any one of Claims 1 to 6,
wherein the bacteria are bacteria belonging to the genus *Paraprevotella,* the genus *Prevotella,* the genus *Prevotellamasilia*, or the genus *Bacteroidetes.*

8. The composition for decomposition of trypsin or TMPRSS2 according to Claim 7,
wherein the bacteria belonging to the genus *Paraprevotella* are *Paraprevotella clara,* and the bacteria belonging to the genus *Prevotella* are at least one type of bacteria selected from the group consisting of *Prevotella rara*, *Prevotella rodentium*, and *Prevotella muris.*

9. The composition for decomposition of trypsin or TMPRSS2 according to any one of Claims 1 to 8,
wherein the bacteria are bacteria that have a 16S rRNA gene consisting of a base sequence set forth in SEQ ID NO: 3 or a base sequence set forth in SEQ ID NO: 4, or bacteria that have a 16S rRNA gene consisting of a base sequence having 97% or higher sequence identity with the base sequence set forth in SEQ ID NO: 3 or the base sequence set forth in SEQ ID NO: 4.

10. The composition for decomposition of trypsin or TMPRSS2 according to any one of Claims 1 to 6,
wherein the bacteria are at least one type of bacteria selected from the group consisting of *Paraprevotella* sp. MSP 0303, *Paraprevotella* sp. MSP 0335, *Prevotellamassilia timonensis*, *Bacteroidetes* sp. MSP 0288, *Bacteroidetes* sp. MSP 0410, *Bacteroidetes* sp. MSP 0435, and *Porphyromonas gingivalis.*

11. The composition for decomposition of trypsin or TMPRSS2 according to any one of Claims 1 to 10,
wherein the bacteria are live bacteria.

12. The composition for decomposition of trypsin or TMPRSS2 according to any one of Claims 1 to 10,
wherein the bacteria are dead bacteria.

13. A composition for decomposition of trypsin or TMPRSS2 comprising, as an active ingredient:
00502 protein, or a protein having 30% or higher sequence identity with an amino acid sequence of the 00502 protein and having a trypsin-binding ability; or
00509 protein, or a protein having 30% or higher sequence identity with an amino acid sequence of the 00509 protein and having a trypsin-binding ability.

14. A composition for decomposition of trypsin or TMPRSS2 comprising, as an active ingredient:
00502 protein, or a protein having 30% or higher sequence identity with an amino acid sequence of the 00502 protein and having a trypsin-binding ability; and
00509 protein, or a protein having 30% or higher sequence identity with an amino acid sequence of the 00509 protein and having a trypsin-binding ability.

15. The composition for decomposition of trypsin or TMPRSS2 according to any one of Claims 1 to 14,
wherein the composition for decomposition of trypsin or TMPRSS2 is for a treatment of a disease caused by trypsin or TMPRSS2.

16. The composition for decomposition of trypsin or TMPRSS2 according to any one of Claims 1 to 15,
wherein the disease caused by trypsin or TMPRSS2 is an inflammatory bowel disease (ulcerative colitis, Crohn's disease), irritable bowel syndrome, an infectious disease, acute pancreatitis, or chronic pancreatitis.

17. The composition for decomposition of trypsin or TMPRSS2 according to Claim 16,
wherein the infectious disease is a virus infectious disease or a bacterial infectious disease.

18. The composition for decomposition of trypsin or TMPRSS2 according to Claim 16 or 17,
wherein the inflammatory bowel disease, the irritable bowel syndrome, or the infectious disease is a disease associated with TMPRSS2 or IgA.

19. A diagnostic drug for diseases caused by trypsin or TMPRSS2, the diagnostic drug comprising:
a specific binding substance that detects
00502 protein, or a protein having 30% or higher sequence identity with an amino acid sequence of the 00502 protein and having a trypsin-binding ability, or
00509 protein, or a protein having 30% or higher sequence identity with an amino acid sequence of the 00509 protein and having a trypsin-binding ability.

20. A diagnostic drug for diseases caused by trypsin or TMPRSS2, the diagnostic drug comprising:
a primer set or a probe that detects
a gene consisting of a base sequence set forth in SEQ ID NO: 1, or a gene having 30% or higher sequence identity with the base sequence set forth in SEQ ID NO: 1 and encoding a protein having a trypsin-binding ability, or
a gene consisting of a base sequence set forth in SEQ ID NO: 2, or a gene having 30% or higher sequence identity with the base sequence set forth in SEQ ID NO: 2 and encoding a protein having a trypsin-binding ability.

21. A quasi-drug for diseases caused by trypsin or TMPRSS2, the quasi-drug comprising, as an active ingredient:
bacteria that have
00502 protein, or a protein having 30% or higher sequence identity with an amino acid sequence of the 00502 protein and having a trypsin-binding ability, or
00509 protein, or a protein having 30% or higher sequence identity with an amino acid sequence of the 00509 protein and having a trypsin-binding ability.

22. A quasi-drug for diseases caused by trypsin or TMPRSS2, the quasi-drug comprising, as an active ingredient:
00502 protein, or a protein having 30% or higher sequence identity with an amino acid sequence of the 00502 protein and having a trypsin-binding ability; or
00509 protein, or a protein having 30% or higher sequence identity with an amino acid sequence of the 00509 protein and having a trypsin-binding ability.
